(19) Europäisches Patentamt
European Patent Office
Office européen des brevets

(11) EP 0 644 190 B1

(12) # EUROPÄISCHE PATENTSCHRIFT

(45) Veröffentlichungstag und Bekanntmachung des Hinweises auf die Patenterteilung:
**17.03.1999 Patentblatt 1999/11**

(51) Int Cl.$^{6}$: **C07D 307/83**, C08K 5/15

(21) Anmeldenummer: **94810520.0**

(22) Anmeldetag: **08.09.1994**

(54) **Benzofuran-2-one als Stabilisatoren**

Benzofuran-2-ones as stabilizers

Benzofuran-2-ones en tant que stabilisateurs

(84) Benannte Vertragsstaaten:
**AT BE CH DE ES FR GB IT LI NL SE**

(30) Priorität: **17.09.1993 CH 2811/93**

(43) Veröffentlichungstag der Anmeldung:
**22.03.1995 Patentblatt 1995/12**

(73) Patentinhaber: **Ciba Specialty Chemicals Holding Inc.**
**4057 Basel (CH)**

(72) Erfinder:
- **Nesvadba, Peter, Dr.**
  **CH-1723 Marly (CH)**
- **Evans, Samuel, Dr.**
  **CH-1723 Marly (CH)**

(56) Entgegenhaltungen:
**EP-A- 0 415 887**
**GB-A- 2 257 140**
**WO-A-80/01566**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 644 190 B1

## Beschreibung

Die vorliegende Erfindung betrifft Zusammensetzungen, enthaltend ein organisches Material, bevorzugt ein Polymer, und Benzofuran-2-one als Stabilisatoren, die Verwendung derselben zum Stabilisieren von organischen Materialien gegen oxidativen, thermischen oder lichtinduzierten Abbau sowie neue Benzofuran-2-one.

Einzelne Benzofuran-2-one sind in der Literatur bekannt, und wurden beispielsweise in Beilstein 18, 17 und Beilstein E III/IV, 18, 154-166 erwähnt oder von Th. Kappe et al., Monatshefte für Chemie 99, 990 (1968); J. Morvan et al., Bull. Soc. Chim. Fr. 1979, 583; L. F. Clarke et al., J. Org. Chem. 57, 362 (1992); M. Julia et al., Bull. Soc. Chim. Fr. 1965, 2175 oder H. Sterk et al., Monatshefte für Chemie 99, 2223 (1968) beschrieben. In keiner Publikation werden diese Verbindungen als Stabilisatoren für organische Materialien verwendet.

Die Verwendung von einigen Benzofuran-2-onen als Stabilisatoren für organische Polymere ist beispielsweise aus US-A-4 325 863; US-A-4 338 244 und US-A-5 175 312 bekannt.

Es wurde nun gefunden, dass eine ausgewählte Gruppe solcher Benzofuran-2-one sich besonders gut als Stabilisatoren für organische Materialien, die gegen oxidativen, thermischen oder lichtinduzierten Abbau empfindlich sind, eignen.

Die vorliegende Erfindung betrifft daher Zusammensetzungen enthaltend

a) ein dem oxidativen, thermischen oder lichtinduzierten Abbau unterworfenes organisches Material und
b) mindestens eine Verbindung der Formel I

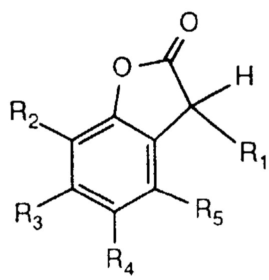
(I)

worin
$R_1$ Halogen oder -OR'$_1$ darstellt,
R'$_1$ Wasserstoff, $C_1$-$C_{25}$-Alkanoyl, $C_3$-$C_{25}$-Alkenoyl, durch Sauerstoff, Schwefel oder >N-$R_6$ unterbrochenes $C_3$-$C_{25}$-Alkanoyl; $C_6$-$C_9$-Cycloalkylcarbonyl, Thenoyl, Furoyl, Benzoyl oder durch $C_1$-$C_{12}$-Alkyl substituiertes Benzoyl; Naphtoyl oder durch $C_1$-$C_{12}$-Alkyl substituiertes Naphtoyl; $C_1$-$C_{25}$-Alkansulfonyl, durch Fluor substituiertes $C_1$-$C_{25}$-Alkansulfonyl; Phenylsulfonyl oder durch $C_1$-$C_{12}$-Alkyl substituiertes Phenylsulfonyl;

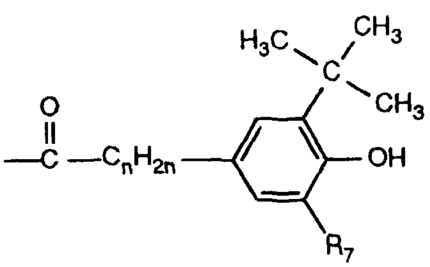
,
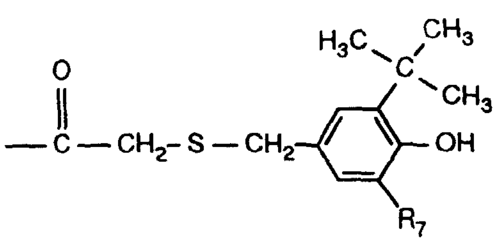
,

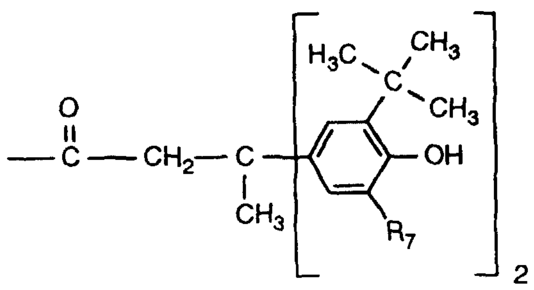, 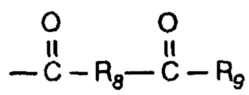 oder 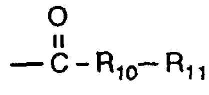

bedeutet,

$R_2$, $R_3$, $R_4$ und $R_5$ unabhängig voneinander Wasserstoff, Chlor, Hydroxy, $C_1$-$C_{25}$-Alkyl, $C_7$-$C_9$-Phenylalkyl, unsubstituiertes oder durch $C_1$-$C_4$-Alkyl substituiertes Phenyl, unsubstituiertes oder durch $C_1$-$C_4$-Alkyl substituiertes $C_5$-$C_8$-Cycloalkyl; $C_1$-$C_{18}$-Alkoxy, $C_1$-$C_{18}$-Alkylthio, $C_1$-$C_4$-Alkylamino, Di-($C_1$-$C_4$-alkyl)amino, $C_1$-$C_{25}$-Alkanoyloxy, $C_1$-$C_{25}$-Alkanoylamino, $C_3$-$C_{25}$-Alkenoyloxy, durch Sauerstoff, Schwefel oder $>$N-$R_6$ unterbrochenes $C_3$-$C_{25}$-Alkanoyloxy; $C_6$-$C_9$-Cycloalkylcarbonyloxy, Benzoyloxy oder durch $C_1$-$C_{12}$-Alkyl substituiertes Benzoyloxy darstellen, oder ferner die Reste $R_2$ und $R_3$ oder die Reste $R_3$ und $R_4$ oder die Reste $R_4$ und $R_5$ zusammen mit den Kohlenstoffatomen, an die sie gebunden sind, einen Benzoring bilden, $R_4$ zusätzlich -$(CH_2)_p$-$COR_9$ oder $(CH_2)_qOH$ darstellt, oder wenn $R_3$ und $R_5$ Wasserstoff sind, $R_4$ zusätzlich einen Rest der Formel II

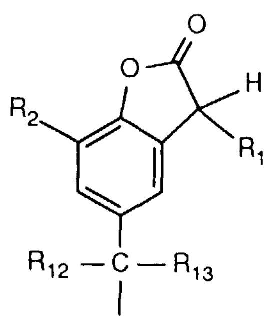 (II)

bedeutet,

$R_6$ Wasserstoff oder $C_1$-$C_8$-Alkyl darstellt,

$R_7$ Wasserstoff oder $C_1$-$C_8$-Alkyl bedeutet,

$R_8$ eine direkte Bindung, $C_1$-$C_{18}$-Alkylen, durch Sauerstoff, Schwefel oder $>$N-$R_6$ unterbrochenes $C_2$-$C_{18}$-Alkylen; $C_2$-$C_{18}$-Alkenylen, $C_2$-$C_{20}$-Alkyliden, $C_7$-$C_{20}$-Phenylalkyliden, $C_5$-$C_8$-Cycloalkylen, $C_7$-$C_8$-Bicycloalkylen, unsubstituiertes oder durch $C_1$-$C_4$-Alkyl substituiertes Phenylen,

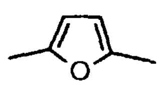 oder 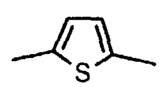

darstellt, $R_9$ Hydroxy, $[-O^{\ominus} \frac{1}{r} M^{r+}]$, $C_1$-$C_{18}$-Alkoxy oder

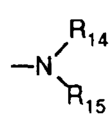

bedeutet,

$R_{10}$ Sauerstoff, -NH- oder

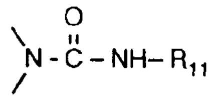

darstellt,

$R_{11}$ $C_1$-$C_{18}$-Alkyl oder Phenyl ist,

$R_{12}$ und $R_{13}$ unabhängig voneinander Wasserstoff, $CF_3$, $C_1$-$C_{12}$-Alkyl oder Phenyl darstellen, oder $R_{12}$ und $R_{13}$ zusammen mit dem C-Atom, an das sie gebunden sind, einen unsubstituierten oder durch 1 bis 3 $C_1$-$C_4$-Alkyl substituierten $C_5$-$C_8$-Cycloalkylidenring bilden;

$R_{14}$ und $R_{15}$ unabhängig voneinander Wasserstoff oder $C_1$-$C_{18}$-Alkyl darstellen,

M ein r-wertiges Metallkation ist,

n 0, 1 oder 2 bedeutet,

p 0, 1 oder 2 darstellt,

q 1, 2, 3, 4, 5 oder 6 bedeutet, und

r 1, 2 oder 3 ist.

Halogen bedeutet beispielsweise Chlor, Brom oder Iod, bevorzugt Chlor.

Alkanoyl mit bis zu 25 Kohlenstoffatomen bedeutet einen verzweigten oder unverzweigten Rest wie beispielsweise Formyl, Acetyl, Propionyl, Butanoyl, Pentanoyl, Hexanoyl, Heptanoyl, Octanoyl, Nonanoyl, Decanoyl, Undecanoyl, Dodecanoyl, Tridecanoyl, Tetradecanoyl, Pentadecanoyl, Hexadecanoyl, Heptadecanoyl, Octadecanoyl, Eicosanoyl oder Docosanoyl. $R'_1$ als Alkanoyl hat vorzugsweise 2 bis 18, insbesondere 2 bis 12, z.B. 2 bis 6 Kohlenstoffatome. Besonders bevorzugt ist Acetyl.

Alkanoyloxy mit bis zu 25 Kohlenstoffatomen bedeutet einen verzweigten oder unverzweigten Rest wie beispielsweise Formyloxy, Acetoxy, Propionyloxy, Butanoyloxy, Pentanoyloxy, Hexanoyloxy, Heptanoyloxy, Octanoyloxy, Nonanoyloxy, Decanoyloxy, Undecanoyloxy, Dodecanoyloxy, Tridecanoyloxy, Tetradecanoylxoy, Pentadecanoyloxy, Hexadecanoyloxy, Heptadecanoyloxy, Octadecanoyloxy, Eicosanoyloxy oder Docosanoyloxy. Bevorzugt ist Alkanoyloxy mit 2 bis 18, insbesondere 2 bis 12, z.B. 2 bis 6 Kohlenstoffatomen. Besonders bevorzugt ist Acetoxy.

Alkenoyl mit 3 bis 25 Kohlenstoffatomen bedeutet einen verzweigten oder unverzweigten Rest wie beispielsweise Propenoyl, 2-Butenoyl, 3-Butenoyl, Isobutenoyl, n-2,4-Pentadienoyl, 3-Methyl-2-butenoyl, n-2-Octenoyl, n-2-Dodecenoyl, iso-Dodecenoyl, Oleoyl, n-2-Octadecenoyl oder n-4-Octadecenoyl. Bevorzugt ist Alkenoyl mit 3 bis 18, insbesondere 3 bis 12, z.B. 3 bis 6, vor allem 3 bis 4 Kohlenstoffatomen.

Alkenoyloxy mit 3 bis 25 Kohlenstoffatomen bedeutet einen verzweigten oder unverzweigten Rest wie beispielsweise Propenoyloxy, 2-Butenoyloxy, 3-Butenoyloxy, Isobutenoyloxy, n-2,4-Pentadienoyloxy, 3-Methyl-2-butenoyloxy, n-2-Octenoyloxy, n-2-Dodecenoyloxy, iso-Dodecenoyloxy, Oleoyloxy, n-2-Octadecenoyloxy oder n-4-Octadecenoyloxy. Bevorzugt ist Alkenoyloxy mit 3 bis 18, insbesondere 3 bis 12, z.B. 3 bis 6, vor allem 3 bis 4 Kohlenstoffatomen.

Durch Sauerstoff, Schwefel oder $>$N-$R_6$ unterbrochenes $C_3$-$C_{25}$-Alkanoyl bedeutet beispielsweise $CH_3$-O-$CH_2$CO-, $CH_3$-S-$CH_2$CO-, $CH_3$-NH-$CH_2$CO-, $CH_3$-N($CH_3$)-$CH_2$CO-, $CH_3$-O-$CH_2$$CH_2$-O-$CH_2$CO-, $CH_3$-(O-$CH_2$$CH_2$-$)_2$O-$CH_2$CO-, $CH_3$-(O-$CH_2$$CH_2$-$)_3$O-$CH_2$CO- oder $CH_3$-(O-$CH_2$$CH_2$-$)_4$O-$CH_2$CO-.

Durch Sauerstoff, Schwefel oder $>$N-$R_6$ unterbrochenes $C_3$-$C_{25}$-Alkanoyloxy bedeutet beispielsweise $CH_3$-O-$CH_2$COO-, $CH_3$-S-$CH_2$COO-, $CH_3$-NH-$CH_2$COO-, $CH_3$-N($CH_3$)-$CH_2$COO-, $CH_3$-O-$CH_2$$CH_2$-O-$CH_2$COO-, $CH_3$-(O-$CH_2$$CH_2$-$)_2$O-$CH_2$COO-, $CH_3$-(O-$CH_2$$CH_2$-$)_3$O-$CH_2$COO- oder $CH_3$-(O-$CH_2$$CH_2$-$)_4$O-$CH_2$COO-.

$C_6$-$C_9$-Cycloalkylcarbonyl bedeutet beispielsweise Cyclopentylcarbonyl, Cyclohexylcarbonyl, Cycloheptylcarbonyl oder Cyclooctylcarbonyl. Cyclohexylcarbonyl ist bevorzugt.

$C_6$-$C_9$-Cycloalkylcarbonyloxy bedeutet beispielsweise Cyclopentylcarbonyloxy, Cyclohexylcarbonyloxy, Cycloheptylcarbonyloxy oder Cyclooctylcarbonyloxy. Cyclohexylcarbonyloxy ist bevorzugt.

Durch $C_1$-$C_{12}$-Alkyl substituiertes Benzoyl, das vorzugsweise 1 bis 3, insbesondere 1 oder 2 Alkylgruppen trägt, bedeutet beispielsweise o-, m- oder p-Methylbenzoyl, 2,3-Dimethylbenzoyl, 2,4-Dimethylbenzoyl, 2,5-Dimethylbenzoyl, 2,6-Dimethylbenzoyl, 3,4-Dimethylbenzoyl, 3,5-Dimethylbenzoyl, 2-Methyl-6-ethylbenzoyl, 4-tert-Butylbenzoyl, 2-Ethylbenzoyl, 2,4,6-Trimethylbenzoyl, 2,6-Dimethyl-4-tert-butylbenzoyl oder 3,5-Di-tert-butylbenzoyl. Bevorzugte Substituenten sind $C_1$-$C_8$-Alkyl, insbesondere $C_1$-$C_4$-Alkyl.

Durch $C_1$-$C_{12}$-Alkyl substituiertes Benzoyloxy, das vorzugsweise 1 bis 3, insbesondere 1 oder 2 Alkylgruppen trägt, bedeutet beispielsweise o-, m- oder p-Methylbenzoyloxy, 2,3-Dimethylbenzoyloxy, 2,4-Dimethylbenzoyloxy, 2,5-Dimethylbenzoyloxy, 2,6-Dimethylbenzoyloxy, 3,4-Dimethylbenzoyloxy, 3,5-Dimethylbenzoyloxy, 2-Methyl-6-ethylbenzoyloxy, 4-tert-Butylbenzoyloxy, 2-Ethylbenzoyloxy, 2,4,6-Trimethylbenzoyloxy, 2,6-Dimethyl-4-tert-butylbenzoyloxy oder 3,5-Di-tert-butylbenzoyloxy. Bevorzugte Substituenten sind $C_1$-$C_8$-Alkyl, insbesondere $C_1$-$C_4$-Alkyl.

Durch $C_1$-$C_{12}$-Alkyl substituiertes Naphtoyl, das 1-Naphtoyl oder 2-Naphtoyl bedeutet und das vorzugsweise 1 bis 3, insbesondere 1 oder 2 Alkylgruppen enthält, bedeutet beispielsweise 1-, 2-, 3-, 4-, 5-, 6-, 7- oder 8-Methyl-naphtoyl, 1-, 2-, 3-, 4-, 5-, 6-, 7- oder 8-Ethyl-naphtoyl, 4-tert-Butyl-naphtoyl oder 6-tert-Butyl-naphtoyl. Besonders bevorzugte Substituenten sind $C_1$-$C_8$-Alkyl, insbesondere $C_1$-$C_4$-Alkyl.

$C_1$-$C_{25}$-Alkansulfonyl bedeutet einen verzweigten oder unverzweigten Rest wie beispielsweise Methansulfonyl, Ethansulfonyl, Propansulfonyl, Butansulfonyl, Pentansulfonyl, Hexansulfonyl, Heptansulfonyl, Oktansulfonyl, Nonansulfonyl oder Docosansulfonyl. Bevorzugt ist Alkansulfonyl mit 1 bis 18, insbesondere 1 bis 12, z.B. 2 bis 6 Kohlenstoffatomen.

Besonders bevorzugt ist Methansulfonyl.

Durch Fluor substituiertes $C_1$-$C_{25}$-Alkansulfonyl bedeutet beispielsweise Trifluormethansulfonyl.

Durch $C_1$-$C_{12}$-Alkyl substituiertes Phenylsulfonyl, das vorzugsweise 1 bis 3, insbesondere 1 oder 2 Alkylgruppen trägt, bedeutet beispielsweise o-, m- oder p-Methylphenylsulfonyl, p-Ethylphenylsulfonyl, p-Propylphenylsulfonyl oder p-Butylphenylsulfonyl. Bevorzugte Substituenten sind $C_1$-$C_8$-Alkyl, insbesondere $C_1$-$C_4$-Alkyl. Besonders bevorzugt ist p-Methylphenylsulfonyl.

Alkyl mit bis zu 25 Kohlenstoffatomen bedeutet einen verzweigten oder unverzweigten Rest wie beispielsweise Methyl, Ethyl, Propyl, Isopropyl, n-Butyl, sec-Butyl, Isobutyl, tert-Butyl, 2-Ethylbutyl, n-Pentyl, Isopentyl, 1-Methylpentyl, 1,3-Dimethylbutyl, n-Hexyl, 1-Methylhexyl, n-Heptyl, Isoheptyl, 1,1,3,3-Tetramethylbutyl, 1-Methylheptyl, 3-Methylheptyl, n-Octyl, 2-Ethylhexyl, 1,1,3-Trimethylhexyl, 1,1,3,3-Tetramethylpentyl, Nonyl, Decyl, Undecyl, 1-Methylundecyl, Dodecyl, 1,1,3,3,5,5-Hexamethylhexyl, Tridecyl, Tetradecyl, Pentadecyl, Hexadecyl, Heptadecyl, Octadecyl, Eicosyl oder Docosyl. Eine der bevorzugten Bedeutungen von $R_2$ und $R_4$ ist beispielweise $C_1$-$C_{18}$-Alkyl. Eine besonders bevorzugte Bedeutung von $R_4$ ist $C_1$-$C_4$-Alkyl.

$C_7$-$C_9$-Phenylalkyl bedeutet beispielsweise Benzyl, $\alpha$-Methylbenzyl, $\alpha,\alpha$-Dimethylbenzyl oder 2-Phenylethyl. Benzyl und $\alpha,\alpha$-Dimethylbenzyl ist bevorzugt.

Durch $C_1$-$C_4$-Alkyl substituiertes Phenyl, das vorzugsweise 1 bis 3, insbesondere 1 oder 2 Alkylgruppen enthält, bedeutet beispielsweise o-, m- oder p-Methylphenyl, 2,3-Dimethylphenyl, 2,4-Dimethylphenyl, 2,5-Dimethylphenyl, 2,6-Dimethylphenyl, 3,4-Dimethylphenyl, 3,5-Dimethylphenyl, 2-Methyl-6-ethylphenyl, 4-tert-Butylphenyl, 2-Ethylphenyl oder 2,6-Diethylphenyl.

Unsubstituiertes oder durch $C_1$-$C_4$-Alkyl substituiertes $C_5$-$C_8$-Cycloalkyl bedeutet beispielsweise Cyclopentyl, Methylcyclopentyl, Dimethylcyclopentyl, Cyclohexyl, Methylcyclohexyl, Dimethylcyclohexyl, Trimethylcyclohexyl, tert-Butylcyclohexyl, Cycloheptyl oder Cyclooctyl. Bevorzugt ist Cyclohexyl und tert-Butylcyclohexyl.

Alkoxy mit bis zu 18 Kohlenstoffatomen bedeutet einen verzweigten oder unverzweigten Rest wie beispielsweise Methoxy, Ethoxy, Propoxy, Isopropoxy, n-Butoxy, Isobutoxy, Pentoxy, Isopentoxy, Hexoxy, Heptoxy, Octoxy, Decyloxy, Tetradecyloxy, Hexadecyloxy oder Octadecyloxy. Bevorzugt ist Alkoxy mit 1 bis 12, insbesondere 1 bis 8, z.B. 1 bis 6 Kohlenstoffatomen.

Alkylthio mit bis zu 18 Kohlenstoffatomen bedeutet einen verzweigten oder unverzweigten Rest wie beispielsweise Methylthio, Ethylthio, Propylthio, Isopropylthio, n-Butylthio, Isobutylthio, Pentylthio, Isopentylthio, Hexylthio, Heptylthio, Octylthio, Decylthio, Tetradecylthio, Hexadecylthio oder Octadecylthio. Bevorzugt ist Alkylthio mit 1 bis 12, insbesondere 1 bis 8, z.B. 1 bis 6 Kohlenstoffatomen.

Alkylamino mit bis zu 4 Kohlenstoffatomen bedeutet einen verzweigten oder unverzweigten Rest wie beispielsweise Methylamino, Ethylamino, Propylamino, Isopropylamino, n-Butylamino, Isobutylamino oder tert-Butylamino.

Di-($C_1$-$C_4$-alkyl)amino bedeutet auch, dass die beiden Reste unabhängig voneinander verzweigt oder unverzweigt sind wie beispielsweise Dimethylamino, Methylethylamino, Diethylamino, Methyl-n-propylamino, Methylisopropylamino, Methyl-n-butylamino, Methylisobutylamino, Ethylisopropylamino, Ethyl-n-butylamino, Ethylisobutylamino, Ethyl-tert-butylamino, Diethylamino, Diisopropylamino, Isopropyl-n-butylamino, Isopropylisobutylamino, Di-n-butylamino oder Di-isobutylamino.

Alkanoylamino mit bis zu 25 Kohlenstoffatomen bedeutet einen verzweigten oder unverzweigten Rest wie beispielsweise Formylamino, Acetylamino, Propionylamino, Butanoylamino, Pentanoylamino, Hexanoylamino, Heptanoylamino, Octanoylamino, Nonanoylamino, Decanoylamino, Undecanoylamino, Dodecanoylamino, Tridecanoylamino, Tetradecanoylamino, Pentadecanoylamino, Hexadecanoylamino, Heptadecanoylamino, Octadecanoylamino, Eicosanoylamino oder Docosanoylamino. Bevorzugt ist Alkanoylamino mit 2 bis 18, insbesondere 2 bis 12, z.B. 2 bis 6 Kohlenstoffatomen.

$C_1$-$C_{18}$-Alkylen bedeutet einen verzweigten oder unverzweigten Rest wie beispielsweise Methylen, Ethylen, Propylen, Trimethylen, Tetramethylen, Pentamethylen, Hexamethylen, Heptamethylen, Octamethylen, Decamethylen, Dodecamethylen oder Octadecamethylen. Bevorzugt ist $C_1$-$C_{12}$-Alkylen, insbesondere $C_1$-$C_8$-Alkylen.

Durch Sauerstoff, Schwefel oder $\rangle N-R_6$ unterbrochenes $C_2$-$C_{18}$-Alkylen bedeutet beispielsweise $-CH_2-O-CH_2-$, $-CH_2-S-CH_2-$, $-CH_2-NH-CH_2-$, $-CH_2-N(CH_3)-CH_2-$, $-CH_2-O-CH_2CH_2-O-CH_2-$, $-CH_2-(O-CH_2CH_2-)_2O-CH_2-$, $-CH_2-(O-CH_2CH_2-)_3O-CH_2-$, $-CH_2-(O-CH_2CH_2-)_4O-CH_2-$ oder $-CH_2CH_2-S-CH_2CH_2-$.

$C_2$-$C_{18}$-Alkenylen bedeutet beispielsweise Vinylen, Methylvinylen, Octenylethylen oder Dodecenylethylen. Bevorzugt ist $C_2$-$C_8$-Alkenylen.

Alkyliden mit 2 bis 20 Kohlenstoffatomen bedeutet beispielsweise Ethyliden, Propyliden, Butyliden, Pentyliden, 4-Methylpentyliden, Heptyliden, Nonyliden, Tridecyliden, Nonadecyliden, 1-Methylethyliden, 1-Ethylpropyliden oder 1-Ethylpentyliden. Bevorzugt ist $C_2$-$C_8$-Alkyliden.

Phenylalkyliden mit 7 bis 20 Kohlenstoffatomen bedeutet beispielsweise Benzyliden, 2-Phenylethyliden oder 1-Phenyl-2-hexyliden. Bevorzugt ist $C_7$-$C_9$-Phenylalkyliden.

$C_5$-$C_8$-Cycloalkylen bedeutet eine gesättigte Kohlenwasserstoffgruppe mit zwei freien Valenzen und mindestens einer Ringeinheit und ist beispielsweise Cyclopentylen, Cyclohexylen, Cycloheptylen oder Cyclooctylen. Bevorzugt ist Cyclohexylen.

$C_7$-$C_8$-Bicycloalkylen bedeutet beispielsweise Bicycloheptylen oder Bicyclooctylen.

Unsubstituiertes oder durch $C_1$-$C_4$-Alkyl substituiertes Phenylen bedeutet beispielsweise 1,2-, 1,3- oder 1,4-Phenylen.

Durch $C_1$-$C_4$-Alkyl substituierter $C_5$-$C_8$-Cycloalkylidenring, der vorzugsweise 1 bis 3, insbesondere 1 oder 2 verzweigte oder unverzweigte Alkylgruppen-Reste enthält, bedeutet beispielsweise Cyclopentyliden, Methylcyclopentyliden, Dimethylcyclopentyliden, Cyclohexyliden, Methylcyclohexyliden, Dimethylcyclohexyliden, Trimethylcyclohexyliden, tert-Butylcyclohexyliden, Cycloheptyliden oder Cyclooctyliden. Bevorzugt ist Cyclohexyliden und tert-Butylcyclohexyliden.

Ein ein-, zwei- oder drei-wertiges Metallkation ist vorzugsweise ein Alkalimetall-, Erdalkalimetall- oder Aluminium-Kation, beispielsweise $Na^+$, $K^+$, $Mg^{++}$, $Ca^{++}$ oder $Al^{+++}$.

Von Interesse sind Zusammensetzungen enthaltend Verbindungen der Formel I, worin $R'_1$ Wasserstoff, $C_1$-$C_{18}$-Alkanoyl, $C_3$-$C_{18}$-Alkenoyl, durch Sauerstoff, Schwefel oder $>$N-$R_6$ unterbrochenes $C_3$-$C_{18}$-Alkanoyl; $C_6$-$C_8$-Cycloalkylcarbonyl, Thenoyl, Furoyl, Benzoyl oder durch $C_1$-$C_8$-Alkyl substituiertes Benzoyl; Naphtoyl oder durch $C_1$-$C_8$-Alkyl substituiertes Naphtoyl; $C_1$-$C_{18}$-Alkansulfonyl, durch Fluor substituiertes $C_1$-$C_{18}$-Alkansulfonyl; Phenylsulfonyl oder durch $C_1$-$C_8$-Alkyl substituiertes Phenylsulfonyl;

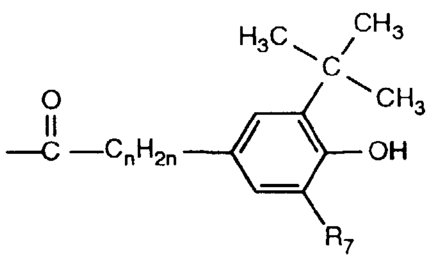 , 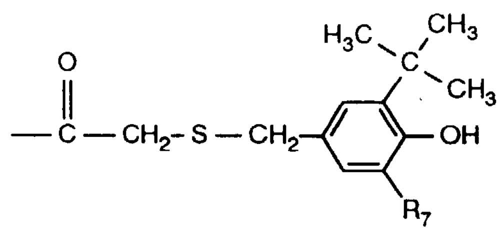 ,

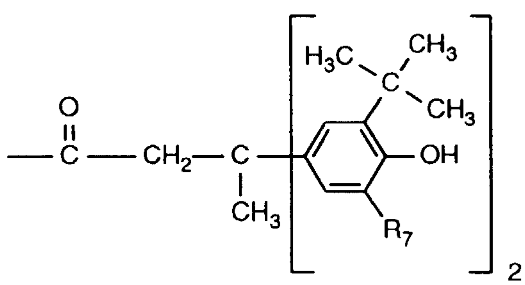 , 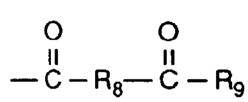 oder 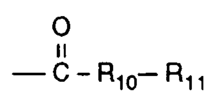

bedeutet,

$R_2$, $R_3$, $R_4$ und $R_5$ unabhängig voneinander Wasserstoff, Chlor, Hydroxy, $C_1$-$C_{25}$-Alkyl, $C_7$-$C_9$-Phenylalkyl, unsubstituiertes oder durch $C_1$-$C_4$-Alkyl substituiertes Phenyl, unsubstituiertes oder durch $C_1$-$C_4$-Alkyl substituiertes $C_5$-$C_8$-Cycloalkyl; $C_1$-$C_{12}$-Alkoxy, $C_1$-$C_{12}$-Alkylthio, $C_1$-$C_4$-Alkylamino, Di-($C_1$-$C_4$-alkyl)amino, $C_1$-$C_{18}$-Alkanoyloxy, $C_1$-$C_{18}$-Alkanoylamino, $C_3$-$C_{18}$-Alkenoyloxy, durch Sauerstoff, Schwefel oder $>$N-$R_6$ unterbrochenes $C_3$-$C_{18}$-Alkanoyloxy; $C_6$-$C_8$-Cycloalkylcarbonyloxy, Benzoyloxy oder durch $C_1$-$C_8$-Alkyl substituiertes Benzoyloxy darstellen, oder ferner die Reste $R_2$ und $R_3$ oder die Reste $R_3$ und $R_4$ oder die Reste $R_4$ und $R_5$ zusammen mit den Kohlenstoffatomen, an die sie gebunden sind, einen Benzoring bilden, $R_4$ zusätzlich -$(CH_2)_p$-$COR_9$ oder $(CH_2)_qOH$ darstellt, oder wenn $R_3$ und $R_5$ Wasserstoff sind, $R_4$ zusätzlich einen Rest der Formel II

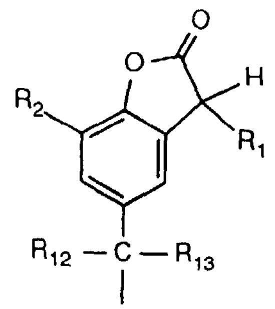

(II)

bedeutet,

$R_6$ Wasserstoff oder $C_1$-$C_6$-Alkyl darstellt,

$R_7$ Wasserstoff oder $C_1$-$C_6$-Alkyl bedeutet,

$R_8$ eine direkte Bindung, $C_1$-$C_{12}$-Alkylen, durch Sauerstoff, Schwefel oder $>$N-$R_6$ unterbrochenes $C_2$-$C_{12}$-Alkylen; $C_2$-$C_{12}$-Alkenylen, $C_2$-$C_{12}$-Alkyliden, $C_7$-$C_{12}$-Phenylalkyliden, $C_5$-$C_8$-Cycloalkylen, $C_7$-$C_8$-Bicycloalkylen oder Phenylen darstellt,

$R_9$ Hydroxy, $C_1$-$C_{12}$-Alkoxy oder

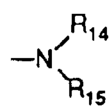

bedeutet,

$R_{10}$ Sauerstoff oder -NH- darstellt,

$R_{11}$ $C_1$-$C_{12}$-Alkyl oder Phenyl ist,

$R_{12}$ und $R_{13}$ Methylgruppen sind oder zusammen mit dem C-Atom, an das sie gebunden sind, einen unsubstituierten oder durch 1 bis 3 $C_1$-$C_4$-Alkyl substituierten $C_5$-$C_8$-Cycloalkylidenring bilden;

$R_{14}$ und $R_{15}$ unabhängig voneinander Wasserstoff oder $C_1$-$C_8$-Alkyl darstellen, und

q 2, 3, 4, 5 oder 6 bedeutet.

Bevorzugt sind Zusammensetzungen enthaltend Verbindungen der Formel I, worin mindestens zwei der Reste $R_2$, $R_3$, $R_4$ und $R_5$ Wasserstoff sind.

Bevorzugt sind auch Zusammensetzungen enthaltend Verbindungen der Formel I, worin $R_3$ und $R_5$ Wasserstoff sind.

Bevorzugt sind ebenfalls Zusammensetzungen enthaltend Verbindungen der Formel I, worin

$R_1$ Chlor, Brom oder -OR'$_1$ darstellt,

R'$_1$ Wasserstoff, $C_1$-$C_{12}$-Alkanoyl, durch Sauerstoff unterbrochenes $C_3$-$C_{12}$-Alkanoyl; Cyclohexylcarbonyl, Benzoyl, Naphtoyl, $C_1$-$C_{12}$-Alkansulfonyl, durch Fluor substituiertes $C_1$-$C_{12}$-Alkansulfonyl; Phenylsulfonyl oder durch $C_1$-$C_4$-Alkyl substituiertes Phenylsulfonyl;

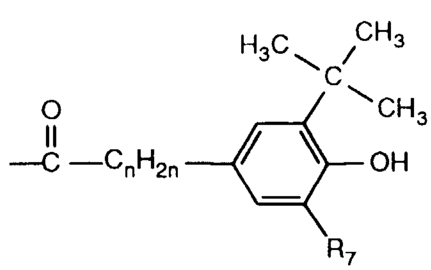 , 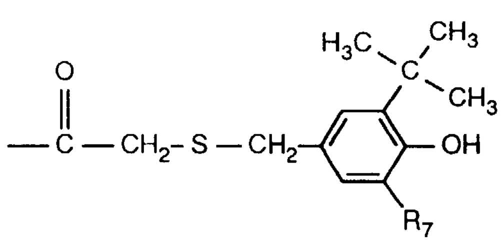 ,

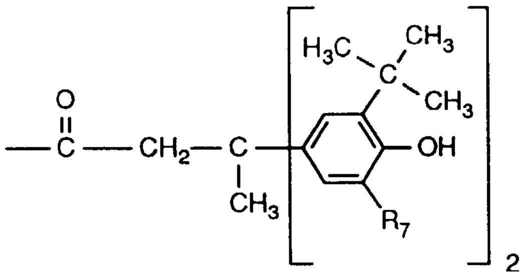 , 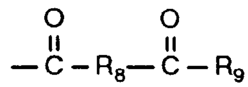 oder 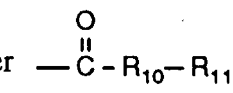

bedeutet,
$R_7$ Wasserstoff oder $C_1$-$C_6$-Alkyl darstellt,
$R_8$ eine direkte Bindung, $C_1$-$C_{12}$-Alkylen, durch Sauerstoff, Schwefel oder $\rangle$N-$R_6$ unterbrochenes $C_2$-$C_{12}$-Alkylen; $C_2$-$C_{12}$-Alkenylen, $C_2$-$C_{12}$-Alkyliden, $C_7$-$C_{12}$-Phenylalkyliden, $C_5$-$C_8$-Cycloalkylen, $C_7$-$C_8$-Bicycloalkylen oder Phenylen bedeutet,
$R_9$ Hydroxy, $C_1$-$C_{12}$-Alkoxy oder

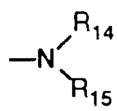

darstellt,
$R_{10}$ Sauerstoff oder -NH- bedeutet,
$R_{11}$ $C_1$-$C_{12}$-Alkyl oder Phenyl ist, und
$R_{14}$ und $R_{15}$ unabhängig voneinander Wasserstoff oder $C_1$-$C_8$-Alkyl darstellen.

Von besonderem Interesse sind Zusammensetzungen enthaltend Verbindungen der Formel I, worin
R'$_1$ Wasserstoff, $C_1$-$C_8$-Alkanoyl, Benzoyl, Methansulfonyl, p-Methylphenylsulfonyl,

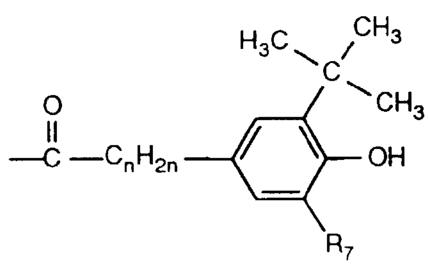 oder 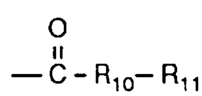

bedeutet, $R_7$ Wasserstoff oder $C_1$-$C_4$-Alkyl darstellt,
$R_{10}$ -NH- ist,
$R_{11}$ $C_1$-$C_8$-Alkyl oder Phenyl bedeutet, und
n 2 ist.

Besonders bevorzugt sind Zusammensetzungen enthaltend Verbindungen der Formel I, worin
$R_2$, $R_3$, $R_4$ und $R_5$ unabhängig voneinander Wasserstoff, Chlor, Hydroxy, $C_1$-$C_{18}$-Alkyl, $C_7$-$C_9$-Phenylalkyl, Phenyl, $C_5$-$C_8$-Cycloalkyl, $C_1$-$C_6$-Alkoxy, Cyclohexylcarbonyloxy oder Benzoyloxy darstellen, oder ferner die Reste $R_2$ und $R_3$ oder die Reste $R_3$ und $R_4$ oder die Reste $R_4$ und $R_5$ zusammen mit den Kohlenstoffatomen, an die sie gebunden sind, einen Benzoring bilden, $R_4$ zusätzlich -$(CH_2)_p$-$COR_9$ darstellt, oder wenn $R_3$ und $R_5$ Wasserstoff sind, $R_4$ zusätzlich einen Rest der Formel II bedeutet,
$R_7$ Wasserstoff oder $C_1$-$C_4$-Alkyl darstellt,
$R_8$ $C_1$-$C_{12}$-Alkylen oder Phenylen bedeutet,
$R_9$ Hydroxy oder $C_1$-$C_8$-Alkoxy darstellt,
$R_{10}$ -NH- ist,
$R_{11}$ $C_1$-$C_8$-Alkyl oder Phenyl bedeutet, und
$R_{12}$ und $R_{13}$ Methylgruppen sind oder zusammen mit dem C-Atom, an das sie gebunden sind, einen $C_5$-$C_8$-Cycloalkylidenring bilden.

Speziell bevorzugt sind Zusammensetzungen enthaltend Verbindungen der Formel I, worin

$R_1$ Chlor oder $-OR'_1$ darstellt,
$R'_1$ Wasserstoff, $C_1$-$C_8$-Alkanoyl, Benzoyl, Methansulfonyl, Trifluormethansulfonyl; Phenylsulfonyl oder durch $C_1$-$C_4$-Alkyl substituiertes Phenylsulfonyl;

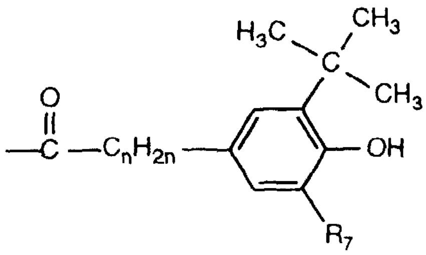 oder 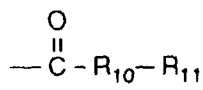

bedeutet,
$R_2$, $R_3$, $R_4$ und $R_5$ unabhängig voneinander Wasserstoff, Chlor, $C_1$-$C_{18}$-Alkyl, $C_7$-$C_9$-Phenylalkyl, Phenyl, Cyclohexyl, $C_1$-$C_6$-Alkoxy darstellen, $R_4$ zusätzlich $-(CH_2)_p-COR_9$ darstellt, oder wenn $R_3$ und $R_5$ Wasserstoff sind, $R_4$ zusätzlich einen Rest der Formel II

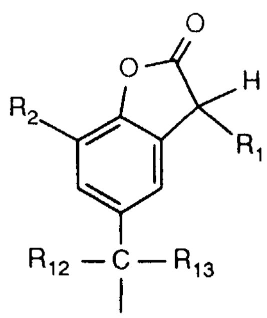 (II)

bedeutet,
$R_7$ Wasserstoff oder $C_1$-$C_4$-Alkyl darstellt,
$R_9$ Hydroxy oder $C_1$-$C_8$-Alkoxy bedeutet,
$R_{10}$ -NH- ist,
$R_{11}$ $C_1$-$C_4$-Alkyl oder Phenyl darstellt,
$R_{12}$ und $R_{13}$ zusammen mit dem C-Atom, an das sie gebunden sind, einen Cyclohexylidenring bilden,
n 2 bedeutet, und
p 2 ist.

Ganz speziell bevorzugt sind Zusammensetzungen enthaltend Verbindungen der Formel I, worin
$R_1$ Chlor oder $-OR'_1$ darstellt,
$R'_1$ Wasserstoff, Acetyl

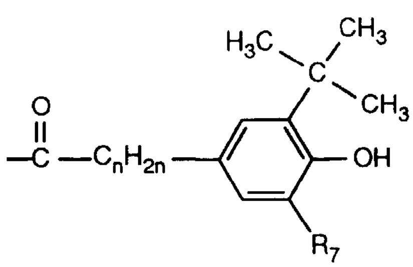 oder 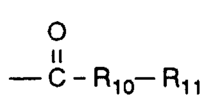

bedeutet,
$R_2$ $C_1$-$C_{16}$-Alkyl, $C_7$-$C_9$-Phenylalkyl oder Cyclohexyl darstellt,
$R_3$ Wasserstoff ist,
$R_4$ $C_1$-$C_4$-Alkyl, $C_7$-$C_9$-Phenylalkyl, Cyclohexyl, $-CH_2CH_2COOH$ oder einen Rest der Formel II

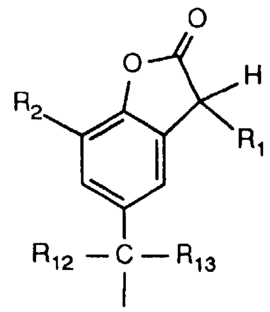

(II)

bedeutet,
$R_5$ Wasserstoff ist,
$R_7$ Wasserstoff oder $C_1$-$C_4$-Alkyl darstellt,
$R_{10}$ -NH- ist,
$R_{11}$ $C_1$-$C_4$-Alkyl bedeutet,
$R_{12}$ und $R_{13}$ zusammen mit dem C-Atom, an das sie gebunden sind, einen Cyclohexylidenring bilden, und
n 2 ist.

Die Verbindungen der Formel I eignen sich zum Stabilisieren von organischen Materialien gegen thermischen, oxidativen oder lichtinduzierten Abbau.

Beispiele für derartige Materialien sind:

1. Polymere von Mono- und Diolefinen, beispielsweise Polypropylen, Polyisobutylen, Polybuten- 1, Poly-4-methylpenten- 1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE), verzweigtes Polyethylen niederer Dichte (VLDPE).

   Polyolefine, d.h. Polymere von Monoolefinen, wie sie beispielhaft im vorstehenden Absatz erwähnt sind, insbesondere Polyethylen und Polypropylen, können nach verschiedenen Verfahren hergestellt werden, insbesondere nach den folgenden Methoden:

   a) radikalisch (gewöhnlich bei hohem Druck und hoher Temperatur).

   b) mittels Katalysator, wobei der Katalysator gewöhnlich ein oder mehrere Metalle der Gruppe IVb, Vb, VIb oder VIII enthält. Diese Metalle besitzen gewöhnlich einen oder mehrere Liganden wie Oxide, Halogenide, Alkoholate, Ester, Ether, Amine, Alkyle, Alkenyle und/oder Aryle, die entweder $\pi$- oder $\sigma$-koordiniert sein können. Diese Metallkomplexe können frei oder auf Träger fixiert sein, wie beispielsweise auf aktiviertem Magnesiumchlorid, Titan(III)chlorid, Aluminiumoxid oder Siliziumoxid. Diese Katalysatoren können im Polymerisationsmedium löslich oder unlöslich sein. Die Katalysatoren können als solche in der Polymerisation aktiv sein, oder es können weitere Aktivatoren verwendet werden, wie beispielsweise Metallalkyle, Metallhydride, Metallalkylhalogenide, Metallalkyloxide oder Metallalkyloxane, wobei die Metalle Elemente der Gruppen Ia, IIa und/oder IIIa sind. Die Aktivatoren können beipielsweise mit weiteren Ester-, Ether-, Amin- oder Silylether-Gruppen modifiziert sein. Diese Katalysatorsysteme werden gewöhnlich als Phillips, Standard Oil Indiana, Ziegler (-Natta), TNZ (DuPont), Metallocen oder Single Site Katalysatoren (SSC) bezeichnet.

2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).

3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat- Copolymere, Ethylen-Vinylacetat-Copolymere und deren Copolymere mit Kohlenstoffmonoxid, oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere, LLDPE/Ethylen-Acrylsäure-Copolymere und alternierend oder statistisch aufgebaute Polyalkylen/Kohlenstoffmonoxid-Copolymere und deren Mischungen mit anderen Polymeren wie z.B. Polyamiden.

4. Kohlenwasserstoffharze (z.B. $C_5$-$C_9$) inklusive hydrierte Modifikationen davon (z.B. Klebrigmacherharze) und Mischungen von Polyalkylenen und Stärke.

5. Polystyrol, Poly-(p-methylstyrol), Poly-($\alpha$-methylstyrol).

6. Copolymere von Styrol oder $\alpha$-Methylstyrol mit Dienen oder Acrylderivaten, wie z.B. Styrol-Butadien, Styrol-Acrylnitril, Styrol-Alkylmethacrylat, Styrol-Butadien-Alkylacrylat und -methacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

7. Pfropfcopolymere von Styrol oder $\alpha$-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 6) genannten Copolymeren, wie sie z.B. als sogenannte ABS-, MBS-, ASA- oder AES-Polymere bekannt sind.

8. Halogenhaltige Polymere, wie z.B. Polychloropren, Chlorkautschuk, chloriertes oder chlorsulfoniertes Polyethylen, Copolymere von Ethylen und chloriertem Ethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.

9. Polymere, die sich von $\alpha,\beta$-ungesättigten Säuren und deren Derivaten ableiten, wie Polyacrylate und Polymethacrylate, mit Butylacrylat schlagzäh modifizierte Polymethylmethacrylate, Polyacrylamide und Polyacrylnitrile.

10. Copolymere der unter 9) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-VinylhalogenidCopolymere oder Acrylnitril-Alkylmethacrylat-Butadien-Terpolymere.

11. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.

12. Homo- und Copolymere von cyclischen Ethern, wie Polyalkylenglykole, Polyethylenoxyd, Polypropylenoxyd oder deren Copolymere mit Bisglycidylethern.

13. Polyacetale, wie Polyoxymethylen, sowie solche Polyoxymethylene, die Comonomere, wie z.B. Ethylenoxid, enthalten; Polyacetale, die mit thermoplastischen Polyurethanen, Acrylaten oder MBS modifiziert sind.

14. Polyphenylenoxide und -sulfide und deren Mischungen mit Styrolpolymeren oder Polyamiden.

15. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.

16. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, 12/12, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid oder Poly-m-phenylen-isophthalamid. Block-Copolymere der vorstehend genanntenPolyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").

17. Polyharnstoffe, Polyimide, Polyamid-imide und Polybenzimidazole.

18. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.

19. Polycarbonate und Polyestercarbonate.

20. Polysulfone, Polyethersulfone und Polyetherketone.

21. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

22. Trocknende und nicht-trocknende Alkydharze.

23. Ungesättigte Polyesterharze, die sich von Copolyestern gsättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

24. Vernetzbare Acrylharze, die sich von substituierten Acrylsäureestern ableiten, wie z.B. von Epoxyacrylaten, Urethan-acrylaten oder Polyester-acrylaten.

25. Alkydharze, Polyesterharze und Acrylatharze, die mit Melaminharzen, Harnstoffharzen, Polyisocyanaten oder Epoxidharzen vernetzt sind.

26. Vernetzte Epoxidharze, die sich von Polyepoxiden ableiten, z.B. von Bis-glycidylethern oder von cycloaliphatischen Diepoxiden.

27. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.

28. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.

29. Natürliche und synthetische organische Stoffe, die reine monomere Verbindungen oder Mischungen von solchen darstellen, beispielsweise Mineralöle, tierische oder pflanzliche Fette, Oele und Wachse, oder Oele, Wachse und Fette auf Basis synthetischer Ester (z.B. Phthalate, Adipate, Phosphate oder Trimellitate), sowie Abmischungen synthetischer Ester mit Mineralölen in beliebigen Gewichtsverhältnissen, wie sie z.B. als Spinnpräparationen Anwendung finden, sowie deren wässrige Emulsionen.

30. Wässrige Emulsionen natürlicher oder synthetischer Kautschuke, wie z.B. Naturkautschuk-Latex oder Latices von carboxylierten Styrol-Butadien-Copolymeren.

Bevorzugte organische Materialien sind Polymere, z.B. natürliche, halbsynthetische oder vor allem synthetische Polymere, insbesondere thermoplastische Polymere, Tackifiers oder Klebstoffe. Besonders bevorzugt sind Polyolefine, z.B. Polypropylen oder Polyethylen.

Besonders hervorzuheben ist die Wirkung der erfindungsgemässen Verbindungen gegen thermischen und oxidativen Abbau, vor allem bei thermischer Belastung, wie sie bei der Verarbeitung von Thermoplasten auftritt. Die erfindungsgemässen Verbindungen sind daher hervorragend als Verarbeitungsstabilisatoren einzusetzen.

Vorzugsweise werden die Verbindungen der Formel I dem zu stabilisierenden Material in Mengen von 0,0005 bis 5 %, insbesondere 0,001 bis 2 %, beispielsweise 0,01 bis 2 %, zugesetzt, bezogen auf das Gewicht des zu stabilisierenden organischen Materials.

Zusätzlich zu den Verbindungen der Formel I können die erfindungsgemässen Zusammensetzungen weitere Costabilisatoren enthalten, wie beispielsweise die folgenden:

1. Antioxidantien

1.1. Alkylierte Monophenole, z.B. 2,6-Di-tert-butyl-4-methylphenol, 2-butyl-4,6-dimethylphenol, 2,6-Di-tert-butyl-4-ethylphenol, 2,6-Di-tert-butyl-4-n-butylphenol, 2,6-Di-tert-butyl-4-iso-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert-butyl-4-methoxymethylphenol, 2,6-Di-nonyl-4-methylphenol, 2,4-Dimethyl-6-(1'-methyl-undec-1'-yl)-phenol, 2,4-Dimethyl-6-(1'-methyl-heptadec-1'-yl)-phenol, 2,4-Dimethyl-6-(1'-methyl-tridec-1'-yl)-phenol und Mischungen davon.

1.2. Alkylthiomethylphenole, z.B. 2,4-Di-octylthiomethyl-6-tert-butylphenol, 2,4-Di-octylthiomethyl-6-methylphenol, 2,4-Di-octylthiomethyl-6-ethylphenol, 2,6-Di-dodecylthiomethyl-4-nonylphenol.

1.3. Hydrochinone und alkylierte Hydrochinone, z.B. 2,6-Di-tert-butyl-4-methoxyphenol, 2,5-Di-tert-butyl-hydrochinon, 2,5-Di-tert-amyl-hydrochinon, 2,6-Diphenyl-4-octadecyloxyphenol, 2,6-Di-tert-butyl-hydrochinon, 2,5-Di-tert-butyl-4-hydroxyanisol, 3,5-Di-tert-butyl-4-hydroxyanisol, 3,5-Di-tert-butyl-4-hydroxyphenyl-stearat, Bis-(3,5-di-tert-butyl-4-hydroxyphenyl)adipat.

1.4. Tocopherole, z.B. α-Tocopherol, β-Tocopherol, γ-Tocopherol, δ-Tocopherol und Mischungen davon (Vitamin E).

1.5. Hydroxylierte Thiodiphenylether, z.B. 2,2'-Thio-bis-(6-tert-butyl-4-methylphenol), 2,2'-Thio-bis-(4-octylphenol), 4,4'-Thio-bis-(6-tert-butyl-3-methylphenol), 4,4'-Thio-bis-(6-tert-butyl-2-methylphenol), 4,4'-Thio-bis-(3,6-di-sec.-amylphenol), 4,4'-Bis-(2,6-dimethyl-4-hydroxyphenyl)-disulfid.

1.6. Alkyliden-Bisphenole, z.B. 2,2'-Methylen-bis-(6-tert-butyl-4-methylphenol), 2,2'-Methylen-bis-(6-tert-butyl-4-ethylphenol), 2,2'-Methylen-bis-[4-methyl-6-(α-methylcyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert-butylphenol), 2,2'-Ethyliden-bis-(4,6-di-tert-butylphenol), 2,2'-Ethyliden-bis-(6-tert-butyl-4-isobutylphenol), 2,2'-Methylen-bis-[6-(α-methylbenzyl)4-nonylphenol], 2,2'-Methylen-bis-[6-(α,α-dimethylbenzyl)-4-nonylphenol], 4,4'-Methylen-bis-(2,6-di-tert-butylphenol), 4,4'-Methylen-bis-(6-tert-butyl-2-methylphenol), 1,1-Bis-(5-tert-butyl-4-hydroxy-2-methylphenyl)-butan, 2,6-Bis-(3-tert-butyl-5-methyl-2-hydroxybenzyl)-4-methylphenol, 1,1,3-Tris-(5-tert-butyl-4-hydroxy-2-methylphenyl)-butan, 1,1-Bis-(5-tert-butyl-4-hydroxy-2-methylphenyl)-3-n-dodecylmercaptobutan, Ethylenglycol-bis-[3,3-bis-(3'-tert-butyl-4'-hydroxyphenyl)-butyrat], Bis-(3-tert-butyl-4-hydroxy-5-methyl-phenyl)-dicyclopentadien, Bis-[2-(3'-tert-butyl-2'-hydroxy-5'-methyl-benzyl)-6-tert-butyl-4-methyl-phenyl]-terephthalat, 1,1-Bis-(3,5-dimethyl-2-hydroxyphenyl)-butan, 2,2-Bis-(3,5-di-tert-butyl-4-hydroxyphenyl)-propan, 2,2-Bis-(5-tert-butyl-4-hydroxy-2-methylphenyl)-4-n-dodecylmercaptobutan, 1,1,5,5-Tetra-(5-tert-butyl-4-hydroxy-2-methylphenyl)-pentan.

1.7. O-, N- und S-Benzylverbindungen, z.B. 3,5,3',5'-Tetra-tert-butyl-4,4'-dihydroxydibenzylether, Octadecyl-4-hydroxy-3,5-dimethylbenzyl-mercaptoacetat, Tris-(3,5-di-tert-butyl-4-hydroxybenzyl)-amin, Bis-(4-tert-butyl-3-hydroxy-2,6-dimethylbenzyl)-dithioterephthalat, Bis-(3,5-di-tert-butyl-4-hydroxybenzyl)-sulfid, Isooctyl-3,5-di-tert-butyl-4-hydroxybenzyl-mercaptoacetat.

1.8. Hydroxybenzylierte Malonate, z.B. Dioctadecyl-2,2-bis-(3,5-di-tert-butyl-2-hydroxybenzyl)-malonat, Di-octadecyl-2-(3-tert-butyl-4-hydroxy-5-methylbenzyl)-malonat, Di-dodecylmercaptoethyl-2,2-bis-(3,5-di-tert-butyl-4-hydroxybenzyl)-malonat. Di-[4-(1,1,3,3-tetramethylbutyl)-phenyl]-2,2-bis-(3,5-di-tert-butyl-4-hydroxybenzyl)-malonat.

1.9. Hydroxybenzyl-Aromaten, z.B. 1,3,5-Tris-(3,5-di-tert-butyl-4-hydroxybenzyl)-2,4,6-trimethylbenzol, 1,4-Bis-(3,5-di-tert-butyl-4-hydroxybenzyl)-2,3,5,6-tetramethylbenzol, 2,4,6-Tris-(3,5-di-tert-butyl-4-hydroxybenzyl)-phenol.

1.10. Triazinverbindungen, z.B. 2,4-Bis-octylmercapto-6-(3,5-di-tert-butyl-4-hydroxyanilino)-1,3,5-triazin, 2-Octylmercapto-4,6-bis-(3,5-di-tert-butyl-4-hydroxyanilino)- 1,3,5-triazin, 2-Octylmercapto-4,6-bis-(3,5-di-tert-butyl-4-hydroxyphenoxy)-1,3,5-triazin, 2,4,6-Tris-(3,5-di-tert-butyl-4-hydroxyphenoxy)-1,2,3-triazin, 1,3,5-Tris-(3,5-di-tert-butyl-4-hydroxybenzyl)-isocyanurat, 1,3,5-Tris-(4-tert-butyl-3-hydroxy-2,6-dimethylbenzyl)-isocyanurat, 2,4,6-Tris-(3,5-di-tert-butyl-4-hydroxyphenylethyl)-1,3,5-triazin, 1,3,5-Tris-(3,5-di-tert-butyl-4-hydroxyphenylpropionyl)-hexahydro-1,3,5-triazin,1,3,5-Tris-(3,5-dicyclohexyl-4-hydroxybenzyl)-isocyanurat.

1.11. Benzylphosphonate, z.B. Dimethyl-2,5-di-tert-butyl-4-hydroxybenzylphosphonat, Diethyl-3,5-di-tert-butyl-4-hydroxybenzylphosphonat, Dioctadecyl-3,5-di-tert-butyl-4-hydroxybenzylphosphonat, Dioctadecyl-5-tert-butyl-4-hydroxy-3-methylbenzylphosphonat, Ca-Salz des 3,5-Di-tert-butyl-4-hydroxybenzyl-phosphonsäure-monoethylesters.

1.12. Acylaminophenole, z.B. 4-Hydroxy-laurinsäureanilid, 4-Hydroxystearinsäureanilid, N-(3,5-di-tert-butyl-4-hydroxyphenyl)-carbaminsäureoctylester.

1.13. Ester der β-(3,5-Di-tert-butyl-4-hydroxyphenyl)-propionsäure mit ein- oder mehr wertigen Alkoholen, wie z. B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.14. Ester der β-(5-tert-Butyl-4-hydroxy-3-methylphenyl)-propionsäure mit ein- oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.15. Ester der β-(3,5-Dicyclohexyl-4-hydroxyphenyl)-propionsäure mit ein- oder mehr wertigen Alkoholen, wie z. B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.16. Ester der 3,5-Di-tert-butyl-4-hydroxyphenylessigsäure mit ein- oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N, N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.17. Amide der β-(3,5-Di-tert-butyl-4-hydroxyphenyl)-propionsäure, wie z.B. N,N'-Bis-(3,5-di-tert-butyl-4-hydroxyphenylpropionyl)-hexamethylendiamin, N,N'-Bis-(3,5-di-tert-butyl-4-hydroxyphenylpropionyl)-trimethylendiamin, N,N'-Bis-(3,5-di-tert-butyl-4-hydroxyphenylpropionyl)-hydrazin.

2. UV-Absorber und Lichtschutzmittel

2.1. 2-(2'-Hydroxyphenyl)-benzotriazole, wie z.B. 2-(2'-Hydroxy-5'-methylphenyl)-benzotriazol, 2-(3',5'-Di-tert-butyl-2'-hydroxyphenyl)-benzotriazol, 2-(5'-tert-Butyl-2'-hydroxyphenyl)-benzotriazol, 2-(2'-Hydroxy-5-(1,1,3,3-tetramethylbutyl)phenyl)-benzotriazol, 2-(3',5'-Di-tert-butyl-2'-hydroxyphenyl)-5-chlor-benzotriazol, 2-(3'-tert-Butyl-2'-hydroxy-5'-methylphenyl)-5-chlor-benzotriazol, 2-(3'-sec-Butyl-5'-tert-butyl-2'-hydroxyphenyl)-benzotriazol, 2-(2'-Hydroxy-4'-octoxyphenyl)-benzotriazol, 2-(3',5'-Di-tert-amyl-2'-hydroxyphenyl)-benzotriazol, 2-(3',5'-Bis-(α,α-dimethylbenzyl)-2'-hydroxyphenyl)-benzotriazol, Mischung aus 2-(3-tert-Butyl-2'-hydroxy-5'-(2-octyloxycarbonylethyl)-phenyl)-5-chlor-benzotriazol, 2-(3'-tert-Butyl-5'-[2-(2-ethylhexyloxy)-carbonylethyl]-2'-hydroxyphenyl)-5-chlor-benzotriazol, 2-(3'-tert-Butyl-2'-hydroxy-5'-(2-methoxycarbonylethyl)phenyl)-5-chlor-benzotriazol, 2-(3'-tert-Butyl-2'-hydroxy-5'-(2-methoxycarbonylethyl)phenyl)-benzotriazol, 2-(3'-tert-Butyl-2'-hydroxy-5'-(2-octyloxycarbonylethyl)phenyl)-benzotriazol, 2-(3'-tert-Butyl-5'-[2-(2-ethylhexyloxy)carbonylethyl]-2'-hydroxyphenyl)-benzotriazol, 2-(3'-Dodecyl-2'-hydroxy-5'-methylphenyl)-benzotriazol, und 2-(3'-tert-Butyl-2'-hydroxy-5'-(2-isooctyloxycarbonylethyl)phenyl-benzotriazol, 2,2'-Methylen-bis[4-(1,1,3,3-tetramethylbutyl)-6-benzotriazol-2-yl-phenol]; Umesterungsprodukt von 2-[3'-tert-Butyl-5'-(2-methoxycarbonylethyl)-2'-hydroxy-phenyl]-benzotriazol mit Polyethylenglycol 300; $[R-CH_2CH_2-COO(CH_2)_3]_2$ mit R = 3'-tert-Butyl-4'-hydroxy-5'-2H-benzotriazol-2-yl-phenyl.

2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2,4'-Trihydroxy-, 2'-Hydroxy-4,4'-dimethoxy-Derivat.

2.3. Ester von gegebenenfalls substituierten Benzoesäuren, wie z.B. 4-tert-Butyl-phenyl-salicylat, Phenylsalicylat, Octylphenyl-salicylat, Dibenzoylresorcin, Bis-(4-tert-butylbenzoyl)-resorcin, Benzoylresorcin, 3,5-Di-tert-butyl-4-hydroxybenzoesäure-2,4-di-tert-butylphenylester, 3,5-Di-tert-butyl-4-hydroxybenzoesäurehexadecylester, 3,5-Di-tert-butyl-4-hydroxybenzoesäure-octadecylester, 3,5-Di-tert-butyl-4-hydroxybenzoesäure-2-methyl-4,6-di-tert-butylphenylester.

2.4. Acrylate, wie z.B. α-Cyan-β,β-diphenylacrylsäure-ethylester bzw. -isooctylester, α-Carbomethoxy-zimtsäure-methylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.

2.5. Nickelverbindungen, wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert-butylbenzylphosphonsäure-monoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.

2.6. Sterisch gehinderte Amine, wie z.B. Bis-(2,2,6,6-tetramethyl-piperidyl)-sebacat, Bis-(2,2,6,6-tetramethyl-piperidyl)-succinat, Bis-(1,2,2,6,6-pentamethylpiperidyl)-sebacat, n-Butyl-3,5-di-tert-butyl-4-hydroxybenzyl-malonsäure-bis(1,2, 2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-Bis-(2,2,6,6-Tetramethyl-4-piperidyl)-hexamethylendiamin und 4-tert-Octylamino-2,6-dichlor-1,3,5-s-triazin, Tris-(2,2,6,6-tetramethyl- 4-piperidyl)-nitrilotriacetat, Tetrakis-(2,2,6,6-tetramethyl-4-piperidyl)-1,2,3,4-butantetraoat, 1,1'-(1,2-Ethandiyl)-bis-(3,3,5,5-tetramethyl-piperazinon), 4-Benzoyl-2,2,6,6-tetramethylpiperidin, 4-Stearyloxy-2,2,6,6-tetramethylpiperidin, Bis-(1,2,2,6,6-pentamethylpiperidyl)-2-n-butyl-2-(2-hydroxy-3,5-di-tert-butylbenzyl)-malonat, 3-n-Octyl-7,7,9,9-tetramethyl-1,3,8-triazaspiro[4.5]decan-2,4-dion, Bis-(1-octyloxy-2,2,6,6-tetramethylpiperidyl)-sebacat, Bis-(1-octyloxy-2,2,6,6-tetramethylpiperidyl)-succinat, Kondensationsprodukt aus N,N'-Bis-(2,2,6,6-tetramethyl-4-piperidyl)-hexamethylendiamin und 4-Morpholino-2,6-dichlor-1,3,5-triazin, Kondensationsprodukt aus 2-Chlor-4,6-di-(4-n-butylamino-2,2,6,6-tetramethylpiperidyl)-1,3,5-triazin und 1,2-Bis-(3-aminopropylamino)äthan, Kondensationsprodukt aus 2-Chlor-4,6-di-(4-n-butylamino-1,2,2,6,6-pentamethylpiperidyl)-1,3,5-triazin und 1,2-Bis-(3-aminopropylamino)-äthan, 8-Acetyl-3-dodecyl-7,7,9,9-tetramethyl-1,3,8-triazaspiro[4.5]decan-2,4-dion, 3-Dodecyl-1-(2,2,6,6-tetramethyl-4-piperidyl)pyrrolidin-2,5-dion, 3-Dodecyl-1-(1,2,2,6,6-pentamethyl-4-piperidyl)-pyrrolidin-2,5-dion.

2.7. Oxalsäurediamide, wie z.B. 4,4'-Di-octyloxy-oxanilid, 2,2'-Diethoxy-oxanilid, 2,2'-Di-octyloxy-5,5'-di-tert-butyl-oxanilid, 2,2'-Di-dodecyloxy-5,5'di-tert-butyl-oxanilid, 2-Ethoxy-2'-ethyl-oxanilid, N,N'-Bis-(3-dimethylaminopropyl)-oxalamid, 2-Ethoxy-5-tert-butyl-2'-ethyloxanilid und dessen Gemisch mit 2-Ethoxy-2'-ethyl-5,4'-di-tert-butyl-oxanilid, Gemische von o- und p-Methoxy- sowie von o- und p-Ethoxy-di-substituierten Oxaniliden.

2.8. 2-(2-Hydroxyphenyl)-1,3,5-triazine, wie z.B. 2,4,6-Tris(2-hydroxy-4-octyloxyphenyl)-1,3,5-triazin, 2-(2-Hydroxy-4-octyloxyphenyl)-4,6-bis-(2,4-dimethylphenyl)-1,3,5-triazin, 2-(2,4-Dihydroxyphenyl)-4,6-bis(2,4-dimethylphenyl)-1,3,5-triazin, 2,4-Bis-(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin, 2-[2-hydroxy-4-(2-hydroxy-3-butyloxy-propyloxy)phenyl]-4,6-bis(2,4-dimethylphenyl)-1,3,5-triazin, 2-[2-hydroxy-4-(2-hydroxy-3-octyloxy-propyloxy)phenyl]-4,6-bis(2,4-dimethylphenyl)-1,3,5-triazin.

3. Metalldesaktivatoren, wie z.B. N,N'-Diphenyloxalsäurediamid, N-Salicylal-N'-salicyloylhydrazin, N,N'-Bis-(salicyloyl)-hydrazin, N,N'-Bis-(3,5-di-tert-butyl-4-hydroxyphenylpropionyl)-hydrazin, 3-Salicyloylamino-1,2,4-triazol, Bis-(benzyliden)-oxalsäuredihydrazid, Oxanilid, Isophthalsäure-dihydrazid, Sebacinsäure-bis-phenylhydrazid, N, N'-Diacetyl-adipinsäure-dihydrazid, N,N'-Bis-salicyloyl-oxalsäure-dihydrazid, N,N'-Bis-salicyloyl-thiopropionsäure-dihydrazid.

4. Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert-butylphenyl)-phosphit, Diisodecylpentaerythrit-diphosphit, Bis-(2,4-di-tert-butylphenyl)-pentaerythritdiphosphit, Bis-(2,6-di-tert-butyl-4-methylphenyl)-pentaerythritdiphosphit, Bis-isodecyloxy-pentaerythritdiphosphit, Bis-(2,4-di-tert-butyl-6-methylphenyl)-pentaerythritdiphosphit, Bis-(2,4,6-tri-tert-butylphenyl)-pentaerythritdiphosphit, Tristearyl-sorbit-triphosphit, Tetrakis-(2,4-di-tert-butylphenyl)-4,4'-biphenylen-diphosphonit, 6-Isooctyloxy-2,4,8,10-tetra-tert-butyl-12H-dibenz[d,g]-1,3,2-dioxaphosphocin, 6-Fluor-2,4,8, 10-tetra-tert-butyl- 1 2-methyl-dibenz[d,g]-1,3,2-dioxaphosphocin, Bis-(2,4-di-tert-butyl-6-methylphenyl)-methylphosphit, Bis-(2,4-di-tert-butyl-6-methylphenyl)-ethylphosphit.

5. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.

6. Polyamidstabilisatoren, wie z.B. Kupfersalze in Kombination mit Jodiden und/oder Phosphorverbindungen und Salze des zweiwertigen Mangans.

7. Basische Co-Stabilisatoren, wie z.B. Melamin, Polyvinylpyrrolidon, Dicyandiamid, Tri-allylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Behenat, Mg-Stearat, Na-Ricinoleat, K-Palmitat, Antimonbrenzcatechinat oder Zinnbrenzcatechinat.

8. Nukleierungsmittel, wie z.B. 4-tert-Butylbenzoesäure, Adipinsäure, Diphenylessigsäure.

9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Ruß, Graphit.

10. Sonstige Zusätze, wie z.B. Weichmacher, Gleitmittel, Emulgatoren, Pigmente, Optische Aufheller, Flammschutzmittel, Antistatika, Treibmittel.

Die Costabilisatoren werden beispielsweise in Konzentrationen von 0,01 bis 10 %, bezogen auf das Gesamtgewicht des zu stabilisierenden Materials, zugesetzt.

Weitere bevorzugte Zusammensetzungen enthalten neben der Komponente (a) und den Verbindungen der Formel I zusätzlich noch weitere Additive, insbesondere phenolische Antioxidantien, Lichtschutzmittel und/oder Verarbeitungsstabilisatoren.

Besonders bevorzugte Additive sind phenolische Antioxidantien (Punkt 1 der Liste), sterisch gehinderte Amine (Punkt 2.6 der Liste), Phosphite und Phosphonite (Punkt 4 der Liste) und peroxidzerstörende Verbindungen (Punkt 5 der Liste).

Die Einarbeitung der Verbindungen der Formel I sowie gegebenenfalls weiterer Additive in das polymere, organische Material erfolgt nach bekannten Methoden, beispielsweise vor oder während der Formgebung oder auch durch Aufbringen der gelösten oder dispergierten Verbindungen auf das polymere, organische Material, gegebenenfalls unter nachträglichem Verdunsten des Lösungsmittels. Die Verbindungen der Formel I können auch in Form eines Masterbatches, der diese beispielsweise in einer Konzentration von 2,5 bis 25 Gew.-% enthält, den zu stabilisierenden Materialien zugesetzt werden.

Die Verbindungen der Formel I können auch vor oder während der Polymerisation oder vor der Vernetzung zugegeben werden.

Die Verbindungen der Formel I können in reiner Form oder in Wachsen, Oelen oder Polymeren verkapselt in das zu stabilisierende Material eingearbeitet werden.

Die Verbindungen der Formel I können auch auf das zu stabilisierende Polymer aufgesprüht werden. Sie sind in der Lage, andere Zusätze (z.B. die oben angegebenen herkömmlichen Additive) bzw. deren Schmelzen zu verdünnen, so dass sie auch zusammen mit diesen Zusätzen auf das zu stabilisierende Polymer aufgesprüht werden können. Besonders vorteilhaft ist die Zugabe durch Aufsprühen während der Desaktivierung der Polymerisationskatalysatoren,

wobei z.B. der zur Desaktivierung verwendete Dampf zum Versprühen verwendet werden kann.

Bei kugelförmig polymerisierten Polyolefinen kann es z.B. vorteilhaft sein, die Verbindungen der Formel I, gegebenenfalls zusammen mit anderen Additiven, durch Aufsprühen zu applizieren.

Die so stabilisierten Materialien können in verschiedenster Form angewendet werden, z.B. als Folien, Fasern, Bändchen, Formmassen, Profile oder als Bindemittel für Lacke, Klebstoffe oder Kitte.

Die vorliegende Erfindung betrifft auch ein Verfahren zum Stabilisieren eines organischen Materials gegen oxidativen, thermischen oder lichtinduzierten Abbau, das dadurch gekennzeichnet ist, dass man diesem mindestens eine Verbindung der Formel I einverleibt oder auf dieses aufbringt.

Wie bereits hervorgehoben, werden die erfindungsgemässen Verbindungen besonders vorteilhaft als Stabilisatoren in Polyolefinen eingesetzt, vor allem als Thermostabilisatoren. Ausgezeichnete Stabilisierung wird z.B. dann erhalten, wenn man sie in Kombination mit organischen Phosphiten oder Phosphoniten einsetzt. Dabei weisen die erfindungsgemässen Verbindungen den Vorteil auf, dass sie bereits in ausserordentlich geringen Mengen wirksam sind. Sie werden z.B. in Mengen von 0,0001 bis 0,050, insbesondere 0,0001 bis 0,015 Gew. % bezogen auf das Polyolefin, eingesetzt. Das organische Phosphit oder Phosphonit wird zweckmässig in einer Menge von 0,01 bis 2, insbesondere 0,01 bis 1 Gew. %, ebenfalls bezogen auf das Polyolefin, eingesetzt. Als organische Phosphite bzw. Phosphonite werden vorzugsweise solche eingesetzt, wie sie in der deutschen Offenlegungsschrift DE-A- 42 02 276 beschrieben sind. Siehe dort insbesondere die Patentansprüche, die Beispiele sowie die Seiten 4, letzter Absatz bis Seite 8. Besonders zweckmässige Phosphite und Phosphonite sind auch Punkt 4 der obigen Auflistung von Costabilisatoren zu entnehmen.

Eine speziell ausgezeichnete Stabilisierung von Polyolefinen wird beispielsweise dann erhalten, wenn die erfindungsgemässen Verbindungen in einer Dreierkombination mit organischen Phosphiten oder Phosphoniten und einem phenolischen Antioxidans eingesetzt werden.

Ebenfalls Gegenstand der Erfindung sind neue Verbindungen der Formel Ia

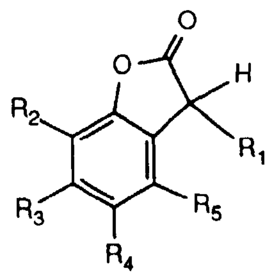

(Ia)

worin

$R_1$ Halogen oder -OR'$_1$ darstellt,

R'$_1$ Wasserstoff, $C_1$-$C_{25}$-Alkanoyl, $C_3$-$C_{25}$-Alkenoyl, durch Sauerstoff, Schwefel oder >N-$R_6$ unterbrochenes $C_3$-$C_{25}$-Alkanoyl; $C_6$-$C_9$-Cycloalkylcarbonyl, Thenoyl, Furoyl, Benzoyl oder durch $C_1$-$C_{12}$-Alkyl substituiertes Benzoyl; Naphtoyl oder durch $C_1$-$C_{12}$-Alkyl substituiertes Naphtoyl; $C_1$-$C_{25}$-Alkansulfonyl, durch Fluor substituiertes $C_1$-$C_{25}$-Alkansulfonyl; Phenylsulfonyl oder durch $C_1$-$C_{12}$-Alkyl substituiertes Phenylsulfonyl;

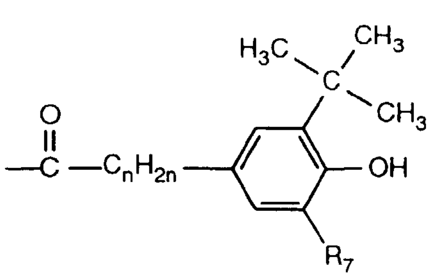 , 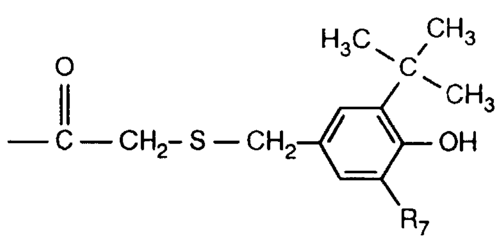 ,

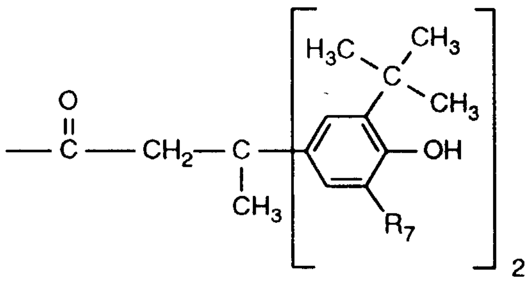, 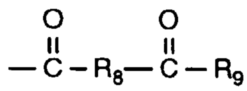 oder 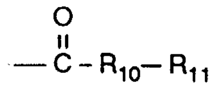

bedeutet,

$R_2$, $R_3$, $R_4$ und $R_5$ unabhängig voneinander Wasserstoff, Chlor, Hydroxy, $C_1$-$C_{25}$-Alkyl, $C_7$-$C_9$-Phenylalkyl, unsubstituiertes oder durch $C_1$-$C_4$-Alkyl substituiertes Phenyl, unsubstituiertes oder durch $C_1$-$C_4$-Alkyl substituiertes $C_5$-$C_8$-Cycloalkyl; $C_1$-$C_{18}$-Alkoxy, $C_1$-$C_{18}$-Alkylthio, $C_1$-$C_4$-Alkylamino, Di-($C_1$-$C_4$-alkyl)amino, $C_1$-$C_{25}$-Alkanoyloxy, $C_1$-$C_{25}$-Alkanoylamino, $C_3$-$C_{25}$-Alkenoyloxy, durch Sauerstoff, Schwefel oder $>$N-$R_6$ unterbrochenes $C_3$-$C_{25}$-Alkanoyloxy; $C_6$-$C_9$-Cycloalkylcarbonyloxy, Benzoyloxy oder durch $C_1$-$C_{12}$-Alkyl substituiertes Benzoyloxy darstellen, oder ferner die Reste $R_2$ und $R_3$ oder die Reste $R_3$ und $R_4$ oder die Reste $R_4$ und $R_5$ zusammen mit den Kohlenstoffatomen, an die sie gebunden sind, einen Benzoring bilden; mit der Bedingung, dass mindestens einer der Reste $R_2$, $R_3$, $R_4$ oder $R_5$ von Wasserstoff verschieden ist; wenn $R_1$ Halogen oder Hydroxy bedeutet, $R_2$ von Wasserstoff verschieden ist; und wenn $R_2$ Methyl oder Methylamin darstellt, $R_4$ verschieden von Wasserstoff und Hydroxy ist; $R_4$ zusätzlich $-(CH_2)_p-COR_9$ oder $-(CH_2)_qOH$ darstellt, oder wenn $R_3$ und $R_5$ Wasserstoff sind, $R_4$ zusätzlich einen Rest der Formel IIa

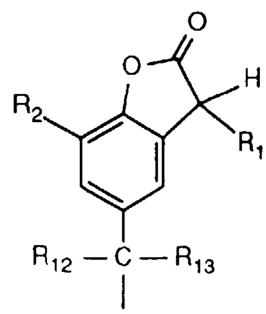

(IIa)

bedeutet,

$R_6$ Wasserstoff oder $C_1$-$C_8$-Alkyl darstellt,

$R_7$ Wasserstoff oder $C_1$-$C_8$-Alkyl bedeutet,

$R_8$ eine direkte Bindung, $C_1$-$C_{18}$-Alkylen, durch Sauerstoff, Schwefel oder $>$N-$R_6$ unterbrochenes $C_2$-$C_{18}$-Alkylen; $C_2$-$C_{18}$-Alkenylen, $C_2$-$C_{20}$-Alkyliden, $C_7$-$C_{20}$-Phenylalkyliden, $C_5$-$C_8$-Cycloalkylen, $C_7$-$C_8$-Bicycloalkylen, unsubstituiertes oder durch $C_1$-$C_4$-Alkyl substituiertes Phenylen,

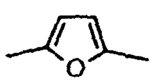 oder 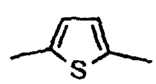

darstellt,

$R_9$ Hydroxy, $[-O^{\ominus} \frac{1}{r} M^{r+}]$, $C_1$-$C_{18}$-Alkoxy oder

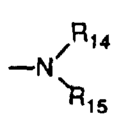

bedeutet,

$R_{10}$ Sauerstoff, -NH- oder

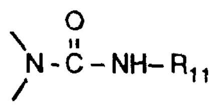

darstellt,

$R_{11}$ $C_1$-$C_{18}$-Alkyl oder Phenyl ist,

$R_{12}$ und $R_{13}$ unabhängig voneinander Wasserstoff, $CF_3$, $C_1$-$C_{12}$-Alkyl oder Phenyl darstellen, oder $R_{12}$ und $R_{13}$ zusammen mit dem C-Atom, an das sie gebunden sind, einen unsubstituierten oder durch 1 bis 3 $C_1$-$C_4$-Alkyl substituierten $C_5$-$C_8$-Cycloalkylidenring bilden;

$R_{14}$ und $R_{15}$ unabhängig voneinander Wasserstoff oder $C_1$-$C_{18}$-Alkyl darstellen,

M ein r-wertiges Metallkation ist,

n 0, 1 oder 2 bedeutet,

p 0, 1 oder 2 darstellt,

q 1, 2, 3, 4, 5 oder 6 bedeutet und

r 1, 2 oder 3 ist.

Bevorzugte Gruppen von neuen Verbindungen der Formel Ia entsprechen den in den oben für die erfindungsgemässen Zusammensetzungen ausgedrückten Bevorzugungen.

Bevorzugt sind ausserdem Verbindungen der Formel Ia, worin $R_3$ und $R_5$ Wasserstoff sind.

Besonders bevorzugt sind Verbindungen der Formel Ia, worin

$R_1$ Chlor, Brom oder -OR'$_1$ darstellt,

R'$_1$ Wasserstoff, $C_1$-$C_{12}$-Alkanoyl, durch Sauerstoff unterbrochenes $C_3$-$C_{12}$-Alkanoyl; Cyclohexylcarbonyl, Benzoyl, Naphtoyl, $C_1$-$C_{12}$-Alkansulfonyl, durch Fluor substituiertes $C_1$-$C_{12}$-Alkansulfonyl; Phenylsulfonyl oder durch $C_1$-$C_4$-Alkyl substituiertes Phenylsulfonyl;

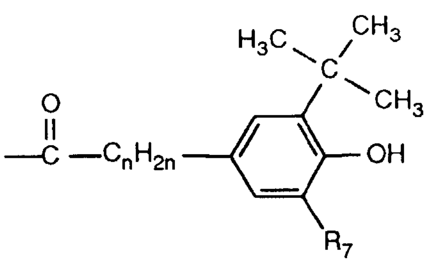 , 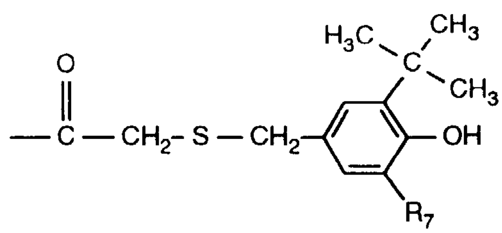 ,

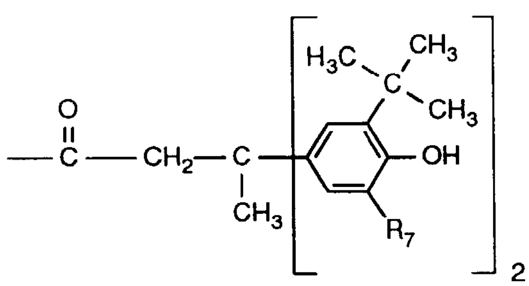 , 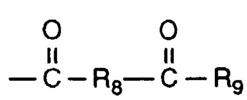 oder 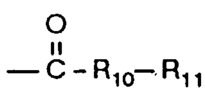

bedeutet,

$R_2$, $R_3$, $R_4$ und $R_5$ unabhängig voneinander Wasserstoff, Chlor, Hydroxy, $C_1$-$C_{18}$-Alkyl, $C_7$-$C_9$-Phenylalkyl, Phenyl, $C_5$-$C_8$-Cycloalkyl, $C_1$-$C_6$-Alkoxy, Cyclohexylcarbonyloxy oder Benzoyloxy darstellen, oder ferner die Reste $R_2$ und $R_3$ oder die Reste $R_3$ und $R_4$ oder die Reste $R_4$ und $R_5$ zusammen mit den Kohlenstoffatomen, an die sie gebunden sind, einen Benzoring bilden; mit der Bedingung, dass mindestens einer der Reste $R_2$, $R_3$, $R_4$ oder $R_5$ von Wasserstoff verschieden ist; wenn $R_1$ Hydroxy, Chlor oder Brom bedeutet, $R_2$ von Wasserstoff verschieden ist; und wenn $R_2$ Methyl oder Hydroxy darstellt, $R_4$ verschieden von Wasserstoff und Hydroxy ist; $R_4$ zusätzlich -$(CH_2)_p$-$COR_9$ darstellt, oder wenn $R_3$ und $R_5$ Wasserstoff sind, $R_4$ zusätzlich einen Rest der Formel IIa

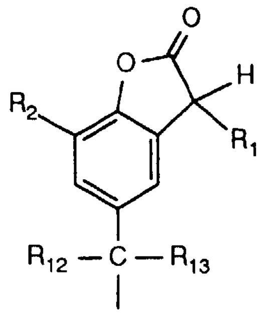

(IIa)

bedeutet,

$R_7$ Wasserstoff oder $C_1$-$C_4$-Alkyl darstellt,

$R_8$ $C_1$-$C_{12}$-Alkylen oder Phenylen bedeutet,

$R_9$ Hydroxy oder $C_1$-$C_8$-Alkoxy darstellt,

$R_{10}$ -NH- ist,

$R_{11}$ $C_1$-$C_8$-Alkyl oder Phenyl bedeutet und

$R_{12}$ und $R_{13}$ Methylgruppen sind oder zusammen mit dem C-Atom, an das sie gebunden sind, einen $C_5$-$C_8$-Cydoalkylidenring bilden.

Speziell bevorzugt sind Verbindungen der Formel Ia, worin

$R_1$ Chlor oder -OR'$_1$ darstellt,

R'$_1$ Wasserstoff, $C_1$-$C_8$-Alkanoyl, Benzoyl, Methansulfonyl, Trifluormethansulfonyl; Phenylsulfonyl oder durch $C_1$-$C_4$-Alkyl substituiertes Phenylsulfonyl;

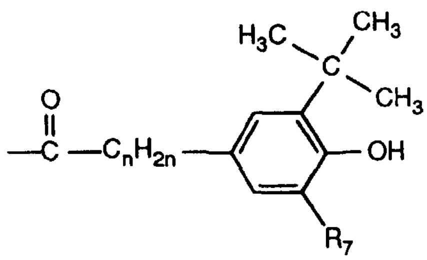

oder

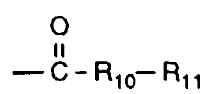

bedeutet,

$R_2$, $R_3$, $R_4$ und $R_5$ unabhängig voneinander Wasserstoff, Chlor, $C_1$-$C_{18}$-Alkyl, $C_7$-$C_9$-Phenylalkyl, Phenyl, Cyclohexyl, $C_1$-$C_6$-Alkoxy darstellen; mit der Bedingung, dass mindestens einer der Reste $R_2$, $R_3$, $R_4$ oder $R_5$ von Wasserstoff verschieden ist; wenn $R_1$ Hydroxy oder Chlor bedeutet, $R_2$ von Wasserstoff verschieden ist; und wenn $R_2$ Methyl darstellt, $R_4$ verschieden von Wasserstoff ist; $R_4$ zusätzlich -$(CH_2)_p$-$COR_9$ darstellt, oder wenn $R_3$ und $R_5$ Wasserstoff sind, $R_4$ zusätzlich einen Rest der Formel IIa

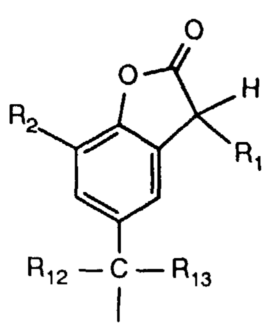

(IIa)

bedeutet,

$R_7$ Wasserstoff oder $C_1$-$C_4$-Alkyl darstellt,

$R_9$ Hydroxy oder $C_1$-$C_8$-Alkoxy bedeutet,
$R_{10}$ -NH- ist,
$R_{11}$ $C_1$-$C_4$-Alkyl oder Phenyl darstellt,
$R_{12}$ und $R_{13}$ zusammen mit dem C-Atom, an das sie gebunden sind, einen Cyclohexylidenring bilden,
n 2 bedeutet, und
p 2 ist.

Die Verbindungen der Formel I beziehungsweise Formel Ia, worin $R'_1$ Wasserstoff bedeutet, können, wie von H. Sterk et al., Monatshefte für Chemie 99, 2223 (1968) beschrieben, zum Teil in ihren tautomeren Formen der Formel Ib oder Formel Ic

(Ib) 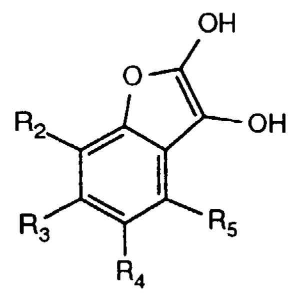 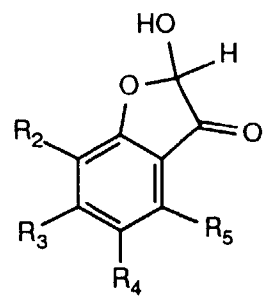 (Ic)

vorliegen. Im Rahmen dieser Anmeldung sind die Formeln I bzw. Ia in allen Fällen so zu verstehen, dass sie auch die beiden obigen tautomeren Formeln Ib und Ic mitumfassen.

Die erfindungsgemässen Verbindungen der Formel I können in Analogie zu eingangs erwähnten Literaturvorschriften auf an sich bekannte Weise hergestellt werden.

Diese bekannten Methoden sind relativ aufwendig und benötigen zum Teil teure und vom ökologischen Standpunkt aus betrachtet nicht völlig unbedenkliche Reagentien wie beispielsweise Selendioxid.

Ein weiterer Gegenstand der Erfindung ist daher auch ein neues Verfahren zur Herstellung von Verbindungen der Formel I, dadurch gekennzeichnet, dass

a) ein Aequivalent Phenol der Formel III

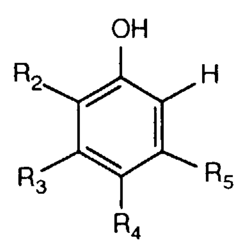 (III)

worin die allgemeinen Symbole wie in Formel I definiert sind, mit 0,8 bis 2,0 Aequivalent Glyoxylsäure, insbesondere 0,8 bis 1,2 Aequivalent Glyoxylsäure, zu einer Verbindung der Formel IV

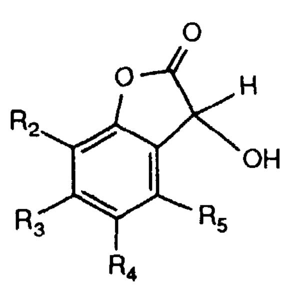 (IV)

worin die allgemeinen Symbole wie in Formel I definiert sind, umgesetzt wird, und

b) zur Herstellung von Verbindungen der Formel I, worin $R'_1$ nicht Wasserstoff bedeutet, die erhaltene Verbindung der Formel IV mit einer Halogenwasserstoffsäure, einem Halogenid einer Schwefel-Sauerstoffsäure, einem Halogenid der Phosphorsäure, einem Halogenid der phosphorigen Säure, einer Säure der Formel V

$$R'_1\text{-OH} \qquad (V)$$

einem Säurehalogenid der Formel VI

$$R'_1\text{-Y} \qquad (VI)$$

einem Ester der Formel VII

$$R'_1\text{-O-}R_{16} \qquad (VII)$$

einem symmetrischen oder unsymmetrischen Anhydrid der Formel VIII

$$R'_1\text{-O-}R'_1 \qquad (VIII)$$

oder einem Isocyanat der Formel IX

$$R_{11}\text{-N=C=O} \qquad (IX)$$

worin $R'_1$ und $R_{11}$ die obige Bedeutung haben, mit der Bedingung, dass $R'_1$ in den Verbindungen der Formeln V, VI VII und VIII nicht Wasserstoff bedeutet;
$R_{16}$ $C_1$-$C_8$-Alkyl darstellt, und
Y Fluor, Chlor, Brom oder Iod bedeutet, umgesetzt wird.

Die Glyoxylsäure kann entweder kristallin oder vorteilhaft in Form der handelsüblichen wässrigen Lösung, in der Regel 40 bis 60 % wässrigen Lösung, eingesetzt werden.
Von besonderem Interesse ist deshalb ein Verfahren zur Herstellung von Verbindungen der Formel IV, worin die Glyoxylsäure als 40 bis 60 % wässrige Glyoxylsäure, insbesondere 50 % wässrige Glyoxylsäure, verwendet wird.
Das in der Glyoxylsäure vorhandene Wasser sowie das Reaktionswasser wird während der Umsetzung abdestilliert. Dabei wird vorteilhaft ein mit Wasser azeotrop siedendes Lösungsmittel verwendet.
Geeignete mit Wasser azeotrop siedende Lösungsmittel beteiligen sich nicht an der Umsetzung und sind beispielsweise Kohlenwasserstoffe wie z.B. Cyclohexan oder Methylcyclohexan; Aromaten wie z.B. Benzol, Toluol oder Xylol; halogenierte Kohlenwasserstoffe wie z.B. 1,2-Dichlorethan; oder Aether wie z.B. Methyl-tert-butylether.
Wird die Umsetzung des Phenols der Formel III mit Glyoxylsäure ohne Lösungsmittel zu den Verbindungen der Formel IV in der Schmelze durchgeführt, wird das Reaktionswasser zweckmässig bei Normaldruck, insbesondere vorteilhaft unter leichtem Vakuum, abdestilliert.
Die Reaktion wird bevorzugt bei erhöhter Temperatur, insbesondere in einem Temperaturbereich von 60 bis 120°C, durchgeführt. Ein speziell bevorzugter Temperaturbereich ist 60 bis 90°C.
Die Umsetzung des Phenols der Formel III mit Glyoxylsäure wird bevorzugt in Gegenwart eines Katalysators durchgeführt.
Als Katalysatoren eignen sich Protonensäuren, Lewis-Säuren, Aluminiumsilikate, Ionenaustauscherharze, Zeolithe, natürlich vorkommende Schichtsilikate oder modifizierte Schichtsilikate.
Geeignete Protonensäuren sind beispielsweise Säuren von anorganischen oder organischen Salzen, wie z. B. Salzsäure, Schwefelsäure, Phosphorsäure, Methansulfonsäure, p-Toluolsulfonsäure oder Carbonsäuren wie z.B. Essigsäure. Besonders bevorzugt ist die p-Toluolsulfonsäure.
Geeignete Lewis-Säuren sind beispielsweise Zinntetrachlorid, Aluminiumchlorid, Zinkchlorid oder Bortrifluorid-etherat. Zinntetrachlorid und Aluminiumchlorid sind speziell bevorzugt.

Geeignete Aluminiumsilikate sind beispielsweise solche, die in der Petrochemie weit verbreitet sind und auch als amorphe Aluminiumsilikate bezeichnet werden. Diese Verbindungen enthalten ca. 10-30 % Siliciumoxid und 70-90 % Aluminiumoxid. Ein besonders bevorzugtes Aluminiumsilikat ist HA-HPV® von Ketjen (Akzo).

Geeignete Ionenaustauscherharze sind beispielsweise Styrol-Divinylbenzol-Harze, die noch Sulfosäuregruppen tragen, wie z.B. Amberlite 200® und Amberlyst® von Rohm und Haas oder Dowex 50® von Dow Chemicals; perfluorierte Ionenaustauscherharze, wie z.B. Nafion H® von DuPont; oder andere supersaure Ionenaustauscherharze wie von T. Yamaguchi, Applied Catalysis, 61, 1-25 (1990) oder M. Hino et al., J. Chem. Soc. Chem. Commun. 1980, 851-852 beschrieben.

Geeignete Zeolithe sind beispielsweise solche, die in der Petrochemie als Cracking-Katalysatoren weit verbreitet sind und als kristalline Silicium-Aluminiumoxide mit unterschiedlicher Kristallstruktur bekannt sind. Besonders bevorzugt sind die Faujasite der Firma Union Carbide wie beispielsweise Zeolith X®, Zeolith Y® und ultrastabiler Zeolith Y®. Zeolith Beta® und Zeolith ZSM-12® von der Firma Mobil Oil Co.; und Zeolith Mordenit® von der Firma Norton.

Geeignete natürlich vorkommende Schichtsilikate werden auch als "Saure Erden" bezeichnet und sind beispielsweise Bentonite oder Montmorillonite, die grosstechnisch abgebaut, gemahlen, mit Mineralsäuren behandelt und kalziniert werden. Besonders geeignete natürlich vorkommende Schichtsilikate sind die Fulcat®-Typen der Firma Laporte Adsorbents Co., wie z.B. Fulcat 22A®, Fulcat 22B®, Fulcat 20® oder Fulcat 40®; oder die Fulmont®-Typen der Firma Laporte Adsobents Co., wie z.B. Fulmont XMP-3® oder Fulmont XMP-4®. Ein speziell bevorzugter Katalysator ist Fulcat 22B®. Die anderen Fulcat®-Typen und Fulmont®-Typen sind aber ebenfalls in diese bevorzugte Klasse einzuordnen, weil es nur geringfügige Unterschiede zwischen den einzelnen Typen gibt, wie beispielsweise in der Anzahl der sauren Zentren.

Modifizierte Schichtsilikate werden auch als "Pillared Clays" bezeichnet und leiten sich von den oben beschriebenen natürlich vorkommenden Schichtsilikaten ab, indem sie zwischen den Silicatschichten noch Oxide von beispielsweise Zirkon, Eisen, Zink, Nickel, Chrom, Cobalt oder Magnesium enthalten. Dieser Katalysator-Typ ist in der Literatur, wie z.B. von J. Clark et al., J. Chem. Soc. Chem. Commun. 1989, 1353-1354 beschrieben, weit verbreitet, wird aber nur von sehr wenigen Firmen hergestellt. Besonders bevorzugte modifizierte Schichtsilikate sind beispielsweise Envirocat EPZ-10®, Envirocat EPZG® oder Envirocat EPIC® von der Firma Contract Chemicals.

Bevorzugte Katalysatoren sind natürlich vorkommende Schichtsilikate oder modifizierte Schichtsilikate.

Bevorzugt wird die Umsetzung des Phenols der Formel III mit Glyoxylsäure in Gegenwart eines Fulcat®-Typ-Katalysators durchgeführt.

Die Menge an Katalysator beträgt 0,01 bis 5 Mol-%, bevorzugt 0,1 bis 1,0 Mol-%, bezüglich eingesetztem Phenol der Formel III.

Die Reaktionsbedingungen für den Verfahrensschritt b) zur Herstellung von Verbindungen der Formel I, worin $R'_1$ nicht Wasserstoff bedeutet, ausgehend von Verbindungen der Formel IV, sind allgemein bekannt und können beispielsweise in Analogie zu Veresterungsvorschriften gemäss Organikum 1986, Seiten 186-191, Seite 388 und Seiten 402-408, ausgewählt werden.

Geeignete Halogenwasserstoffsäuren sind beispielsweise Salzsäure, Bromwasserstoffsäure oder Jodwasserstoffsäure. Salzsäure ist bevorzugt.

Geeignete Halogenide einer Schwefel-Sauerstoffsäure sind beispielsweise Thionylchlorid, Sulfurylchlorid oder Thionylbromid. Thionylchlorid ist bevorzugt.

Geeignete Halogenide der Phosphorsäure und der phosphorigen Säure sind beispielsweise Phosphortrichlorid, Phosphortribromid, Phosphortrijodid, Phosphorpentachlorid, Phosphoroxychlorid oder Phosphorpentafluorid. Besonders bevorzugt ist Phosphoroxychlorid.

Im Verfahrensschritt b) wird bevorzugt ein Halogenid einer Schwefel-Wasserstoffsäure, wie beispielsweise Thionylchlorid; ein Säurehalogenid der Formel VI; ein Ester der Formel VII; oder ein symmetrisches Anhydrid der Formel VIII verwendet.

Wird im Verfahrensschritt b) ein Halogenid einer Schwefel-Wasserstoffsäure wie beispielsweise Thionylchlorid verwendet, wird die Umsetzung mit einer Verbindung der Formel IV bevorzugt ohne Lösungsmittel und bei einer Temperatur von 0 bis 40°C, insbesondere Raumtemperatur durchgeführt. Das Thionylchlorid wird zweckmässig in einem 2 bis 10 fachen, insbesondere 2 bis 6 fachen Ueberschuss bezüglich eingesetzter Verbindung der Formel IV verwendet. Die Reaktion kann auch in Gegenwart eines Katalysators wie beispielsweise Dimethylformamid durchgeführt werden.

Wird im Verfahrensschritt b) ein Säurehalogenid der Formel VI ($R'_1$-Y) verwendet, worin Y bevorzugt Chlor oder Brom, insbesondere Chlor, bedeutet, wird die Umsetzung mit einer Verbindung der Formel IV bevorzugt in Gegenwart eines Lösungsmittels und einer Base durchgeführt.

Die Base kann in unterschiedlichen Mengen eingesetzt werden, von katalytischen über stöchiometrische Mengen bis hin zu mehrfachem molarem Überschuß bezüglich der Verbindung der Formel IV. Der beispielsweise bei der Reaktion gebildete Chlorwasserstoff wird gegebenenfalls durch die Base in Chlorid überführt, das durch Filtration

und/oder Waschen mit einer geeigneten wässrigen oder festen Phase entfernt werden kann; dabei kann auch ein zweites, nicht mit Wasser mischbares Lösungsmittel eingesetzt werden. Die Reinigung des Produktes erfolgt zweckmäßig durch Umkristallisation des Rückstandes der eingeengten oder zur Trockne eingedampften organischen Phase.

Geeignete Lösungsmittel zur Durchführung der Reaktion sind u.a. Kohlenwasserstoffe (beispielsweise Toluol, Xylol, Hexan, Pentan oder weitere Petroletherfraktionen), halogenierte Kohlenwasserstoffe (beispielsweise Di- oder Trichlormethan, 1,2-Dichlorethan, 1,1,1-Trichlorethan), Ether (z.B. Diethylether, Dibutylether oder Tetrahydrofuran), ferner Acetonitril, Dimethylformamid, Dimethylsulfoxid, N-Methylpyrrolidon.

Geeignete Basen sind u.a. tertiäre Amine, z.B. Trimethylamin, Triethylamin, Tributylamin, N,N-Dimethylanilin, N,N-Diethylanilin; Pyridine; Hydride (z.B. Lithium-, Natrium-, Kaliumhydrid) oder Alkoholate (z.B. Natriummethylat).

Wird im Verfahrensschritt b) ein Ester der Formel VII ($R'_1$-O-$R_{16}$) verwendet, worin $R_{16}$ bevorzugt $C_1$-$C_4$-Alkyl, insbesondere Methyl oder Ethyl bedeutet, wird die Umsetzung mit einer Verbindung der Formel IV bevorzugt in Gegenwart eines Lösungsmittels, das mit Alkoholen azeotrope Gemische bildet, durchgeführt. Der bei der Reaktion entstehende Alkohol ($R_{16}$-OH) kann kontinuierlich abdestilliert werden.

Geeignete mit Alkoholen azeotrop siedende Lösungsmittel beteiligen sich nicht an der Umsetzung und sind beispielsweise Kohlenwasserstoffe wie z.B. Cyclohexan; Aromaten wie z.B. Benzol oder Toluol; halogenierte Kohlenwasserstoffe wie z.B. 1,2-Dichlorethan; oder Aether wie z.B. Methyl-tert-butylether.

Die Reaktion kann durch Zusatz einer geringen Menge einer Protonensäure wie beispielsweise p-Toluolsulfonsäure, Methansulfonsäure, Schwefelsäure oder Salzsäure; sowie einer Lewis-Säure wie beispielsweise Bortrifluorid-etherat oder Aluminiumchlorid katalysiert werden.

Wird im Verfahrensschritt b) ein symmetrisches Anhydrid der Formel VIII ($R'_1$-O-$R'_1$) verwendet, worin $R'_1$ bevorzugt $C_2$-$C_6$-Alkanoyl, insbesondere Acetyl, bedeutet, wird die Umsetzung mit einer Verbindung der Formel IV bevorzugt ohne Zugabe eines weiteren Lösungsmittels und bei einer Temperatur von 20 bis 200°C, z. B. Siedetemperatur des Anhydrids der Formel VIII, insbesondere 60 bis 180°C, durchgeführt.

Wird im Verfahrensschritt b) ein Isocyanat der Formel IX ($R_{11}$-N=C=O) verwendet, wird die Umsetzung der Formel IV bevorzugt ohne Zugabe eines weiteren Lösungsmittels und bei einer Temperatur von 20 bis 200°C, z.B. Siedetemperatur des Isocyanats der Formel IX, insbesondere 60 bis 180°C, durchgeführt.

Die Umsetzung mit einem Isocyanat wird ebenfalls bevorzugt in Gegenwart eines Katalysators durchgeführt. Bevorzugte Katalysatoren entsprechen denjenigen, wie sie bereits für die Umsetzung des Phenols der Formel III mit Glyoxylsäure oben erwähnt werden.

Die Phenole der Formel III sind bekannt oder können nach an sich bekannten Verfahren erhalten werden.

Bisphenolverbindungen der Formel X

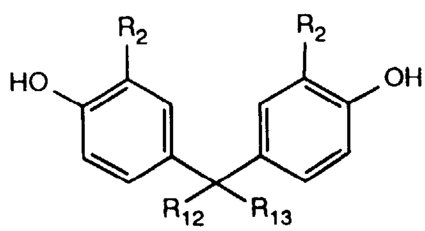

(X)

können gemäss Houben-Weyl, Methoden der organischen Chemie, Band 6/1c, 1030, hergestellt werden.

Die folgenden Beispiele erläutern die Erfindung weiter. Angaben in Teilen oder Prozenten beziehen sich auf das Gewicht.

Beispiel 1: Herstellung von 5,7-Di-tert-butyl-3-hydroxy-3H-benzofuran-2-on (Verbindung (101), Tabelle 1).

Eine Mischung von 21,2 g (0,10 Mol) 2,4-Di-tert-butyl-phenol (97 %ig), 16,3 g (0,11 Mol) 50 % wässrige Glyoxylsäure und 0,05 g (0,26 mMol) p-Toluolsulfonsäure Monohydrat in 30 ml 1,2-Dichlorethan wird unter Stickstoffatmosphäre während 3,5 Stunden am Wasserabscheider unter Rückfluss gekocht. Anschliessend wird das Reaktionsgemisch am Vakuumrotationsverdampfer eingeengt. Der Rückstand wird in 80 ml Hexan aufgenommen und dreimal mit Wasser gewaschen. Die wässrigen Phasen werden im Scheidetrichter abgetrennt und mit 30 ml Hexan nachextrahiert. Die organischen Phasen werden vereinigt, über Magnesiumsulfat getrocknet und am Vakuumrotationsverdampfer eingeengt. Der Rückstand liefert 26,23 g (~100 %) des analytisch reinen 5,7-Di-tert-butyl-3-hydroxy-3H-benzofuran-2-ons in Form eines dicken, gelblichen Harzes (Verbindung (101), Tabelle 1).

In Analogie zu Beispiel 1 werden aus den entsprechenden Phenolen wie beispielsweise 4-tert-Butyl-2-methyl-phenol, 2,4-Dicyclohexyl-phenol, 2-(Hexadec-2-yl)-4-methyl-phenol, 3-[3-tert-Butyl-4-hydroxy-phenyl]-propionsäure, 2,4-Di($\alpha$,$\alpha$-dimethylbenzyl)-phenol und 4-Methyl-2-(1,1,3,3-tetramethyl-but-l-yl)-phenol mit Glyoxylsäure die Verbindungen (103), (104), (105), (109), (110) und (111) hergestellt. Zur Herstellung der Verbindung (107) werden ausgehend vom 1,1-Bis-(3-tert-Butyl-4-hydroxy-phenyl)-cyclohexan zwei Aequivalente Glyoxylsäure eingesetzt.

Beispiel 2: Herstellung von 7-tert-Butyl-3-hydroxy-5-methyl-3H-benzofuran-2-on (Verbindung (102), Tabelle 1).

Eine Mischung von 49,8 g (0,30 Mol) 2-tert-Butyl-4-methyl-phenol, 48,9 g (0,33 Mol) 50 % wässrige Glyoxylsäure und 0,15 g (0,79 mMol) p-Toluolsulfonsäure Monohydrat in 90 ml 1,2-Dichlorethan wird unter Stickstoffatmosphäre während 3,5 Stunden am Wasserabscheider unter Rückfluss gekocht. Das Reaktionsgemisch wird anschliessend auf +5°C abgekühlt und der ausgefallene Niederschlag filtriert und mit kaltem 1,2-Dichloräthan gewaschen. Trocknen des Filterrückstandes im Hochvakuum bei Raumtemperatur liefert 54,0 g (82 %) 7-tert-Butyl-3-hydroxy-5-methyl-3H-benzofuran-2-on, Smp. 152-160°C (Verbindung (102), Tabelle 1).

Beispiel 3: Herstellung von 3-Acetoxy-5,7-di-tert-butyl-3H-benzofuran-2-on (Verbindung (106), Tabelle 1).

Eine Mischung von 21,2 g (0,10 Mol) 2,4-Di-tert-butyl-phenol (97 %ig), 16,3 g (0,11 Mol) 50 % wässrige Glyoxylsäure und 0,05 g (0,26 mMol) p-Toluolsulfonsäure Monohydrat in 30 ml 1,2-Dichlorethan wird unter Stickstoffatmosphäre während 3,5 Stunden am Wasserabscheider unter Rückfluss gekocht. Anschliessend wird das Reaktionsgemisch am Vakuumrotationsverdampfer eingeengt. Der Rückstand wird mit 9,9 ml (0,105 Mol) Essigsäureanhydrid versetzt und während 90 Minuten unter Rückluss gekocht. Anschliessend wird das Reaktionsgemisch auf Raumtemperatur abgekühlt, mit 100 ml tert-Butyl-methylether verdünnt und nacheinander mit Wasser und verdünnter Natriumbicarbonat-Lösung gewaschen. Die wässrigen Phasen werden abgetrennt und mit 50 ml tert-Butyl-methylether nachextrahiert. Die organischen Phasen werden vereinigt, über Magnesiumsulfat getrocknet und am Vakuumrotationsverdampfer eingeengt. Chromatographie des Rückstandes an Kieselgel mit dem Laufmittelsystem Dichlormethan/Hexan = 2:1 liefert 28,0 g (92 %) des 3-Acetoxy-5,7-di-tert-butyl-3H-benzofuran-2-ons als dickes, rötliches Harz (Verbindung (106), Tabelle 1).

Beispiel 4: Herstellung von 7-tert-Butyl-3-chlor-5-methyl-3H-benzofuran-2-on (Verbindung (108), Tabelle 1).

Eine Suspension von 2,2 g (10,0 mMol) 7-tert-Butyl-3-hydroxy-5-methyl-3H-benzofuran-2-on (Verbindung (102), Beispiel 2, Tabelle 1) in 2,4 ml (55,0 mMol) Thionylchlorid wird mit einem Tropfen Dimethylformamid versetzt und 2 Stunden bei Raumtemperatur gerührt. Das überschüssige Thionylchlorid wird anschliessend am Vakuumrotationsverdampfer abdestilliert. Chromatographie des Rückstandes an Kieselgel mit dem Laufmittelsystem Dichlormethan/Hexan = 1:1 und Kristallisation der reinen Fraktionen aus Methanol liefert 0,30 g (13 %) 7-tert-Butyl-3-chlor-5-methyl-3H-benzofuran-2-on, Smp. 81-86°C (Verbindung (108), Tabelle 1).

Beispiel 5: Herstellung von 3-(N-Methylcarbamoyloxy)-5-methyl-7-tert-butyl-3H-benzofuran-2-on (Verbindung (112), Tabelle 1).

Eine Mischung von 5,5 g (25,0 mMol) 7-tert-Butyl-3-hydroxy-5-methyl-3H-benzofuran-2-on (Verbindung (102), Beispiel 2), 3 ml (50,0 mMol) Methylisocyanat und 2 Tropfen Methansulfonsäure werden 3 1/4 Stunden unter Rückfluss gekocht. Anschliessend werden nochmals 3 ml (50,0 mMol) Methylisocyanat und 2 Tropfen Methansulfonsäure zugegeben. Nach weiteren 16 Stunden Rückfluss wird das Reaktionsgemisch abgekühlt, mit Dichlormethan verdünnt und mit Wasser und 5 % wässriger Natriumhydrogencarbonat-Lösung gewaschen. Die organischen Phasen werden vereiningt, über Magnesiumsulfat getrocknet und Vakuumrotationsverdampfer eingeengt. Kristallisations des Rückstandes aus Toluol liefert 4,45 g (65 %) 3-(N-Methylcarbamoyloxy)-5-methyl-7-tert-butyl-3H-benzofuran-2-on, Smp. 138-143°C (Verbindung (112), Tabelle 1).

Beispiel 6: Herstellung von 3-[3-(3,5-Di-tert-butyl-4-hydroxyphenyl)propionyloxy]-7-tert-butyl-5-methyl-3H-benzofuran-2-on (Verbindung (113), Tabelle 1).

Eine Lösung von 5,5 g (25,0 mMol) 7-tert-Butyl-3-hydroxy-5-methyl-3H-benzofuran-2-on (Verbindung (102), Beispiel 2), und 3,9 ml (28,0 mMol) Triethylamin in 30 ml Dichlormethan wird zu einer Lösung von 8,32 g (28,0 mMol) 3-(3,5-Di-tert-butyl-4-hydroxyphenyl)propionsäurechlorid (hergestellt aus 7,8 g (28,0 mMol) 3-(3,5-Di-tert-butyl-4-hydroxyphenyl)propionsäure und Thionylchlorid) in 10 ml Dichlormethan getropft. Das Reaktionsgemisch wird anschliessend 1 Stunde unter Rückfluss gekocht, dann abgekühlt und mit Wasser und 5 % wässriger Natriumhydrogencarbonat-Lösung gewaschen. Die organischen Phasen werden vereiningt, über Magnesiumsulfat getrocknet und Vakuumrotationsverdampfer eingeengt. Dreimalige Kristallisation des Rückstandes aus Acetonitril liefert 4,6 g (38 %) 3-[3-(3,5-Di-tert-butyl-4-hydroxyphenyl)propionyloxy]-7-tert- butyl-5-methyl-3H-benzofuran-2-on, Smp. 151-154°C (Verbindung (113), Tabelle 1).

Tabelle 1:

| Nr. | Verbindung | Smp. (°C) | C (%), H (%) (berechnet/gefunden) | Ausbeute (%) |
|---|---|---|---|---|
| 101 | 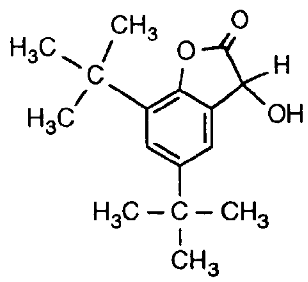 | Harz | 73,25 8,45<br>73,33 8,50 | 100 |
| 102 | 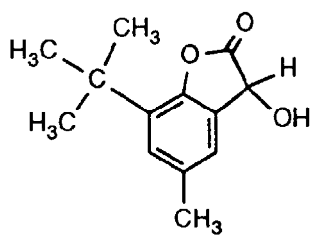 | 152-160 | 70,89 7,32<br>70,40 7,40 | 82 |
| 103 | 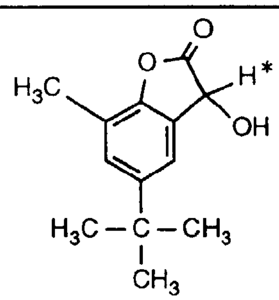 | Harz | Charakterisiert durch $^1$H-NMR ($CDCl_3$)<br>$\delta$(H*) = 5,33 ppm<br>a) Chromatographiert an Kieselgel ($CH_2Cl_2$/Hexan = 4 : 1) | 45[a)] |
| 104 | 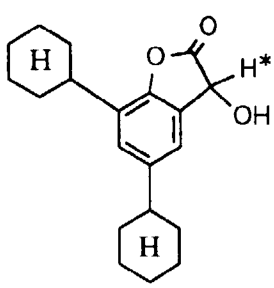 | Harz | Charakterisiert durch $^1$H-NMR ($CDCl_3$)<br>$\delta$(H*) = 5,30 ppm | ~100 |

Tabelle 1: (Fortsetzung)

| Nr. | Verbindung | Smp. (°C) | C (%), H (%) (berechnet/gefunden) | Ausbeute (%) |
|---|---|---|---|---|
| 105 | 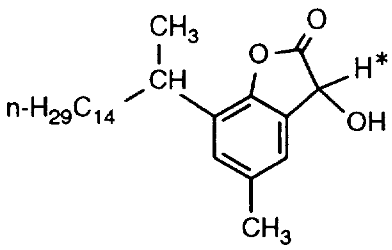 | Harz | Charakterisiert durch $^1$H-NMR ($CDCl_3$) δ(H*) = 5,31 ppm | 98 |
| 106 | 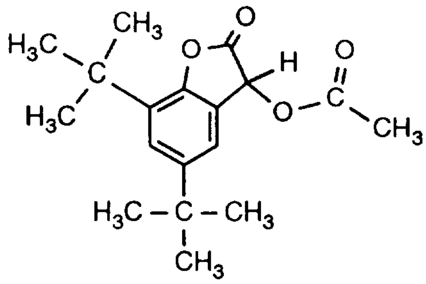 | Harz | 71,03 7,95<br>71,10 7,98 | 92 |
| 107 | 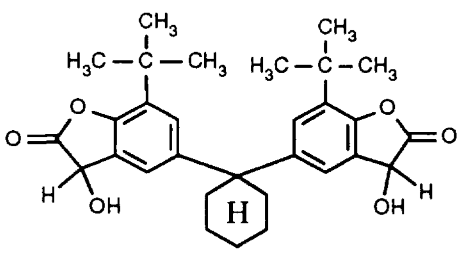 | Harz | Charakterisiert durch $^1$H-NMR ($CDCl_3$) δ(tert-Butyl) = 1,34 ppm | ~100 |
| 108 | 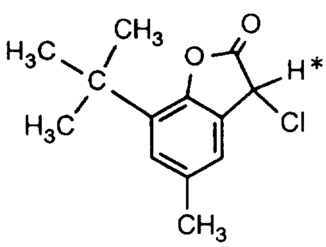 | 81-86 | Charakterisiert durch $^1$H-NMR ($CDCl_3$) δ(H*) = 5,34 ppm | 13 |

Tabelle 1: (Fortsetzung)

| Nr. | Verbindung | Smp. (°C) | C (%), H (%) (berechnet/gefunden) | Ausbeute (%) |
|---|---|---|---|---|
| 109 | 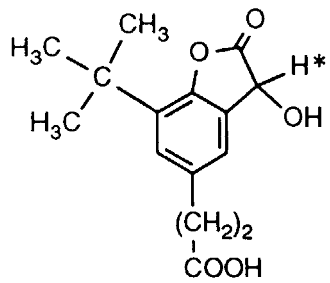 | Harz | Charakterisiert durch $^1$H-NMR ($CDCl_3$) δ(H*) = 5,29 ppm | ~100 |
| 110 | 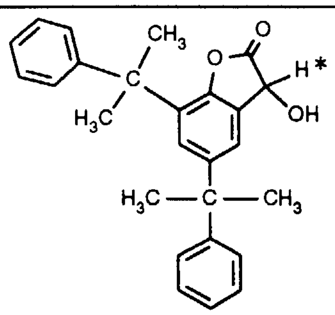 | Harz | Charakterisiert durch $^1$H-NMR ($CDCl_3$) δ(H*) = 5,08 ppm | 38 |
| 111 | 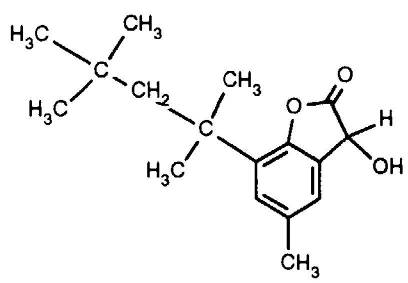 | 100-103 | 73,88 8,75<br>73,73 8,75 | 61 |
| 112 | 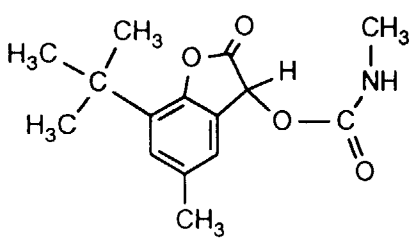 | 138-143 | 64,97 6,91<br>65,02 6,89 | 65 |

Tabelle 1: (Fortsetzung)

| Nr. | Verbindung | Smp. (°C) | C (%), H (%) (berechnet/gefunden) | Ausbeute (%) |
|---|---|---|---|---|
| 113 | 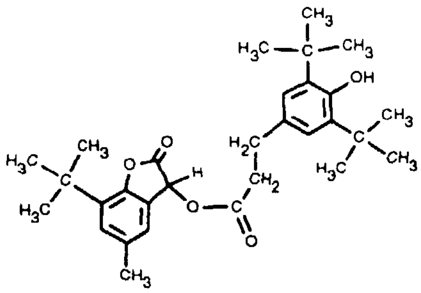 | 151-154 | 74,97 8,39<br>74,83 8,38 | 38 |

Beispiel 7: Stabilisierung von Polypropylen bei Mehrfachextrusion.

1,3 kg Polypropylenpulver (Profax 6501), das mit 0,025 % Irganox® 1076 (3-[3,5-di-tert-butyl-4-hydroxyphenyl]propionsäure-n-octadecylester) vorstabilisiert wurde, (mit einem bei 230°C und mit 2,16 kg gemessenen Schmelzindex von 3,2) werden gemischt mit 0,05 % Irganox® 1010 (Pentaerythrit-tetrakis-[3-(3,5-di-tert-butyl-4-hydroxyphenyl)-propionatl), 0,05 % Calciumstearat, 0,03 % DHT 4A® (Kyowa Chemical Industry Co., Ltd., [$Mg_{4.5}Al_2(OH)_{13}CO_3$•3,5 $H_2O$]) und 0,05 % Verbindung aus Tabelle 1. Diese Mischung wird in einem Extruder mit einem Zylinderdurchmesser von 20 mm und einer Länge von 400 mm mit 100 Umdrehungen pro Minute extrudiert, wobei die 3 Heizzonen auf die folgenden Temperaturen eingestellt werden: 260, 270, 280°C. Das Extrudat wird zur Kühlung durch ein Wasserbad gezogen und anschliessend granuliert. Dieses Granulat wird wiederholt extrudiert. Nach 3 Extrusionen wird der Schmelzindex gemessen (bei 230°C mit 2,16 kg). Grosse Zunahme des Schmelzindex bedeutet starken Kettenabbau, also schlechte Stabilisierung. Die Resultate sind in Tabelle 2 zusammengefasst.

Tabelle 2:

| Verbindung aus Tabelle 1 | Schmelzindex nach 3 Extrusionen |
|---|---|
| — | 21,3 |
| 101 | 7,1 |
| 102 | 6,6 |
| 106 | 8,4 |

**Patentansprüche**

**1.** Zusammensetzung enthaltend

a) ein dem oxidativen, thermischen oder lichtinduzierten Abbau unterworfenes organisches Material und
b) mindestens eine Verbindung der Formel I

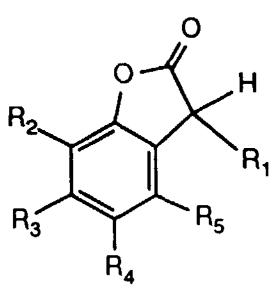 (I)

worin

$R_1$ Halogen oder -OR'$_1$ darstellt,

R'$_1$ Wasserstoff, $C_1$-$C_{25}$-Alkanoyl, $C_3$-$C_{25}$-Alkenoyl, durch Sauerstoff, Schwefel oder $>$N-$R_6$ unterbrochenes $C_3$-$C_{25}$-Alkanoyl; $C_6$-$C_9$-Cycloalkylcarbonyl, Thenoyl, Furoyl, Benzoyl oder durch $C_1$-$C_{12}$-Alkyl substituiertes Benzoyl; Naphtoyl oder durch $C_1$-$C_{12}$-Alkyl substituiertes Naphtoyl; $C_1$-$C_{25}$-Alkansulfonyl, durch Fluor substituiertes $C_1$-$C_{25}$-Alkansulfonyl; Phenylsulfonyl oder durch $C_1$-$C_{12}$-Alkyl substituiertes Phenylsulfonyl;

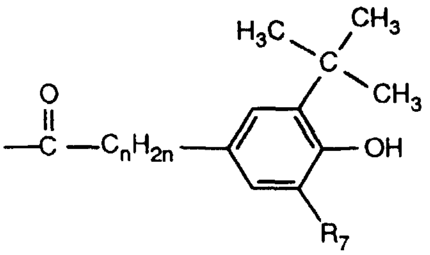 , 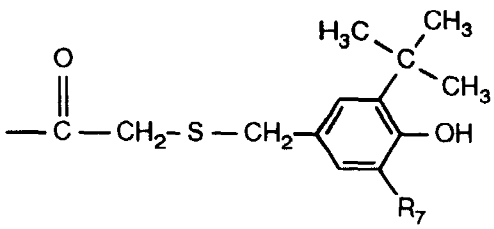 ,

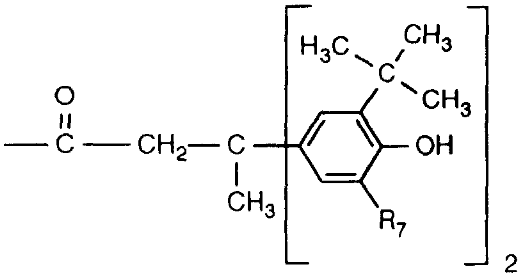 , 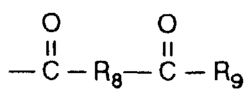 oder 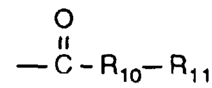

bedeutet,

$R_2$, $R_3$, $R_4$ und $R_5$ unabhängig voneinander Wasserstoff, Chlor, Hydroxy, $C_1$-$C_{25}$-Alkyl, $C_7$-$C_9$-Phenylalkyl, unsubstituiertes oder durch $C_1$-$C_4$-Alkyl substituiertes Phenyl, unsubstituiertes oder durch $C_1$-$C_4$-Alkyl substituiertes $C_5$-$C_8$-Cycloalkyl; $C_1$-$C_{18}$-Alkoxy, $C_1$-$C_{18}$-Alkylthio, $C_1$-$C_4$-Alkylamino, Di-($C_1$-$C_4$-alkyl)amino, $C_1$-$C_{25}$-Alkanoyloxy, $C_1$-$C_{25}$-Alkanoylamino, $C_3$-$C_{25}$-Alkenoyloxy, durch Sauerstoff, Schwefel oder $>$N-$R_6$ unterbrochenes $C_3$-$C_{25}$-Alkanoyloxy; $C_6$-$C_9$-Cycloalkylcarbonyloxy, Benzoyloxy oder durch $C_1$-$C_{12}$-Alkyl substituiertes Benzoyloxy darstellen, oder ferner die Reste $R_2$ und $R_3$ oder die Reste $R_3$ und $R_4$ oder die Reste $R_4$ und $R_5$ zusammen mit den Kohlenstoffatomen, an die sie gebunden sind, einen Benzoring bilden, $R_4$ zusätzlich -$(CH_2)_p$-$COR_9$ oder -$(CH_2)_q$OH darstellt, oder wenn $R_3$ und $R_5$ Wasserstoff sind, $R_4$ zusätzlich einen Rest der Formel II

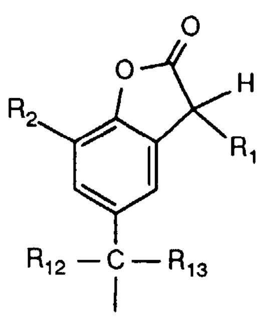 (II)

bedeutet,

$R_6$ Wasserstoff oder $C_1$-$C_8$-Alkyl darstellt,

$R_7$ Wasserstoff oder $C_1$-$C_8$-Alkyl bedeutet,

$R_8$ eine direkte Bindung, $C_1$-$C_{18}$-Alkylen, durch Sauerstoff, Schwefel oder $>$N-$R_6$ unterbrochenes $C_2$-$C_{18}$-Alkylen; $C_2$-$C_{18}$-Alkenylen, $C_2$-$C_{20}$-Alkyliden, $C_7$-$C_{20}$-Phenylalkyliden, $C_5$-$C_8$-Cycloalkylen, $C_7$-$C_8$-Bicycloalkylen, unsubstituiertes oder durch $C_1$-$C_4$-Alkyl substituiertes Phenylen,

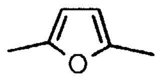 oder 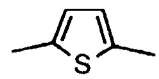

darstellt,
$R_9$ Hydroxy, $[-O^{\ominus}\frac{1}{r}M^{r+}]$, $C_1$-$C_{18}$-Alkoxy oder

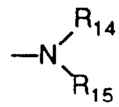

bedeutet,
$R_{10}$ Sauerstoff, -NH- oder

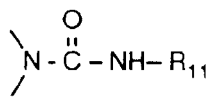

darstellt,
$R_{11}$ $C_1$-$C_{18}$-Alkyl oder Phenyl ist,
$R_{12}$ und $R_{13}$ unabhängig voneinander Wasserstoff, $CF_3$, $C_1$-$C_{12}$-Alkyl oder Phenyl darstellen, oder $R_{12}$ und $R_{13}$ zusammen mit dem C-Atom, an das sie gebunden sind, einen unsubstituierten oder durch 1 bis 3 $C_1$-$C_4$-Alkyl substituierten $C_5$-$C_8$-Cycloalkylidenring bilden;
$R_{14}$ und $R_{15}$ unabhängig voneinander Wasserstoff oder $C_1$-$C_{18}$-Alkyl darstellen,
M ein r-wertiges Metallkation ist,
n 0, 1 oder 2 bedeutet,
p 0, 1 oder 2 darstellt,
q 1, 2, 3, 4, 5 oder 6 bedeutet, und
r 1, 2 oder 3 ist.

**2.** Zusammensetzung gemäss Anspruch 1, worin
R'$_1$ Wasserstoff, $C_1$-$C_{18}$-Alkanoyl, $C_3$-$C_{18}$-Alkenoyl, durch Sauerstoff, Schwefel oder $>N-R_6$ unterbrochenes $C_3$-$C_{18}$-Alkanoyl; $C_6$-$C_8$-Cycloalkylcarbonyl, Thenoyl, Furoyl, Benzoyl oder durch $C_1$-$C_8$-Alkyl substituiertes Benzoyl; Naphtoyl oder durch $C_1$-$C_8$-Alkyl substituiertes Naphtoyl; $C_1$-$C_{18}$-Alkansulfonyl, durch Fluor substituiertes $C_1$-$C_{18}$-Alkansulfonyl; Phenylsulfonyl oder durch $C_1$-$C_8$-Alkyl substituiertes Phenylsulfonyl;

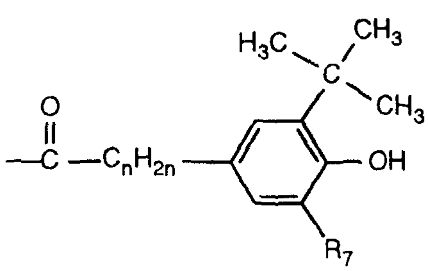 , 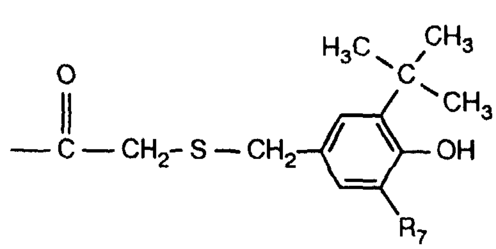 ,

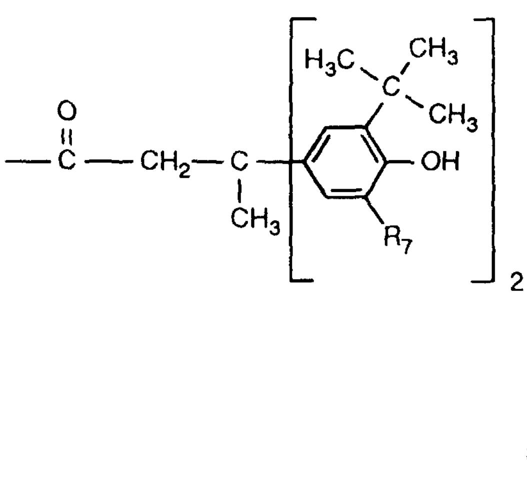 , 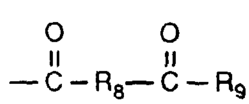 oder 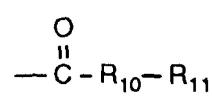

bedeutet,
$R_2$, $R_3$, $R_4$ und $R_5$ unabhängig voneinander Wasserstoff, Chlor, Hydroxy, $C_1$-$C_{25}$-Alkyl, $C_7$-$C_9$-Phenylalkyl, unsubstituiertes oder durch $C_1$-$C_4$-Alkyl substituiertes Phenyl, unsubstituiertes oder durch $C_1$-$C_4$-Alkyl substituiertes $C_5$-$C_8$-CycloaLkyl; $C_1$-$C_{12}$-Alkoxy, $C_1$-$C_{12}$-Alkylthio, $C_1$-$C_4$-Alkylamino, Di-($C_1$-$C_4$-alkyl)amino, $C_1$-$C_{18}$-Alkanoyloxy, $C_1$-$C_{18}$-Alkanoylamino, $C_3$-$C_{18}$-Alkenoyloxy, durch Sauerstoff, Schwefel oder $>$N-$R_6$ unterbrochenes $C_3$-$C_{18}$-Alkanoyloxy; $C_6$-$C_8$-Cycloalkylcarbonyloxy, Benzoyl oxy oder durch $C_1$-$C_8$-Alkyl substituiertes Benzoyloxy darstellen, oder ferner die Reste $R_2$ und $R_3$ oder die Reste $R_3$ und $R_4$ oder die Reste $R_4$ und $R_5$ zusammen mit den Kohlenstoffatomen, an die sie gebunden sind, einen Benzoring bilden, $R_4$ zusätzlich -$(CH_2)_p$-$COR_9$ oder $(CH_2)_q$OH darstellt, oder wenn $R_3$ und $R_5$ Wasserstoff sind, $R_4$ zusätzlich einen Rest der Formel II

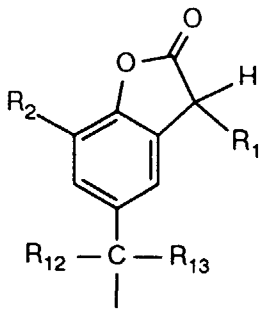

(II)

bedeutet,
$R_6$ Wasserstoff oder $C_1$-$C_6$-Alkyl darstellt,
$R_7$ Wasserstoff oder $C_1$-$C_6$-Alkyl bedeutet,
$R_8$ eine direkte Bindung, $C_1$-$C_{12}$-Alkylen, durch Sauerstoff, Schwefel oder $>$N-$R_6$ unterbrochenes $C_2$-$C_{12}$-Alkylen; $C_2$-$C_{12}$-Alkenylen, $C_2$-$C_{12}$-Alkyliden, $C_7$-$C_{12}$-Phenylalkyliden, $C_5$-$C_8$-Cycloalkylen, $C_7$-$C_8$-Bicycloalkylen oder Phenylen darstellt,
$R_9$ Hydroxy, $C_1$-$C_{12}$-Alkoxy oder

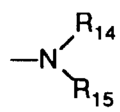

bedeutet,
$R_{10}$ Sauerstoff oder -NH- darstellt,
$R_{11}$ $C_1$-$C_{12}$-Alkyl oder Phenyl ist,
$R_{12}$ und $R_{13}$ Methylgruppen sind oder zusammen mit dem C-Atom, an das sie gebunden sind, einen unsubstituierten oder durch 1 bis 3 $C_1$-$C_4$-Alkyl substituierten $C_5$-$C_8$-Cycloalkylidenring bilden;
$R_{14}$ und $R_{15}$ unabhängig voneinander Wasserstoff oder $C_1$-$C_8$-Alkyl darstellen, und
q 2, 3, 4, 5 oder 6 bedeutet.

**3.** Zusammensetzung gemäss Anspruch 1, worin mindestens zwei der Reste $R_2$, $R_3$, $R_4$ und $R_5$ Wasserstoff sind.

**4.** Zusammensetzung gemäss Anspruch 1, worin $R_3$ und $R_5$ Wasserstoff sind.

**5.** Zusammensetzung gemäss Anspruch 1, worin
$R_1$ Chlor, Brom oder -OR'$_1$ darstellt,
R'$_1$ Wasserstoff, $C_1$-$C_{12}$-Alkanoyl, durch Sauerstoff unterbrochenes $C_3$-$C_{12}$-Alkanoyl; Cyclohexylcarbonyl, Benzoyl, Naphtoyl, $C_1$-$C_{12}$-Alkansulfonyl, durch Fluor substituiertes $C_1$-$C_{12}$-Alkansulfonyl; Phenylsulfonyl oder durch $C_1$-$C_4$-Alkyl substituiertes Phenylsulfonyl;

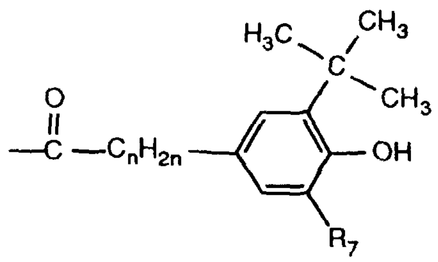 , 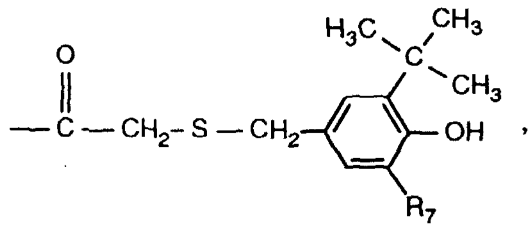 ,

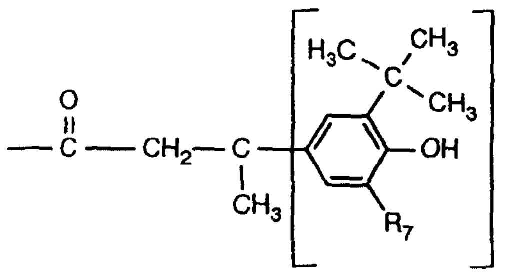 , 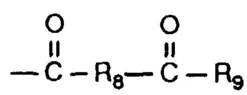 oder 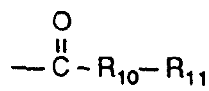

bedeutet,
$R_7$ Wasserstoff oder $C_1$-$C_6$-Alkyl darstellt,
$R_8$ eine direkte Bindung, $C_1$-$C_{12}$-Alkylen, durch Sauerstoff, Schwefel oder $>$N-$R_6$ unterbrochenes $C_2$-$C_{12}$-Alkylen; $C_2$-$C_{12}$-Alkenylen, $C_2$-$C_{12}$-Alkyliden, $C_7$-$C_{12}$-Phenylalkyliden, $C_5$-$C_8$-Cycloalkylen, $C_7$-$C_8$-Bicycloalkylen oder Phenylen bedeutet,
$R_9$ Hydroxy, $C_1$-$C_{12}$-Alkoxy oder

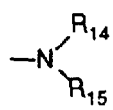

darstellt,
$R_{10}$ Sauerstoff oder -NH- bedeutet,
$R_{11}$ $C_1$-$C_{12}$-Alkyl oder Phenyl ist, und
$R_{14}$ und $R_{15}$ unabhängig voneinander Wasserstoff oder $C_1$-$C_8$-Alkyl darstellen.

**6.** Zusammensetzung gemäss Anspruch 1, worin R'$_1$ Wasserstoff, $C_1$-$C_8$-Alkanoyl, Benzoyl, Methansulfonyl, p-Methylphenylsulfonyl,

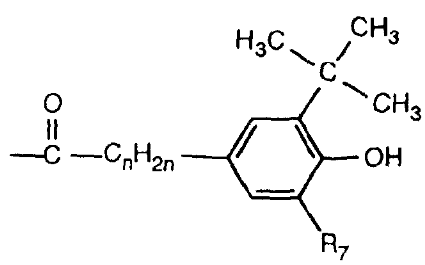

oder

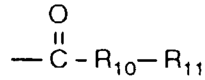

bedeutet,
$R_7$ Wasserstoff oder $C_1$-$C_4$-Alkyl darstellt,
$R_{10}$ -NH- ist,
$R_{11}$ $C_1$-$C_8$-Alkyl oder Phenyl bedeutet, und
n 2 ist.

**7.** Zusammensetzung gemäss Anspruch 1, worin
$R_2$, $R_3$, $R_4$ und $R_5$ unabhängig voneinander Wasserstoff, Chlor, Hydroxy, $C_1$-$C_{18}$-Alkyl, $C_7$-$C_9$-Phenylalkyl, Phenyl, $C_5$-$C_8$-Cycloalkyl, $C_1$-$C_6$-Alkoxy, Cyclohexylcarbonyloxy oder Benzoyloxy darstellen, oder ferner die Reste $R_2$ und $R_3$ oder die Reste $R_3$ und $R_4$ oder die Reste $R_4$ und $R_5$ zusammen mit den Kohlenstoffatomen, an die sie gebunden sind, einen Benzoring bilden, $R_4$ zusätzlich $-(CH_2)_p$-$COR_9$ darstellt, oder wenn $R_3$ und $R_5$ Wasserstoff sind, $R_4$ zusätzlich einen Rest der Formel II bedeutet,
$R_7$ Wasserstoff oder $C_1$-$C_4$-Alkyl darstellt,
$R_8$ $C_1$-$C_{12}$-Alkylen oder Phenylen bedeutet,
$R_9$ Hydroxy oder $C_1$-$C_8$-Alkoxy darstellt,
$R_{10}$ -NH- ist,
$R_{11}$ $C_1$-$C_8$-Alkyl oder Phenyl bedeutet, und
$R_{12}$ und $R_{13}$ Methylgruppen sind oder zusammen mit dem C-Atom, an das sie gebunden sind, einen $C_5$-$C_8$-Cycloalkylidenring bilden.

**8.** Zusammensetzung gemäss Anspruch 1, worin
$R_1$ Chlor oder -OR'$_1$ darstellt,
R'$_1$ Wasserstoff, $C_1$-$C_8$-Alkanoyl, Benzoyl, Methansulfonyl, Trifluormethansulfonyl; Phenylsulfonyl oder durch $C_1$-$C_4$-Alkyl substituiertes Phenylsulfonyl;

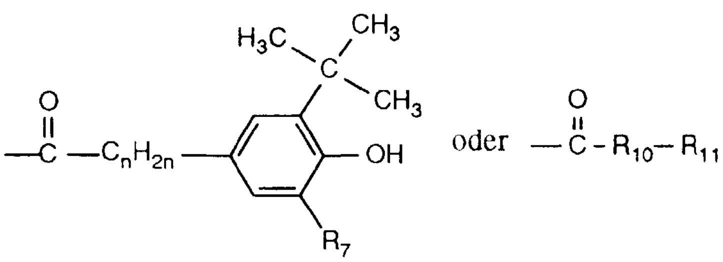

bedeutet,
$R_2$, $R_3$, $R_4$ und $R_5$ unabhängig voneinander Wasserstoff, Chlor, $C_1$-$C_{18}$-Alkyl, $C_7$-$C_9$-Phenylalkyl, Phenyl, Cyclohexyl, $C_1$-$C_6$-Alkoxy darstellen, $R_4$ zusätzlich $-(CH_2)_p$-$COR_9$ darstellt, oder wenn $R_3$ und $R_5$ Wasserstoff sind, $R_4$ zusätzlich einen Rest der Formel II

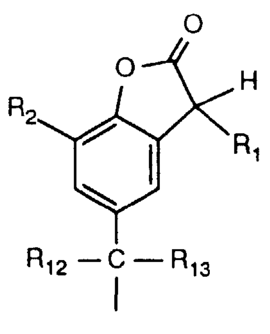

(II)

bedeutet,
$R_7$ Wasserstoff oder $C_1$-$C_4$-Alkyl darstellt,
$R_9$ Hydroxy oder $C_1$-$C_8$-Alkoxy bedeutet,
$R_{10}$ -NH- ist,
$R_{11}$ $C_1$-$C_4$-Alkyl oder Phenyl darstellt,

$R_{12}$ und $R_{13}$ zusammen mit dem C-Atom, an das sie gebunden sind, einen Cyclohexylidenring bilden,
n 2 bedeutet, und
p 2 ist.

**9.** Zusammensetzung gemäss Anspruch 1, worin
$R_1$ Chlor oder -OR'$_1$ darstellt,
R'$_1$ Wasserstoff, Acetyl

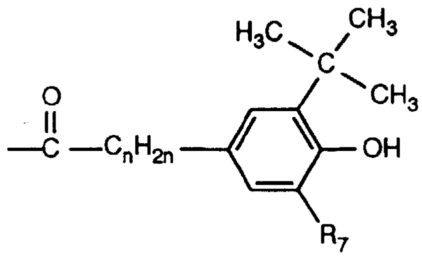 oder 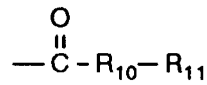

bedeutet,
$R_2$ $C_1$-$C_{16}$-Alkyl, $C_7$-$C_9$-Phenylalkyl oder Cyclohexyl darstellt,
$R_3$ Wasserstoff ist,
$R_4$ $C_1$-$C_4$-Alkyl, $C_7$-$C_9$-Phenylalkyl, Cyclohexyl, $-CH_2CH_2COOH$ oder einen Rest der Formel II

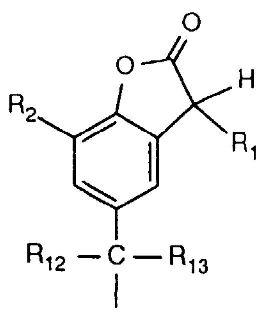 (II)

bedeutet,
$R_5$ Wasserstoff ist,
$R_7$ Wasserstoff oder $C_1$-$C_4$-Alkyl darstellt,
$R_{10}$ -NH- ist,
$R_{11}$ $C_1$-$C_4$-Alkyl bedeutet,
$R_{12}$ und $R_{13}$ zusammen mit dem C-Atom, an das sie gebunden sind, einen Cyclohexylidenring bilden, und
n 2 ist.

**10.** Zusammensetzung gemäss Anspruch 1, enthaltend neben den Komponenten (a) und (b) zusätzlich weitere Additive.

**11.** Zusammensetzung gemäss Anspruch 10, enthaltend als weitere Additive phenolische Antioxidantien, Lichtschutzmittel oder/und Verarbeitungsstabilisatoren.

**12.** Zusammensetzung gemäss Anspruch 10, enthaltend als weiteres Additiv mindestens eine Verbindung vom Typ der organischen Phosphite oder Phosphonite.

**13.** Zusammensetzung gemäss Anspruch 1, enthaltend als Komponente (a) natürliche, halbsynthetische oder synthetische Polymere.

**14.** Zusammensetzung gemäss Anspruch 1, enthaltend als Komponente (a) ein Polyolefin.

**15.** Zusammensetzung gemäss Anspruch 1, enthaltend als Komponente (a) Polyethylen oder Polypropylen.

**16.** Zusammensetzung gemäss Anspruch 1, worin die Komponente (b) in einer Menge von 0,0005 bis 5 % bezogen auf das Gewicht der Komponente (a) vorliegt.

**17.** Verbindungen der Formel Ia

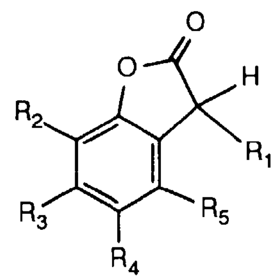

(Ia)

worin
$R_1$ Halogen oder $-OR'_1$ darstellt,
$R'_1$ Wasserstoff, $C_1$-$C_{25}$-Alkanoyl, $C_3$-$C_{25}$-Alkenoyl, durch Sauerstoff, Schwefel oder $>N-R_6$ unterbrochenes $C_3$-$C_{25}$-Alkanoyl; $C_6$-$C_9$-Cycloalkylcarbonyl, Thenoyl, Furoyl, Benzoyl oder durch $C_1$-$C_{12}$-Alkyl substituiertes Benzoyl; Naphtoyl oder durch $C_1$-$C_{12}$-Alkyl substituiertes Naphtoyl; $C_1$-$C_{25}$-Alkansulfonyl, durch Fluor substituiertes $C_1$-$C_{25}$-Alkansulfonyl; Phenylsulfonyl oder durch $C_1$-$C_{12}$-Alkyl substituiertes Phenylsulfonyl;

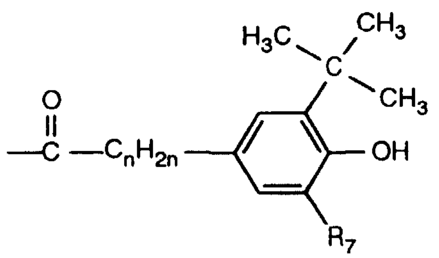 , 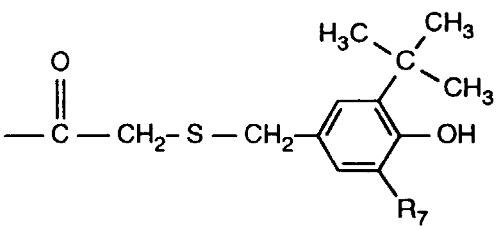 ,

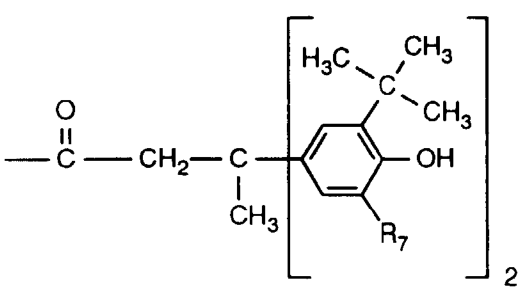 , 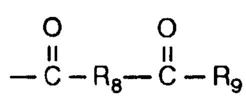 oder 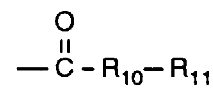

bedeutet,
$R_2$, $R_3$, $R_4$ und $R_5$ unabhängig voneinander Wasserstoff, Chlor, Hydroxy, $C_1$-$C_{25}$-Alkyl, $C_7$-$C_9$-Phenylalkyl, unsubstituiertes oder durch $C_1$-$C_4$-Alkyl substituiertes Phenyl, unsubstituiertes oder durch $C_1$-$C_4$-Alkyl substituiertes $C_5$-$C_8$-Cycloalkyl; $C_1$-$C_{18}$-Alkoxy, $C_1$-$C_{18}$-Alkylthio, $C_1$-$C_4$-Alkylamino, Di-($C_1$-$C_4$-alkyl)amino, $C_1$-$C_{25}$-Alkanoyloxy, $C_1$-$C_{25}$-Alkanoylamino, $C_3$-$C_{25}$-Alkenoyloxy, durch Sauerstoff, Schwefel oder $>N-R_6$ unterbrochenes $C_3$-$C_{25}$-Alkanoyloxy; $C_6$-$C_9$-Cycloallcylcarbonyloxy , Benzoyloxy oder durch $C_1$-$C_{12}$-Alkyl substituiertes Benzoyloxy darstellen, oder ferner die Reste $R_2$ und $R_3$ oder die Reste $R_3$ und $R_4$ oder die Reste $R_4$ und $R_5$ zusammen mit den Kohlenstoffatomen, an die sie gebunden sind, einen Benzoring bilden; mit der Bedingung, dass mindestens einer der Reste $R_2$, $R_3$, $R_4$ oder $R_5$ von Wasserstoff verschieden ist; wenn $R_1$ Halogen oder Hydroxy bedeutet, $R_2$ von Wasserstoff verschieden ist; und wenn $R_2$ Methyl oder Methylamin darstellt, $R_4$ verschieden von Wasserstoff und Hydroxy ist; $R_4$ zusätzlich $-(CH_2)_p-COR_9$ oder $(CH_2)_qOH$ darstellt, oder wenn $R_3$ und $R_5$ Wasserstoff sind, $R_4$ zusätzlich einen Rest der Formel IIa

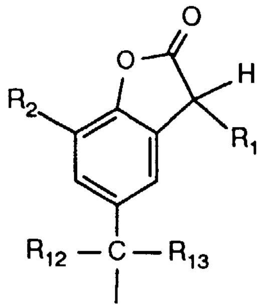

(IIa)

bedeutet,
$R_6$ Wasserstoff oder $C_1$-$C_8$-Alkyl darstellt,
$R_7$ Wasserstoff oder $C_1$-$C_8$-Alkyl bedeutet,
$R_8$ eine direkte Bindung, $C_1$-$C_{18}$-Alkylen, durch Sauerstoff, Schwefel oder $>N-R_6$ unterbrochenes $C_2$-$C_{18}$-Alkylen; $C_2$-$C_{18}$-Alkenylen, $C_2$-$C_{20}$-Alkyliden, $C_7$-$C_{20}$-Phenylalkyliden, $C_5$-$C_8$-Cycloalkylen, $C_7$-$C_8$-Bicycloalkylen, unsubstituiertes oder durch $C_1$-$C_4$-Alkyl substituiertes Phenylen,

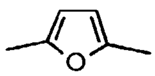 oder 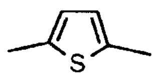

darstellt,
$R_9$ Hydroxy, $[-O^{\ominus}\frac{1}{r}M^{r+}]$, $C_1$-$C_{18}$-Alkoxy oder

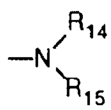

bedeutet,
$R_{10}$ Sauerstoff, -NH- oder

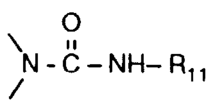

darstellt,
$R_{11}$ $C_1$-$C_{18}$-Alkyl oder Phenyl ist,
$R_{12}$ und $R_{13}$ unabhängig voneinander Wasserstoff, $CF_3$, $C_1$-$C_{12}$-Alkyl oder Phenyl darstellen, oder $R_{12}$ und $R_{13}$ zusammen mit dem C-Atom, an das sie gebunden sind, einen unsubstituierten oder durch 1 bis 3 $C_1$-$C_4$-Alkyl substituierten $C_5$-$C_8$-Cycloalkylidenring bilden;
$R_{14}$ und $R_{15}$ unabhängig voneinander Wasserstoff oder $C_1$-$C_{18}$-Alkyl darstellen,
M ein r-wertiges Metallkation ist,
n 0, 1 oder 2 bedeutet,
p 0, 1 oder 2 darstellt,
q 1, 2, 3, 4, 5 oder 6 bedeutet und
r 1, 2 oder 3 ist.

**18.** Verbindungen gemäss Anspruch 17, worin $R_3$ und $R_5$ Wasserstoff sind.

**19.** Verbindungen gemäss Anspruch 17, worin
$R_1$ Chlor, Brom oder -OR'$_1$ darstellt,
R'$_1$ Wasserstoff, $C_1$-$C_{12}$-Alkanoyl, durch Sauerstoff unterbrochenes $C_3$-$C_{12}$-Alkanoyl; Cyclohexylcarbonyl, Benzoyl, Naphtoyl, $C_1$-$C_{12}$-Alkansulfonyl, durch Fluor substituiertes $C_1$-$C_{12}$-Alkansulfonyl; Phenylsulfonyl oder durch

$C_1$-$C_4$-Alkyl substituiertes Phenylsulfonyl;

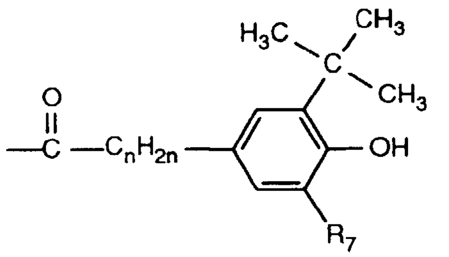 , 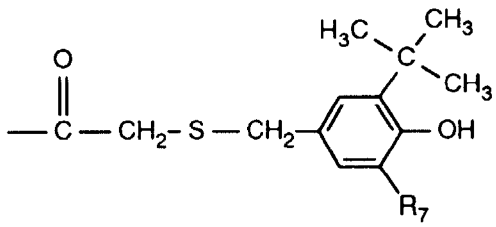 ,

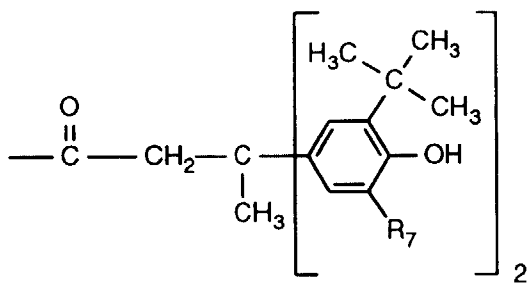 , 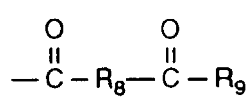 oder 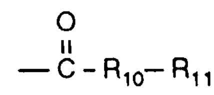

bedeutet,
$R_2$, $R_3$, $R_4$ und $R_5$ unabhängig voneinander Wasserstoff, Chlor, Hydroxy, $C_1$-$C_{18}$-Alkyl, $C_7$-$C_9$-Phenylalkyl, Phenyl, $C_5$-$C_8$-Cycloalkyl, $C_1$-$C_6$-Alkoxy, Cyclohexylcarbonyloxy oder Benzoyloxy darstellen, oder ferner die Reste $R_2$ und $R_3$ oder die Reste $R_3$ und $R_4$ oder die Reste $R_4$ und $R_5$ zusammen mit den Kohlenstoffatomen, an die sie gebunden sind, einen Benzoring bilden; mit der Bedingung, dass mindestens einer der Reste $R_2$, $R_3$, $R_4$ oder $R_5$ von Wasserstoff verschieden ist; wenn $R_1$ Hydroxy, Chlor oder Brom bedeutet, $R_2$ von Wasserstoff verschieden ist; und wenn $R_2$ Methyl oder Hydroxy darstellt, $R_4$ verschieden von Wasserstoff und Hydroxy ist; $R_4$ zusätzlich $-(CH_2)_p$-$COR_9$ darstellt, oder wenn $R_3$ und $R_5$ Wasserstoff sind, $R_4$ zusätzlich einen Rest der Formel IIa

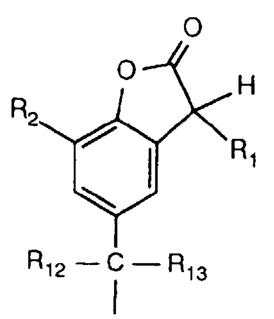 (IIa)

bedeutet,
$R_7$ Wasserstoff oder $C_1$-$C_4$-Alkyl darstellt,
$R_8$ $C_1$-$C_{12}$-Alkylen oder Phenylen bedeutet,
$R_9$ Hydroxy oder $C_1$-$C_8$-Alkoxy darstellt,
$R_{10}$ -NH- ist,
$R_{11}$ $C_1$-$C_8$-Alkyl oder Phenyl bedeutet und
$R_{12}$ und $R_{13}$ Methylgruppen sind oder zusammen mit dem C-Atom, an das sie gebunden sind, einen $C_5$-$C_8$-Cycloalkylidenring bilden.

**20.** Verbindungen gemäss Anspruch 17, worin
$R_1$ Chlor oder -OR'$_1$ darstellt,
R'$_1$ Wasserstoff, $C_1$-$C_8$-Alkanoyl, Benzoyl, Methansulfonyl, Trifluormethansulfonyl; Phenylsulfonyl oder durch $C_1$-

$C_4$-Alkyl substituiertes Phenylsulfonyl;

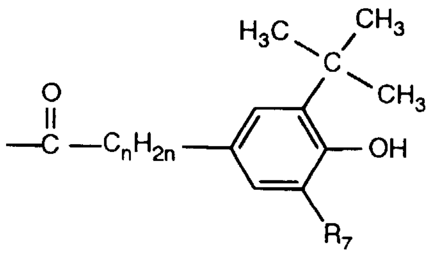

oder

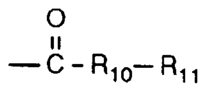

bedeutet,

$R_2$, $R_3$, $R_4$ und $R_5$ unabhängig voneinander Wasserstoff, Chlor, $C_1$-$C_{18}$-Alkyl, $C_7$-$C_9$-Phenylalkyl, Phenyl, Cyclohexyl, $C_1$-$C_6$-Alkoxy darstellen; mit der Bedingung, dass mindestens einer der Reste $R_2$, $R_3$, $R_4$ oder $R_5$ von Wasserstoff verschieden ist; wenn $R_1$ Hydroxy oder Chlor bedeutet, $R_2$ von Wasserstoff verschieden ist; und wenn $R_2$ Methyl darstellt, $R_4$ verschieden von Wasserstoff ist; $R_4$ zusätzlich $-(CH_2)_p-COR_9$ darstellt, oder wenn $R_3$ und $R_5$ Wasserstoff sind, $R_4$ zusätzlich einen Rest der Formel IIa

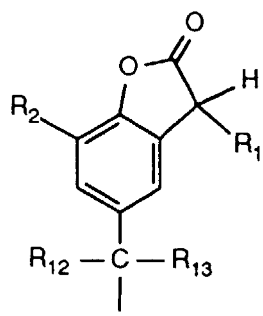

(IIa)

bedeutet,

$R_7$ Wasserstoff oder $C_1$-$C_4$-Alkyl darstellt,

$R_9$ Hydroxy oder $C_1$-$C_8$-Alkoxy bedeutet,

$R_{10}$ -NH- ist,

$R_{11}$ $C_1$-$C_4$-Alkyl oder Phenyl darstellt,

$R_{12}$ und $R_{13}$ zusammen mit dem C-Atom, an das sie gebunden sind, einen Cyclohexylidenring bilden,

n 2 bedeutet, und

p 2 ist.

**21.** Verwendung der Verbindungen der in Anspruch 1 definierten Formel I als Stabilisatoren für organische Materialien gegen oxidativen, thermischen oder lichtinduzierten Abbau.

**22.** Verfahren zum Stabilisieren eines organischen Materials gegen oxidativen, thermischen oder lichtinduzierten Abbau, dadurch gekennzeichnet, dass man diesem mindestens eine Verbindung der in Anspruch 1 definierten Formel I einverleibt oder auf dieses aufbringt.

**23.** Verfahren zur Herstellung von Verbindungen der Formel I, worin die allgemeinen Symbole wie in Anspruch 1 definiert sind, dadurch gekennzeichnet, dass

a) ein Aequivalent Phenol der Formel III

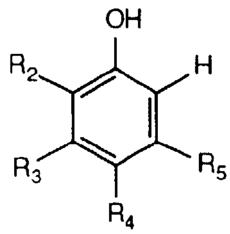

(III)

worin die allgemeinen Symbole wie in Anspruch 1 definiert sind, mit 0,8 bis 2,0 Aequivalent Glyoxylsäure zu einer Verbindung der Formel IV

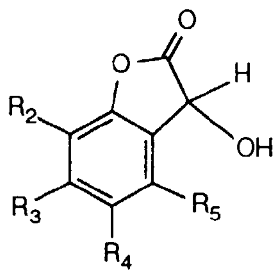

(IV)

worin die allgemeinen Symbole wie in Anspruch 1 definiert sind, umgesetzt wird, und

b) zur Herstellung von Verbindungen der Formel I, worin $R'_1$ nicht Wasserstoff bedeutet, die erhaltene Verbindung der Formel IV mit einer Halogenwasserstoffsäure, einem Halogenid einer Schwefel-Sauerstoffsäure, einem Halogenid der Phosphorsäure, einem Halogenid der phosphorigen Säure, einer Säure der Formel V

$$R'_1\text{-OH} \quad (V)$$

einem Säurehalogenid der Formel VI

$$R'_1\text{-Y} \quad (VI)$$

einem Ester der Formel VII

$$R'_1\text{-O-}R_{16} \quad (VII)$$

einem symmetrischen oder unsymmetrischen Anhydrid der Formel VIII

$$R'_1\text{-O-}R'_1 \quad (VIII)$$

oder einem Isocyanat der Formel IX

$$R_{11}\text{-N=C=O} \quad (IX)$$

worin $R'_1$ und $R_{11}$ in den Verbindungen der Formeln V, VI VII, VIII und IX wie in Anspruch 1 definiert ist, mit der Bedingung, dass $R'_1$ nicht Wasserstoff bedeutet;
$R_{16}$ $C_1$-$C_8$-Alkyl darstellt, und

Y Fluor, Chlor, Brom oder Iod bedeutet, umgesetzt wird.

**24.** Verfahren gemäss Anspruch 23, worin die Glyoxylsäure als 40 bis 60 % wässrige Glyoxylsäure, insbesondere 50 % wässrige Glyoxylsäure, verwendet wird.

## Claims

**1.** A composition comprising

a) an organic material that is subject to oxidative, thermal or light-induced degradation, and
b) at least one compound of formula I

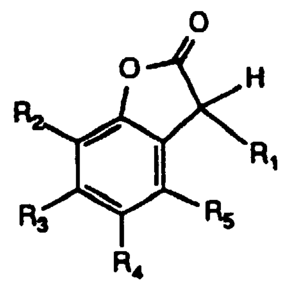 (I)

wherein

$R_1$ is halogen or $-OR'_1$,
$R'_1$ is hydrogen, $C_1$-$C_{25}$alkanoyl, $C_3$-$C_{25}$alkenoyl, $C_3$-$C_{25}$alkanoyl which is interrupted by oxygen, sulfur or $>N-R_6$ ; $C_6$-$C_9$cycloalkylcarbonyl, thenoyl, furoyl, benzoyl or $C_1$-$C_{12}$alkyl-substituted benzoyl; naphthoyl or $C_1$-$C_{12}$alkyl-substituted naphthoyl; $C_1$-$C_{25}$alkanesulfonyl, fluoro-substituted $C_1$-$C_{25}$alkanesulfonyl;phenylsulfonyl or $C_1$-$C_{12}$alkyl-substituted phenylsulfonyl;

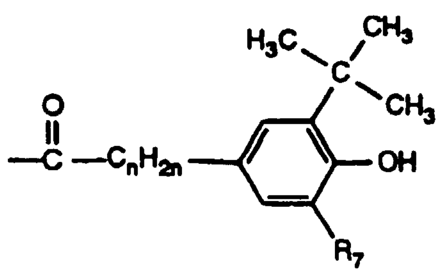 , 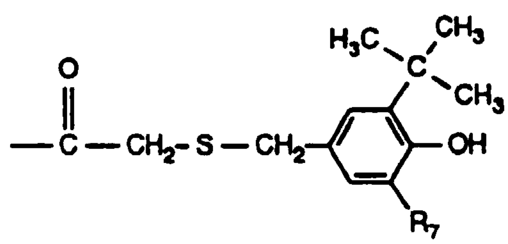 ,

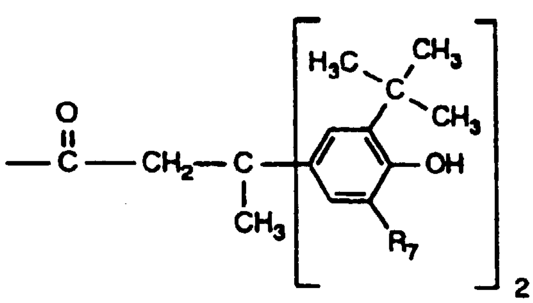 , 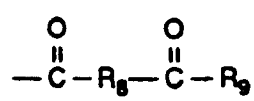

or

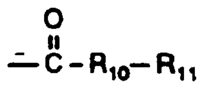

,

$R_2$, $R_3$, $R_4$ and $R_5$ are each independently of one another hydrogen, chloro, hydroxyl, $C_1$-$C_{25}$alkyl, $C_7$-$C_9$phenylalkyl, unsubstituted or $C_1$-$C_4$alkyl-substituted phenyl, unsubstituted or $C_1$-$C_4$alkyl-substituted $C_5$-$C_8$cycloalkyl; $C_1$-$C_{18}$alkoxy, $C_1$-$C_{18}$alkylthio, $C_1$-$C_4$alkylamino, di ($C_1$-$C_4$alkyl)amino, $C_1$-$C_{25}$alkanoyloxy, $C_1$-$C_{25}$alkanoylamino, $C_3$-$C_{25}$alkenoyloxy, $C_3$-$C_{25}$alkanoyloxy which is interrupted by oxygen, sulfur or $>N-R_6$ ; $C_6$-$C_9$cycloalkylcarbonyloxy, benzoyloxy or $C_1$-$C_{12}$alkyl-substituted benzoyloxy; or each pair of radicals $R_2$ and $R_3$ or $R_3$ and $R_4$ or $R_4$ and $R_5$, together with the carbon atoms to which they are attached, forms a benzene ring; $R_4$ is additionally $-(CH_2)_p-COR_9$ or $-(CH_2)_qOH$, or, if $R_3$ and $R_5$ are hydrogen, $R_4$ is additionally a radical of formula II

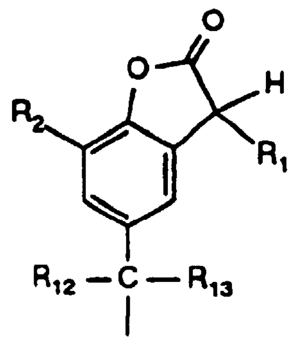

(II),

$R_6$ is hydrogen or $C_1$-$C_8$alkyl,

$R_7$ is hydrogen or $C_1$-$C_8$alkyl,

$R_8$ is a direct bond, $C_1$-$C_{18}$alkylene, $C_2$-$C_{18}$alkylene which is interrupted by oxygen, sulfur or $>N-R_6$ ; $C_2$-$C_{18}$alkenylene, $C_2$-$C_{20}$alkylidene, $C_7$-$C_{20}$phenylalkylidene, $C_5$-$C_8$cycloalkylene, $C_7$-$C_8$bicycloalkylene, unsubstituted or $C_1$-$C_4$alkyl-substituted phenylene,

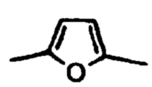

or

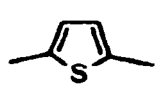

,

$R_9$ is hydroxyl, $[-O^{\ominus} \frac{1}{r} M^{r+}]$, $C_1$-$C_{18}$alkoxy or

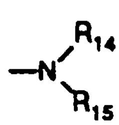

,

$R_{10}$ is oxygen, -NH- or

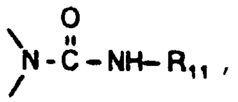

$R_{11}$ is $C_1$-$C_{18}$alkyl or phenyl,

$R_{12}$ and $R_{13}$ are each independently of the other hydrogen, $CF_3$, $C_1$-$C_{12}$alkyl or phenyl, or $R_{12}$ and $R_{13}$, together with the carbon atom to which they are attached, form a $C_5$-$C_8$cycloalkylidene ring which is unsubstituted or substituted by 1 to 3 $C_1$-$C_4$alkyl groups;

$R_{14}$ and $R_{15}$ are each independently of the other hydrogen or $C_1$-$C_{18}$alkyl,

M is a metal cation of valency r,

n is 0, 1 or 2,

p is 0, 1 or 2,

q is 1, 2, 3, 4, 5, or 6, and

r is 1, 2 or 3.

**2.** A composition according to claim 1, wherein

$R'_1$ is hydrogen, $C_1$-$C_{18}$alkanoyl, $C_3$-$C_{18}$alkenoyl, $C_3$-$C_{18}$alkanoyl which is interrupted by oxygen, sulfur or $>N-R_6$ ; $C_6$-$C_8$cycloalkylcarbonyl, thenoyl, furoyl, benzoyl or $C_1$-$C_8$alkylsubstituted benzoyl; naphthoyl or $C_1$-$C_8$alkyl-substituted naphthoyl; $C_1$-$C_{18}$alkanesulfonyl, fluoro-substituted $C_1$-$C_{18}$alkanesulfonyl; phenylsulfonyl or $C_1$-$C_8$alkyl-substituted phenylsulfonyl;

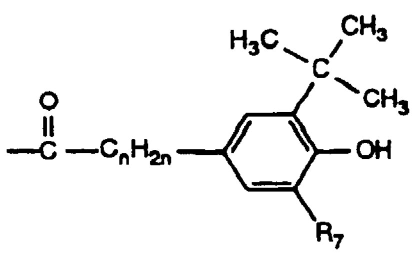 , 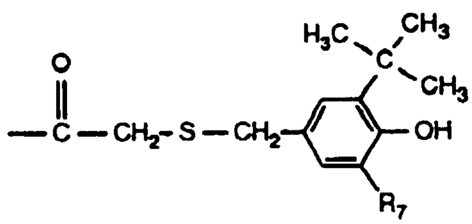 ,

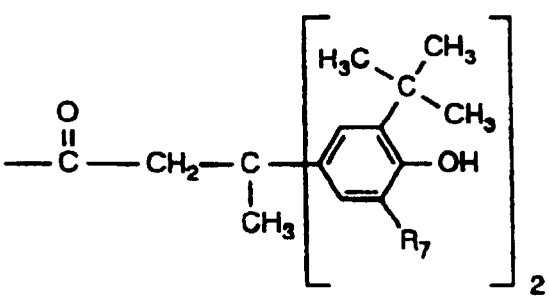 , 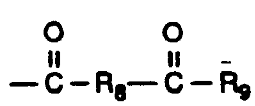 or 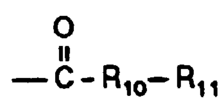

$R_2$, $R_3$, $R_4$ and $R_5$ are each independently of one another hydrogen, chloro, hydroxyl, $C_1$-$C_{25}$alkyl, $C_7$-$C_9$phenylalkyl, unsubstituted or $C_1$-$C_4$alkyl-substituted phenyl, unsubstituted or $C_1$-$C_4$alkyl-substituted $C_5$-$C_8$cycloalkyl; $C_1$-$C_{12}$alkoxy, $C_1$-$C_{12}$alkylthio, $C_1$-$C_4$alkylamino, di($C_1$-$C_4$alkyl)amino, $C_1$-$C_{18}$alkanoyloxy, $C_1$-$C_{18}$alkanoylamino, $C_3$-$C_{18}$alkenoyloxy, $C_3$-$C_{18}$alkanoyloxy which is interrupted by oxygen, sulfur or $>N-R_6$ ; $C_6$-$C_8$cycloalkylcarbonyloxy, benzoyloxy or $C_1$-$C_8$alkyl-substituted benzoyloxy, or each pair of radicals $R_2$ and $R_3$ or $R_3$ and $R_4$ or $R_4$ and $R_5$, together with the carbon atoms to which they are attached, forms a benzene ring; $R_4$ is additionally $-(CH_2)_p-COR_9$ or $-(CH_2)_qOH$, or, if $R_3$ and $R_5$ are hydrogen, $R_4$ is additionally a radical of formula II

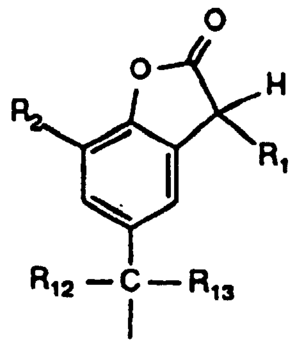

(II)

$R_6$ is hydrogen or $C_1$-$C_6$alkyl,
$R_7$ is hydrogen or $C_1$-$C_6$alkyl,
$R_8$ is a direct bond, $C_1$-$C_{12}$alkylene, $C_2$-$C_{12}$alkylene which is interrupted by oxygen, sulfur or $>N-R_6$ ; $C_2$-$C_{12}$alkenylene, $C_2$-$C_{12}$alkylidene, $C_7$-$C_{12}$phenylalkylidene, $C_5$-$C_8$cycloalkylene, $C_7$-$C_8$bicycloalkylene or phenylene,
$R_9$ is hydroxyl, $C_1$-$C_{12}$alkoxy or

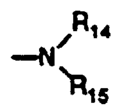
,

$R_{10}$ is oxygen or -NH-,
$R_{11}$ is $C_1$-$C_{12}$alkyl or phenyl,
$R_{12}$ and $R_{13}$ are methyl groups or, together with the carbon atom to which they are attached, form a $C_5$-$C_8$cycloalkylidene ring which is unsubsituted or substituted by 1 to 3 $C_1$-$C_4$alkyl groups;
$R_{14}$ and $R_{15}$ are each independently of the other hydrogen or $C_1$-$C_8$alkyl, and
q is 2, 3, 4, 5 or 6.

**3.** A composition according to claim 1, wherein at least two of the radicals $R_2$, $R_3$, $R_4$ and $R_5$ are hydrogen.

**4.** A composition according to claim 1, wherein $R_3$ and $R_5$ are hydrogen.

**5.** A composition according to claim 1, wherein

$R_1$ is chloro, bromo or -OR'$_1$,
R'$_1$ is hydrogen, $C_1$-$C_{12}$alkanoyl, $C_3$-$C_{12}$alkanoyl which is interrupted by oxygen; cyclohexylcarbonyl, benzoyl, naphthoyl, $C_1$-$C_{12}$alkanesulfonyl, fluoro-substituted $C_1$-$C_{12}$alkanesulfonyl; phenylsulfonyl or $C_1$-$C_4$alkyl-substituted phenylsulfonyl;

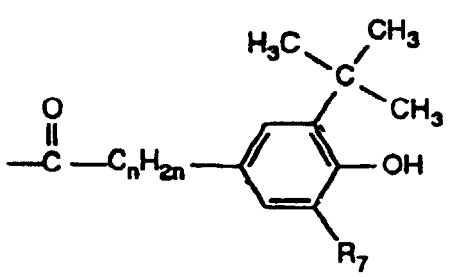
,
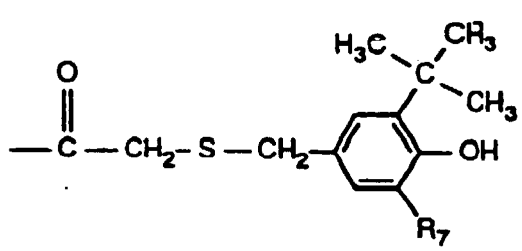
,

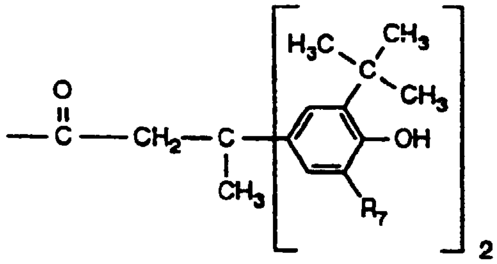, 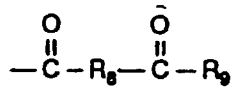 or 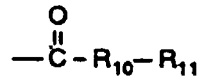

$R_7$ is hydrogen or $C_1$-$C_6$alkyl,
$R_8$ is a direct bond, $C_1$-$C_{12}$alkylene, $C_2$-$C_{12}$alkylene which is interrupted by oxygen, sulfur or $>N—R_6$ ; $C_2$-$C_{12}$alkenylene, $C_2$-$C_{12}$alkylidene, $C_7$-$C_{12}$phenylalkylidene, $C_5$-$C_8$cycloalkylene, $C_7$-$C_8$bicycloalkylene or phenylene,
$R_9$ is hydroxyl, $C_1$-$C_{12}$alkoxy or

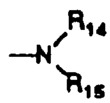,

$R_{10}$ is oxygen or -NH-,
$R_{11}$ is $C_1$-$C_{12}$alkyl or phenyl, and
$R_{14}$ and $R_{15}$ are each independently of the other hydrogen or $C_1$-$C_8$alkyl.

**6.** A composition according to claim 1, wherein

$R'_1$ is hydrogen, $C_1$-$C_8$alkanoyl, benzoyl, methanesulfonyl, p-methylphenylsulfonyl,

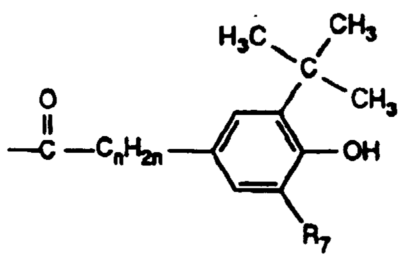

or

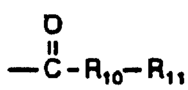,

$R_7$ is hydrogen or $C_1$-$C_4$alkyl,
$R_{10}$ is -NH-,
$R_{11}$ is $C_1$-$C_8$alkyl or phenyl, and
n is 2.

**7.** A composition according to claim 1, wherein

$R_2$, $R_3$, $R_4$ and $R_5$ are each independently of one another hydrogen, chloro, hydroxyl, $C_1$-$C_{18}$alkyl, $C_7$-$C_9$phenylalkyl, phenyl, $C_5$-$C_8$cycloalkyl, $C_1$-$C_6$alkoxy, cyclohexylcarbonyloxy or benzoyloxy, or each pair of radicals $R_2$ and $R_3$ or $R_3$ and $R_4$ or $R_4$ and $R_5$, together with the carbon atoms to which they are attached, forms a benzene ring; $R_4$ is additionally $-(CH_2)_p$-$COR_9$, or, if $R_3$ and $R_5$ are hydrogen, $R_4$ is additionally a radical of formula II,
$R_7$ is hydrogen or $C_1$-$C_4$alkyl,
$R_8$ is $C_1$-$C_{12}$alkylene or phenylene,
$R_9$ is hydroxy or $C_1$-$C_8$alkoxy,
$R_{10}$ is -NH-,
$R_{11}$ is $C_1$-$C_8$alkyl or phenyl, and
$R_{12}$ and $R_{13}$ are methyl groups or, together with the carbon atom to which they are attached, form a $C_5$-$C_8$-cycloalkylidene ring.

**8.** A composition according to claim 1, wherein

$R_1$ is chloro or $-OR'_1$,
$R'_1$ is hydrogen, $C_1$-$C_8$alkanoyl, benzoyl, methanesulfonyl, trifluoromethanesulfonyl, phenylsulfonyl or $C_1$-$C_4$alky-substituted phenylsulfonyl;

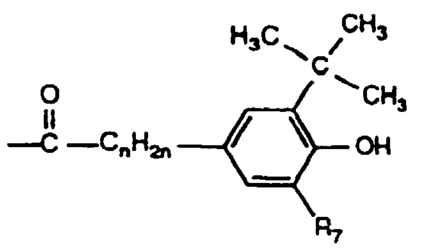 or 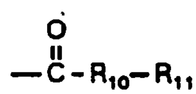 ,

$R_2$, $R_3$, $R_4$ and $R_5$ are each independently of one another hydrogen, chloro, $C_1$-$C_{18}$alkyl, $C_7$-$C_9$phenylalkyl, phenyl, cyclohexyl, $C_1$-$C_6$alkoxy, $R_4$ is additionally $-(CH_2)_p$-$COR_9$, or, if $R_3$ and $R_5$ are hydrogen, $R_4$ is additionally a radical of formula II

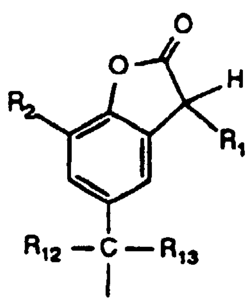 (II) ,

$R_7$ is hydrogen or $C_1$-$C_4$alkyl,
$R_9$ is hydroxyl or $C_1$-$C_8$alkoxy,
$R_{10}$ is -NH-,
$R_{11}$ is $C_1$-$C_4$alkyl or phenyl,
$R_{12}$ and $R_{13}$, together with the carbon atom to which they are attached, form a cyclohexylidene ring,
n is 2, and
p is 2.

**9.** A composition according to claim 1, wherein

$R_1$ is chloro or $-OR'_1$,

$R'_1$ is hydrogen, acetyl,

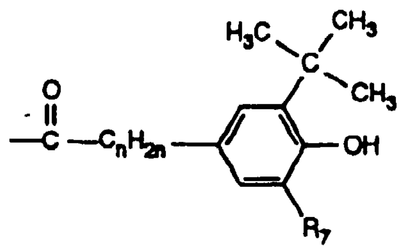

or

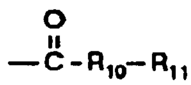 ,

$R_2$ is $C_1$-$C_{16}$alkyl, $C_7$-$C_9$phenylalkyl or cyclohexyl,
$R_3$ is hydrogen,
$R_4$ is $C_1$-$C_4$alkyl, $C_7$-$C_9$phenylalkyl, cyclohexyl, $-CH_2CH_2COOH$ or a radical of formula II

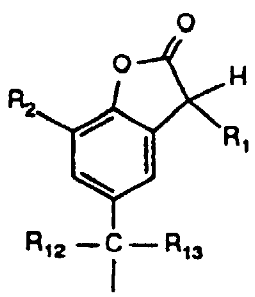 (II) ,

$R_5$ is hydrogen,
$R_7$ is hydrogen or $C_1$-$C_4$alkyl,
$R_{10}$ is -NH-,
$R_{11}$ is $C_1$-$C_4$alkyl,
$R_{12}$ and $R_{13}$, together with the carbon atom to which they are attached, form a cyclohexylidene ring, and n is 2.

**10.** A composition according to claim 1, comprising further additives in addition to components (a) and (b).

**11.** A composition according to claim 10, comprising as further additives phenolic antioxidants, light stabilisers and/or processing stabilisers.

**12.** A composition according to claim 10, comprising as further additive at least one compound of the organic phosphite or phosphonite type.

**13.** A composition according to claim 1, comprising as component (a) natural, semi-synthetic or synthetic polymers.

**14.** A composition according to claim 1, comprising as component (a) a polyolefin.

**15.** A composition according to claim 1, comprising as component (a) polyethylene or polypropylene.

**16.** A composition according to claim 1, which contains from 0.0005 to 5% of component (b), based on the weight of component (a).

**17.** A compound of formula Ia

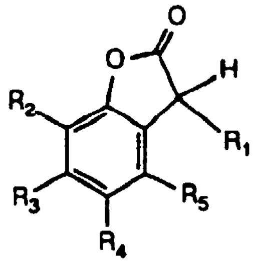

(Ia)

wherein

$R_1$ is halogen or $-OR'_1$,

$R'_1$ is hydrogen, $C_1$-$C_{25}$alkanoyl, $C_3$-$C_{25}$alkenoyl, $C_3$-$C_{25}$alkanoyl which is interrupted by oxygen, sulfur or $>N-R_6$ ; $C_6$-$C_9$cycloalkylcarbonyl, thenoyl, furoyl, benzoyl or $C_1$-$C_{12}$alkyl-substituted benzoyl; naphthoyl or $C_1$-$C_{12}$alkyl-substituted naphthoyl; $C_1$-$C_{25}$alkanesulfonyl, fluoro-substituted $C_1$-$C_{25}$alkanesulfonyl; phenylsulfonyl or $C_1$-$C_{12}$alkyl-substituted phenylsulfonyl;

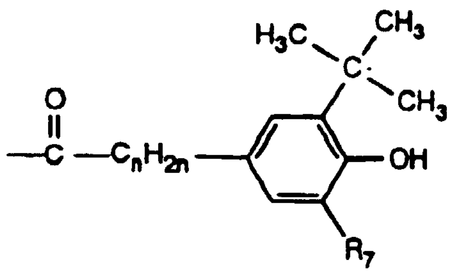
,
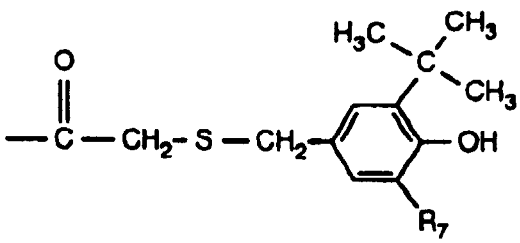
,

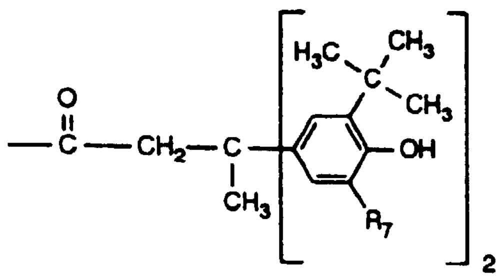
,
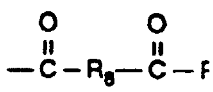
or
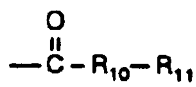
,

$R_2$, $R_3$, $R_4$ and $R_5$ are each independently of one another hydrogen, chloro, hydroxyl, $C_1$-$C_{25}$alkyl, $C_7$-$C_9$phenylalkyl, unsubstituted or $C_1$-$C_4$alkyl-substituted phenyl, unsubstituted or $C_1$-$C_4$alkyl-substituted $C_5$-$C_8$-cycloalkyl; $C_1$-$C_{18}$alkoxy, $C_1$-$C_{18}$alkylthio, $C_1$-$C_4$alkylamino, di($C_1$-$C_4$alkyl)amino, $C_1$-$C_{25}$alkanoyloxy, $C_1$-$C_{25}$alkanoylamino, $C_3$-$C_{25}$alkenoyloxy, $C_3$-$C_{25}$alkanoyloxy which is interrupted by oxygen, sulfur or $>N-R_6$ ; $C_6$-$C_9$cycloalkylcarbonyloxy, benzoyloxy or $C_1$-$C_{12}$alkyl-substituted benzoyloxy; or each pair of radicals $R_2$ and $R_3$ or $R_3$ and $R_4$ or $R_4$ and $R_5$, together with the carbon atoms to which they are attached, forms a benzene ring; with the proviso that at least one of the radicals $R_2$, $R_3$, $R_4$ or $R_5$ is not hydrogen; if $R_1$ is halogen or hydroxyl, $R_2$ is not hydrogen; and, if $R_2$ is methyl or methylamine, $R_4$ is not hydrogen and hydroxyl; $R_4$ is additionally $-(CH_2)_p-COR_9$ or $-(CH_2)_qOH$, or, if $R_3$ and $R_5$ are hydrogen, $R_4$ is additionally a radical of formula IIa

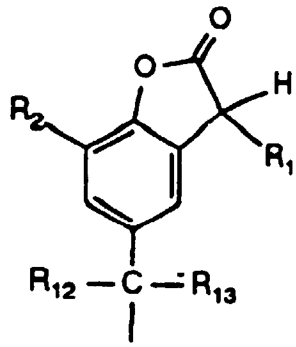

(IIa) ,

$R_6$ is hydrogen or $C_1$-$C_8$alkyl,

$R_7$ is hydrogen or $C_1$-$C_8$alkyl,

$R_8$ is a direct bond, $C_1$-$C_{18}$alkylene, $C_2$-$C_{18}$alkylene which is interrupted by oxygen, sulfur or $>N-R_6$ ; $C_2$-$C_{18}$alkenylene, $C_2$-$C_{20}$alkylidene, $C_7$-$C_{20}$phenylalkylidene, $C_5$-$C_8$cycloalkylene, $C_7$-$C_8$bicycloalkylene, unsubstituted or $C_1$-$C_4$alkyl-substituted phenylene,

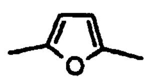 or 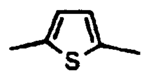 ,

$R_9$ is hydroxy, $[-O^{\ominus} \frac{1}{r} M^{r+}]$, $C_1$-$C_{18}$alkoxy or

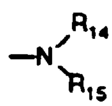 ,

$R_{10}$ is oxygen, -NH- or

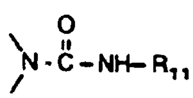 ,

$R_{11}$ is $C_1$-$C_{18}$alkyl or phenyl,

$R_{12}$ and $R_{13}$ are each independently of the other hydrogen, $CF_3$, $C_1$-$C_{12}$alkyl or phenyl, or $R_{12}$ and $R_{13}$, together with the carbon atom to which they are attached, form a $C_5$-$C_8$cycloalkylidene ring which is unsubstituted or substituted by 1 to 3 $C_1$-$C_4$alkyl groups,

$R_{14}$ and $R_{15}$ are each independently of the other hydrogen or $C_1$-$C_{18}$alkyl,

M is a metal cation of valency r,

n is 0, 1 or 2,

p is 0, 1 or 2,

q is 1, 2, 3, 4, 5 or 6, and

r is 1, 2 or 3.

**18.** A compound according to claim 17, wherein $R_3$ and $R_5$ are hydrogen.

**19.** A compound according to claim 17, wherein

$R_1$ is chloro, bromo or $-OR'_1$,

$R'_1$ is hydrogen, $C_1$-$C_{12}$alkanoyl, $C_3$-$C_{12}$alkanoyl which is interrupted by oxygen; cyclohexylcarbonyl, benzoyl, naphthoyl, $C_1$-$C_{12}$alkanesulfonyl, fluoro-substituted $C_1$-$C_{12}$alkanesulfonyl; phenylsulfonyl or $C_1$-$C_4$alkyl-sub-

stituted phenylsulfonyl;

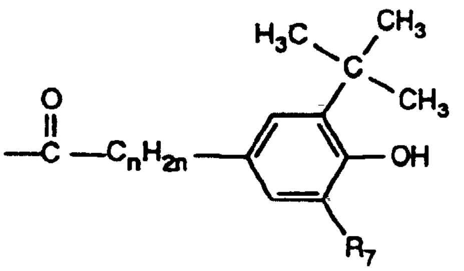 , 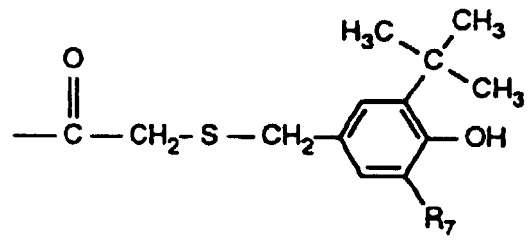 ,

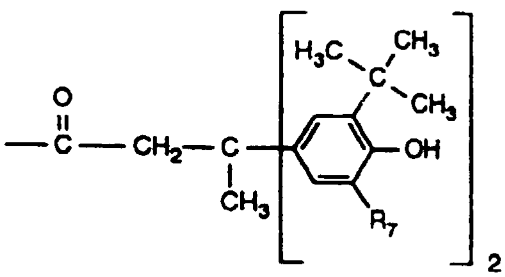 , 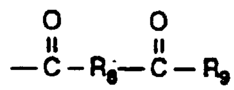 or 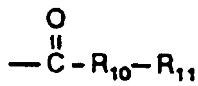 ,

$R_2$, $R_3$, $R_4$ and $R_5$ are each independently of one another hydrogen, chloro, hydroxyl, $C_1$-$C_{18}$alkyl, $C_7$-$C_9$phenylalkyl, phenyl, $C_5$-$C_8$cycloalkyl, $C_1$-$C_6$alkoxy, cyclohexylcarbonyloxy or benzoyloxy, or each pair of radicals $R_2$ and $R_3$ or $R_3$ and $R_4$ or $R_4$ and $R_5$, together with the carbon atoms to which they are attached, forms a benzene ring; with the proviso that at least one of the radicals $R_2$, $R_3$, $R_4$ or $R_5$ is not hydrogen; if $R_1$ is hydroxyl, chloro or bromo, $R_2$ is not hydrogen; and, if $R_2$ is methyl or hydroxyl, $R_4$ is not hydrogen and hydroxyl; $R_4$ is additionally $-(CH_2)_p-COR_9$, or, if $R_3$ and $R_5$ are hydrogen, $R_4$ is additionally a radical of formula IIa

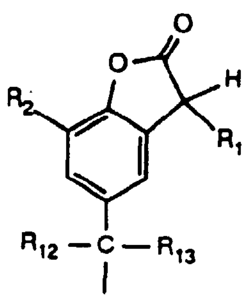 **(IIa)** ,

$R_7$ is hydrogen or $C_1$-$C_4$alkyl,
$R_8$ is $C_1$-$C_{12}$alkylene or phenylene,
$R_9$ is hydroxyl or $C_1$-$C_8$alkoxy,
$R_{10}$ is -NH-,
$R_{11}$ is $C_1$-$C_8$alkyl or phenyl, and
$R_{12}$ and $R_{13}$ are methyl groups or, together with the carbon atom to which they are attached, form a $C_5$-$C_8$-cycloalkylidene ring.

**20.** A compound according to claim 17, wherein

$R_1$ is chloro or $-OR'_1$,
$R'_1$ is hydrogen, $C_1$-$C_8$alkanoyl, benzoyl, methanesulfonyl, trifluoromethanesulfonyl; phenylsulfonyl or $C_1$-$C_4$alkyl-substituted phenylsulfonyl;

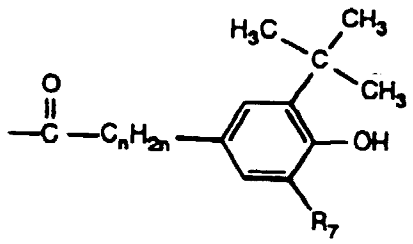 or 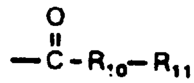 ,

$R_2$, $R_3$, $R_4$ and $R_5$ are each independently of one another hydrogen, chloro, $C_1$-$C_{18}$alkyl, $C_7$-$C_9$phenylalkyl, phenyl, cyclohexyl, $C_1$-$C_6$alkoxy, with the proviso that at least one of the radicals $R_2$, $R_3$, $R_4$ or $R_5$ is not hydrogen; if $R_1$ is hydroxyl or chloro, $R_2$ is not hydrogen; and, if $R_2$ is methyl, $R_4$ is not hydrogen; $R_4$ is additionally $-(CH_2)_p-COR_9$, or, if $R_3$ and $R_5$ are hydrogen, $R_4$ is additionally a radical of formula IIa

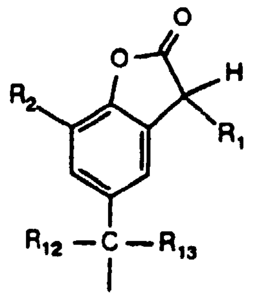 (IIa) ,

$R_7$ is hydrogen or $C_1$-$C_4$alkyl,
$R_9$ is hydroxyl or $C_1$-$C_8$alkoxy,
$R_{10}$ is -NH-,
$R_{11}$ is $C_1$-$C_4$alkyl or phenyl,
$R_{12}$ and $R_{13}$, together with the carbon atom to which they are attached, form a cyclohexylidene ring,
n is 2, and
p is 2.

**21.** Use of a compound of formula I as defined in claim 1 for stabilising organic materials against oxidative, thermal or light-induced degradation.

**22.** A process for stabilising an organic material against oxidative, thermal or light-induced degradation, which comprises incorporating therein or applying thereto at least one compound of formula I as defined in claim 1.

**23.** A process for the preparation of a compound of formula I in which the general symbols are as defined in claim 1, which comprises reacting

a) one equivalent of a phenol of formula III

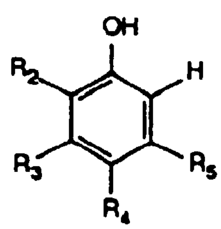 (III)

wherein the general symbols are as defined in claim 1 with 0.8 to 2.0 equivalents of glyoxylic acid to give a compound of formula IV

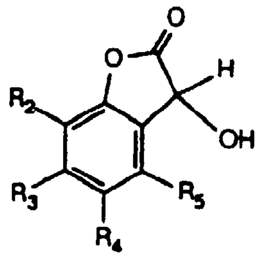 (IV)

wherein the general symbols are as defined in claim 1, and

b) to prepare a compound of formula I wherein $R'_1$ is not hydrogen, reacting the resultant compound of formula IV with a hydrohalic acid, a halide of an oxysulfuric acid, a halide of phosphoric acid, a halide of phosphorous acid, an acid of formula V

$$R'_1\text{-OH} \qquad (V),$$

an acid halide of formula VI

$$R'_1\text{-Y} \qquad (VI),$$

an ester of formula VII

$$R'_1\text{-O-}R_{16} \qquad (VII),$$

a symmetrical or unsymmetrical anhydride of formula VIII

$$R'_1\text{-O-}R'_1 \qquad (VIII),$$

or an isocyanate of formula IX

$$R_{11}\text{-N=C=O} \qquad (IX)$$

wherein $R'_1$ and $R_{11}$ in the compounds of formulae V, VI, VII, VIII and IX are as defined in claim 1, with the proviso that $R'_1$ is not hydrogen;

$R_{16}$ is $C_1$-$C_8$alkyl, and

Y is fluoro, chloro, bromo or iodo.

**24.** A process according to claim 23, wherein the glyoxylic acid is used as 40 to 60%, preferably as 50%, aqueous glyoxylic acid.

**Revendications**

**1.** Composition contenant

a) une matière organique soumise à la dégradation oxydative, thermique ou induite par la lumière,
b) au moins un composé de formule I

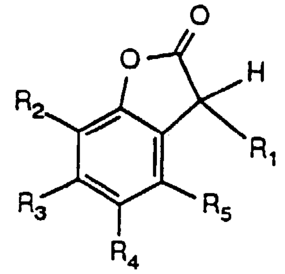

(I)

dans laquelle

| | |
|---|---|
| $R_1$ | représente un atome d'halogène ou $-OR'_1$, |
| $R'_1$ | représente un atome d'hydrogène, des groupes alcanoyle en $C_1$-$C_{25}$, alcénoyle en $C_3$-$C_{25}$, alcanoyle en $C_3$-$C_{25}$ interrompu par des atomes d'oxygène, de soufre ou $>N—R_6$ ; cyclo(alkyl en $C_6$-$C_9$)carbonyle, thénoyle, furoyle, benzoyle ou benzoyle substitué un groupe par alkyle en $C_1$-$C_{12}$ ; naphtoyle ou naphtoyle substitué par alkyle en $C_1$-$C_{12}$ ; (alkane en $C_1$-$C_{25}$)sulfonyle, (alcane en $C_1$-$C_{25}$)sulfonyle substitué par du fluor ; phénylsulfonyle ou phénylsulfonyle substitué par alkyle en $C_1$-$C_{12}$ ; |

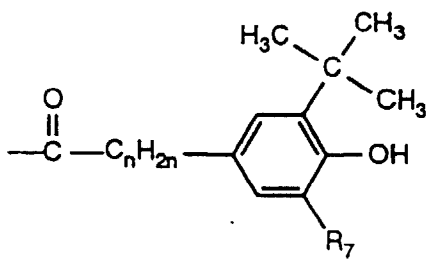

,

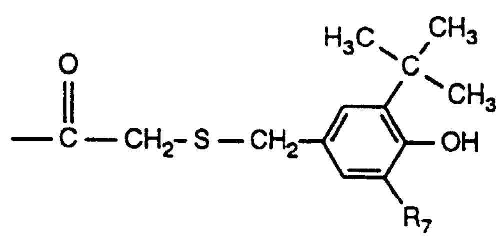

,

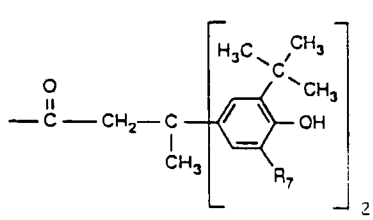

,

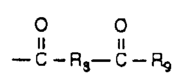

ou

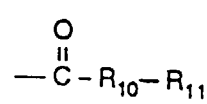

| | |
|---|---|
| $R_2$, $R_3$, $R_4$ et $R_5$, | représentent indépendamment les uns des autres, un atome d'hydrogène, un atome de chlore, des groupes hydroxy, alkyle en $C_1$-$C_{25}$, phénylalkyle en $C_7$-$C_9$, phényle non substitué ou substitué par alkyle en $C_1$-$C_4$, cycloalkyle en $C_5$-$C_8$ non substitué ou substitué par alkyle en $C_1$-$C_4$ ; alkoxy en $C_1$-$C_{18}$, (alkyl en $C_1$-$C_{18}$)thio, (alkyl en $C_1$-$C_4$) amino, di(alkyl en $C_1$-$C_4$)amino, alcanoyloxy en $C_1$-$C_{25}$, (alcanoyl en $C_1$-$C_{25}$)amino, alcénoyloxy en $C_3$-$C_{25}$, alcanoyloxy en $C_3$-$C_{25}$ interrompu par des atomes d'oxygène, de soufre ou $>N—R_6$ ; cyclo(alkyl en $C_6$-$C_9$)-carbonyloxy, benzoyloxy ou benzoyloxy substitué par alkyle en $C_1$-$C_{12}$, ou de plus les restes $R_2$ et $R_3$ ou les restes $R_3$ et $R_4$ ou les restes $R_4$ et $R_5$ forment, conjointement avec les atomes de carbone auxquels ils sont liés, un cycle benzo, $R_4$ représente de plus $-(CH_2)_p-COR_9$ ou $-(CH_2)_qOH$, ou lorsque $R_3$ et $R_5$ représentent un atome d'hydrogène, $R_4$ représente en plus un reste de formule II |

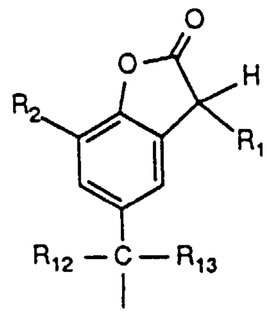

(II)

$R_6$ représente un atome d'hydrogène ou un groupe alkyle en $C_1$-$C_8$,

$R_7$ représente un atome d'hydrogène ou un groupe alkyle en $C_1$-$C_8$,

$R_8$ représente une liaison directe, des groupes alkylène en $C_1$-$C_{18}$, alkylène en $C_2$-$C_{18}$ interrompu par des atomes d'oxygène, de soufre ou $>N-R_6$ ; alcénylène en $C_2$-$C_{18}$, alkylidène en $C_2$-$C_{20}$, phénylalkylidène en $C_7$-$C_{20}$, cycloalkylène en $C_5$-$C_8$, bicycloalkylène en $C_7$-$C_8$, phénylène non substitué ou substitué par alkyle en $C_1$-$C_4$,

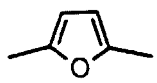 ou 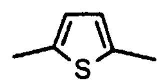

$R_9$ représente des groupes hydroxy, $[-O^{\ominus}\frac{1}{r}M^{r+}]$, alkoxy en $C_1$-$C_{18}$ ou

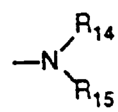

$R_{10}$ représente un atome d'oxygène, -NH- ou

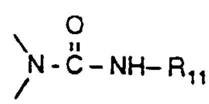

$R_{11}$ représente des groupes alkyle en $C_1$-$C_{18}$ ou phényle,

$R_{12}$ et $R_{13}$ représentent, indépendamment l'un de l'autre, un atome d'hydrogène, des groupes $CF_3$, alkyle en $C_1$-$C_{12}$ ou phényle, ou $R_{12}$ et $R_{13}$, forment, conjointement avec l'atome de carbone auquel ils sont liés, un cycle cycloalkylidène en $C_5$-$C_8$ non substitué ou substitué par 1 à 3 groupes alkyle en $C_1$-$C_4$ ;

$R_{14}$ et $R_{15}$, représentent, indépendamment l'un de l'autre, un atome d'hydrogène ou un groupe alkyle en $C_1$-$C_{18}$,

M représente un cation métallique à fonctionnalité r,

n vaut 0, 1 ou 2,

p vaut 0, 1 ou 2,

q vaut 1, 2, 3, 4, 5 ou, et

r vaut 1, 2 ou 3.

**2.** Composition selon la revendication 1, dans laquelle

$R'_1$ représente un atome d'hydrogène, des groupes alcanoyle en $C_1$-$C_{18}$, alcénoyle en $C_3$-$C_{18}$, alcanoyle en $C_3$-$C_{18}$ interrompu par des atomes d'oxygène, de soufre ou $>N-R_6$ ; (cycloalkyl

en $C_6$-$C_8$)carbonyle, thénoyle, furoyle, benzoyle ou benzoyle substitué par alkyle en $C_1$-$C_8$ ; naphtoyle ou naphtoyle substitué par alkyle en $C_1$-$C_8$ ; (alcane en $C_1$-$C_{18}$)sulfonyle, (alcane en $C_1$-$C_{18}$)sulfonyle substitué par du fluor ; phénylsufonyle ou phénylsulfonyle substitué par alkyle en $C_1$-$C_8$ ;

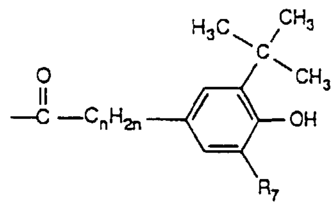
,
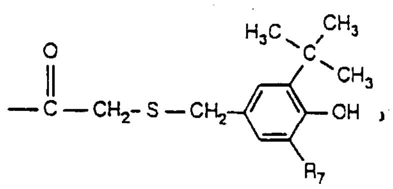
,

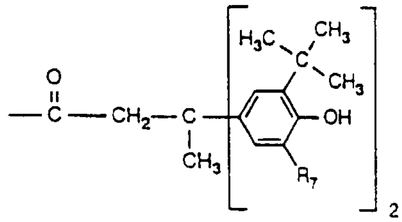
,
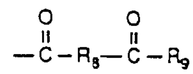
ou
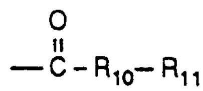

R₂, R₃, R₄ et R₅ représentent, indépendamment les uns des autres, un atome d'hydrogène, un atome de chlore, des groupes hydroxy, alkyle en $C_1$-$C_{25}$, phénylalkyle en $C_7$-$C_9$, phényle non substitué ou substitué par alkyle en $C_1$-$C_4$, cycloalkyle en $C_5$-$C_8$ non substitué ou substitué par alkyle en $C_1$-$C_4$ ; alkoxy en $C_1$-$C_{12}$, (alkyl en $C_1$-$C_{12}$)thio, (alkyl en $C_1$-$C_4$)amino, di(alkyl en $C_1$-$C_4$)-amino, alcanoyloxy en $C_1$-$C_{18}$, (alcanoyl en $C_1$-$C_{18}$)amino, alcénoyloxy en $C_3$-$C_{18}$, alcanoyloxy en $C_3$-$C_{18}$ interrompu par des atomes d'oxygène, de soufre ou $>N—R_6$ ; cyclo(alkyl en $C_6$-$C_8$)-carbonyloxy, benzoyloxy ou benzoyloxy substitué par alkyle $C_1$-$C_8$, ou de plus les restes $R_2$ et $R_3$ ou les restes $R_3$ et $R_4$ ou les restes $R_4$ et $R_5$ forment, conjointement avec les atomes de carbone auxquels ils sont liés, un cycle benzo, $R_4$ en plus représente $-(CH_2)_p-COR_9$ ou $-(CH_2)_qOH$, ou lorsque $R_3$ et $R_5$ représentent un atome d'hydrogène, $R_4$ représente de plus un reste de formule II

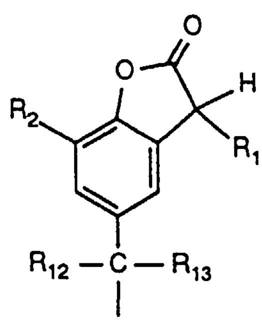
(II)

$R_6$ représente un atome d'hydrogène ou un groupe alkyle en $C_1$-$C_6$,

$R_7$ représente un atome d'hydrogène ou un groupe alkyle en $C_1$-$C_6$,

$R_8$ représente une liaison directe, des groupes alkylène en $C_1$-$C_{12}$, alkylène en $C_2$-$C_{12}$ interrompu par des atomes d'oxygène, de soufre ou $>N—R_6$ ; alcénylène en $C_2$-$C_{12}$, alkylidène en $C_2$-$C_{12}$, phénylalkylidène en $C_7$-$C_{12}$, cycloalkylène en $C_5$-$C_8$, bicycloalkylène en $C_7$-$C_8$ ou phénylène,

$R_9$ représente des groupes hydroxy, alkoxy en $C_1$-$C_{12}$ ou

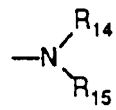

| | |
|---|---|
| $R_{10}$ | représente un atome d'oxygène ou -NH-, |
| $R_{11}$ | représente des groupes alkyle en $C_1$-$C_{12}$ ou phényle, |
| $R_{12}$ et $R_{13}$ | reprèsentent des groupes méthyle ou forment, conjointement avec l'atome de carbone auquel ils sont liés, un cycle cycloalkylidène en $C_5$-$C_8$ non substitué ou substitué par 1 à 3 groupes alkyle en $C_1$-$C_4$ ; |
| $R_{14}$ et $R_{15}$, | représentent indépendamment l'un de l'autre, un atome d'hydrogène ou un groupe alkyle en $C_1$-$C_8$, et |
| q | vaut 2, 3, 4, 5 ou 6. |

**3.** Composition selon la revendication 1, dans laquelle au moins deux des restes $R_2$, $R_3$, $R_4$ et $R_5$ représentent un atome d'hydrogène.

**4.** Composition selon la revendication 1, dans laquelle $R_3$ et $R_5$ représentent un atome d'hydrogène.

**5.** Composition selon la revendication 1, dans laquelle

$R_1$ représente des atomes de chlore, de brome ou un groupe -$OR_1'$,
$R_1'$ représente un atome d'hydrogène, des groupes alcanoyle en $C_1$-$C_{12}$, alcanoyle en $C_3$-$C_{12}$ interrompu pr un atome d'oxygène, cyclohexylcarbonyle, benzoyle, naphtoyle, (alcane en $C_1$-$C_{12}$)sulfonyle, (alcane en $C_1$-$C_{12}$)sulfonyle substitué par du fluor ; phénylsulfonyle ou phénylsulfonyle substitué par alkyle en $C_1$-$C_4$ ;

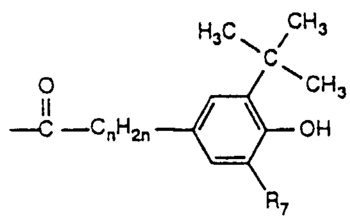
,
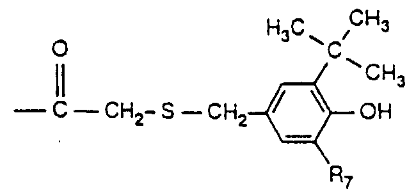
,

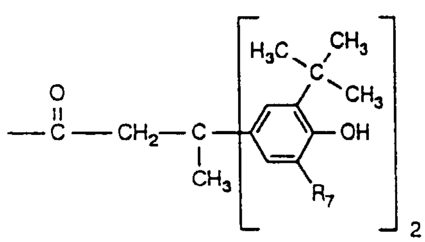
,
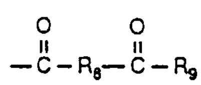

ou
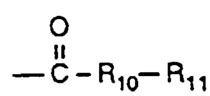

$R_7$ représente un atome d'hydrogène ou un groupe alkyle en $C_1$-$C_6$,
$R_8$ représente une liaison directe, des groupes alkylène en $C_1$-$C_{12}$, alkylène en $C_2$-$C_{12}$ interrompu par des atomes d'oxygène, de soufre ou $>N—R_6$ ; alcénylène en $C_2$-$C_{12}$, alkylidène en $C_2$-$C_{12}$, phénylalkylidène en $C_7$-$C_{12}$, cycloalkylène en $C_5$-$C_8$, bicycloalkylène en $C_7$-$C_8$ ou phénylène,
$R_9$ représente des groupes hydroxy, alkoxy en $C_1$-$C_{12}$ ou

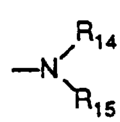

$R_{10}$ représente un atome d'oxygène ou -NH-,
$R_{11}$ représente des groupes alkyle en $C_1$-$C_{12}$ ou phényle, et
$R_{14}$ et $R_{15}$,représentent, indépendamment l'un de l'autre, un atome d'hydrogène ou un groupe alkyle en $C_1$-$C_8$.

**6.** Composition selon la revendication 1, dans laquelle

$R_1'$ représente un atome d'hydrogène, des groupes alcanoyle en $C_1$-$C_8$, benzoyle, méthanesulfonyle, p-méthylphénylsulfonyle

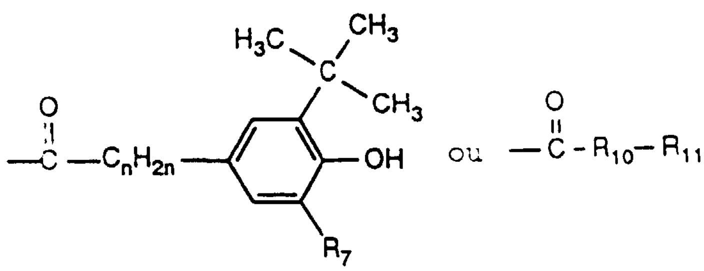

$R_7$ représente un atome d'hydrogène ou un groupe alkyle en $C_1$-$C_4$,
$R_{10}$ représente -NH-,
$R_{11}$ représente des groupes alkyle en $C_1$-$C_8$ ou phényle, et
n vaut 2.

**7.** Composition selon la revendication 1, dans laquelle

| | |
|---|---|
| $R_2$, $R_3$, $R_4$ et $R_5$ | représentent, indépendamment les uns des autres, un atome d'hydrogène, un atome de chlore, des groupes hydroxy, alkyle en $C_1$-$C_{18}$, phénylalkyle en $C_7$-$C_9$, phényle, cycloalkyle en $C_5$-$C_8$, alkoxy en $C_1$-$C_6$, cyclohexylcarbonyloxy ou benzoyloxy, ou de plus les restes $R_4$ et $R_5$ forment, conjointement avec les atomes de carbone auxquels ils sont liés, un cycle benzo, $R_4$ représente de plus $-(CH_2)_p-COR_9$, ou lorsque $R_3$ et $R_5$ représentent un atome d'hydrogène, $R_4$ représente de plus un reste de formule II, |
| $R_7$ | représente un atome d'hydrogène ou un groupe alkyle en $C_1$-$C_4$, |
| $R_8$ | un groupe alkylène en $C_1$-$C_{12}$ ou phénylène, |
| $R_9$ | représente un groupe hydroxy ou alcoxy en $C_1$-$C_8$, |
| $R_{10}$ | représente -NH-, |
| $R_{11}$ | représente un groupe alkyle en $C_1$-$C_8$ ou phényle, et |
| $R_{12}$ et $R_{13}$ | représentent des groupes méthyle ou forment conjointement avec l'atome de carbone auquel ils sont liés un cycle cycloalkylidène en $C_5$-$C_8$. |

**8.** Composition selon la revendication 1, dans laquelle

| | |
|---|---|
| $R_1$ | représente un atome de chlore ou $-OR_1'$, |
| $R_1'$ | représente un atome d'hydrogène, des groupes alcanoyle en $C_1$-$C_8$, benzoyle, méthanesulfonyle, triflorométhanesulfonyle ; phénylsulfonyle ou phénylsulfonyle substitué par alkyle en $C_1$-$C_4$ ; |

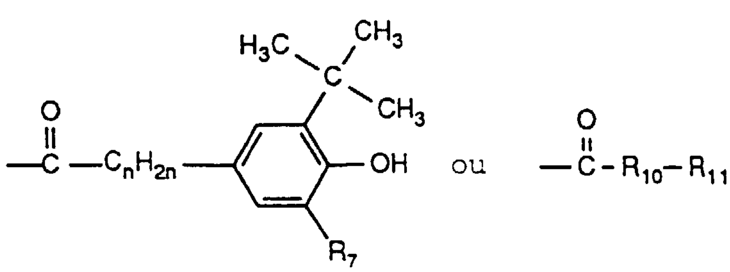

| | |
|---|---|
| $R_2$, $R_3$, $R_4$ et $R_5$ | représentent, indépendamment les uns des autres, un atome d'hydrogène, un atome de |

chlore, des groupes alkyle en $C_1$-$C_{18}$, phénylalkyle en $C_7$-$C_9$, phényle, cyclohexyle, alkoxy en $C_1$-$C_6$, $R_4$ représente de plus $-(CH_2)_p-COR_9$, ou lorsque $R_3$ et $R_5$ représentent un atome d'hydrogène, $R_4$ représente de plus un reste de formule II

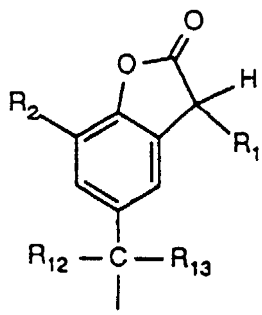

(II)

| | |
|---|---|
| $R_7$ | représente un atome d'hydrogène ou un groupe alkyle en $C_1$-$C_4$, |
| $R_9$ | représente un groupe hydroxy ou alkoxy en $C_1$-$C_8$, |
| $R_{10}$ | représente -NH-, |
| $R_{11}$ | représente un groupe alkyle en $C_1$-$C_4$ ou phényle, |
| $R_{12}$ et $R_{13}$ | forment, conjointement avec l'atome de carbone auquel ils sont liés, un cycle cyclohexylidène, |
| n | vaut 2, et |
| p | vaut 2. |

**9.** Composition selon la revendication 1, dans laquelle

| | |
|---|---|
| $R_1$ | représente un atome de chlore ou $-OR_1'$, |
| $R_1'$ | représente un atome d'hydrogène, un groupe acétyle |

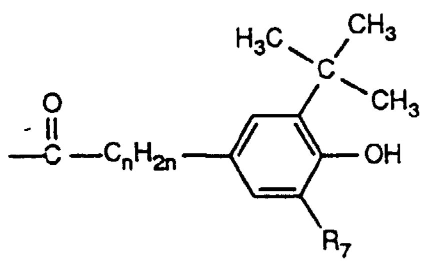

ou

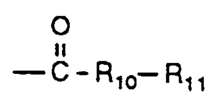

| | |
|---|---|
| $R_2$ | représente des groupes alkyle en $C_1$-$C_{16}$, phénylalkyle en $C_7$-$C_9$ ou cyclohexyle, |
| $R_3$ | représente un atome d'hydrogène, |
| $R_4$ | représente des groupes alkyle en $C_1$-$C_4$, phénylalkyle en $C_7$-$C_9$, cyclohexyle, $-CH_2CH_2COOH$ ou un reste de formule II |

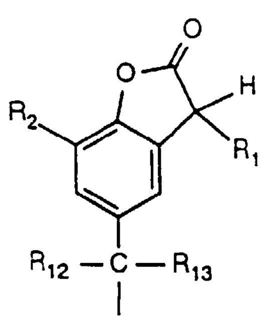

(II)

| | |
|---|---|
| $R_5$ | représente un atome d'hydrogène, |
| $R_7$ | représente un atome d'hydrogène ou un groupe alkyle en $C_1$-$C_4$, |
| $R_{10}$ | représente -NH-, |
| $R_{11}$ | représente un groupe alkyle en $C_1$-$C_4$, |
| $R_{12}$ et $R_{13}$ | forment, ensemble avec l'atome de carbone auquel ils sont liés, un cycle cyclohexylidène, et |
| n | vaut 2. |

**10.** Composition selon la revendication 1, contenant, outre les composants (a) et (b), encore d'autres additifs.

**11.** Composition selon la revendication 10, contenant en tant que d'autres additifs des antioxydants, des agents photoprotecteurs et/ou des adjuvants de mise en oeuvre.

**12.** Composition selon la revendication 10, contenant en tant que autre additif au moins un composé du type des phosphites ou phosphonites organiques.

**13.** Composition selon la revendication 1, contenant en tant que composant (a) des polymères naturels, semi-synthétiques ou synthétiques.

**14.** Composition selon la revendication 1, contenant entant que composant (a) une polyoléfine.

**15.** Composition selon la revendication 1, contenant en tant que composant (a) du polyéthylène ou du polypropylène.

**16.** Composition selon la revendication 1, dans laquelle le composant (b) est contenu dans une quantité de 0,0005 à 5 % par rapport au poids du composant (a).

**17.** Composé de formule la

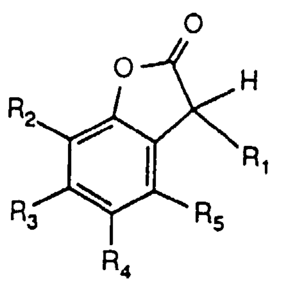

(Ia)

dans laquelle

| | |
|---|---|
| $R_1$ | représente un atome d'halogène ou $OR_1'$, |
| $R_1'$ | représente un atome d'hydrogène, des groupes alcanoyle en $C_1$-$C_{25}$, alcénoyle en $C_3$-$C_{25}$, alcanoyle en $C_3$-$C_{25}$ interrompu par des atomes d'oxygène, de soufre ou $>N—R_6$ ; cyclo (alkyl en $C_6$-$C_9$)-carbonyle, thénoyle, furoyle, benzoyle ou benzoyle substitué par alkyle en $C_1$-$C_{12}$ ; naphtoyle ou naphtoyle substitué par alkyle en $C_1$-$C_{12}$ ; (alcane en $C_1$-$C_{25}$)sulfonyle, (alcane en $C_1$-$C_{25}$)sulfonyle substitué par du fluor ; phénylsulfonyle ou phénylsulfonyle substitué par alkyle en $C_1$-$C_{12}$ ; |

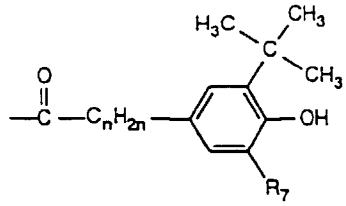
,
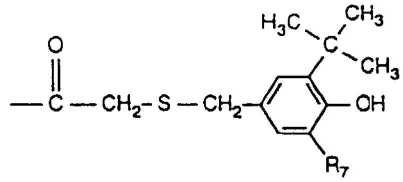
,

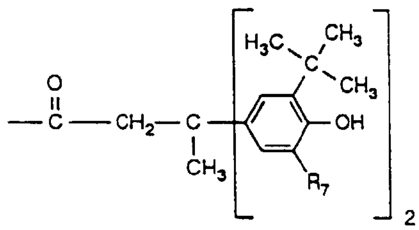
,
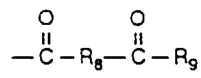
ou
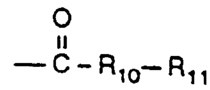

$R_2$, $R_3$, $R_4$ et $R_5$ représentent, indépendamment les uns des autres, un atome d'hydrogène, un atome de chlore, des groupes hydroxy, alkyle en $C_1$-$C_{25}$, phénylalkyle en $C_7$-$C_9$, phényle non substitué ou substitué par alkyle en $C_1$-$C_4$, cycloalkyle en $C_5$-$C_8$ non substitué ou substitué par alkyle en $C_1$-$C_4$ ; alkoxy en $C_1$-$C_{18}$, (alkyl en $C_1$-$C_{18}$)thio, (alkyl en $C_1$-$C_4$)amino, di(alkyl en $C_1$-$C_4$)amino, alcanoyloxy en $C_1$-$C_{25}$, (alcanoyl en $C_1$-$C_{25}$)amino, alcénoyloxy en $C_3$-$C_{25}$, alcanoyloxy en $C_3$-$C_{25}$ interrompu par des atomes d'oxygène, de soufre ou $>N—R_6$ ; cyclo (alkyl en $C_6$-$C_9$)-carbonyloxy, benzoyloxy ou benzoyloxy substitué par alkyle $C_1$-$C_{12}$, ou les restes $R_2$ et $R_3$ ou les restes $R_3$ et $R_4$ ou les restes $R_4$ et $R_5$ de plus forment, conjointement avec les atomes de carbone auxquels ils sont liés, un cycle benzo ; à la condition qu'au moins l'un des restes $R_2$, $R_3$, $R_4$ ou $R_5$ soit différent de l'hydrogène ; lorsque $R_1$ représente un atome d'halogène ou un groupe hydroxy, $R_2$ est différent de l'hydrogène ; et lorsque $R_2$ représente des groupes méthyle ou méthylamine, $R_4$ est différent de l'hydrogène et du groupe hydroxy ; $R_4$ représente de plus $-(CH_2)_p-COR_9$ ou $-(CH_2)_qOH$, ou lorsque $R_3$ et $R_5$ représentent un atome d'hydrogène, $R_4$ représente de plus un reste de formule IIa

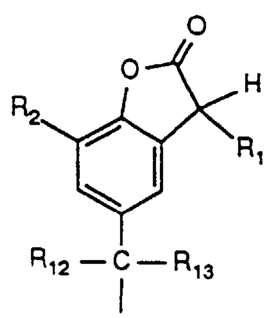

(IIa)

$R_6$ représente un atome d'hydrogène ou un groupe alkyle en $C_1$-$C_8$,

$R_7$ représente un atome d'hydrogène ou un groupe alkyle en $C_1$-$C_8$,

$R_8$ représente une liaison directe, des groupes alkylène en $C_1$-$C_{18}$, alkylène en $C_2$-$C_{18}$ interrompu par des atomes d'oxygène, de soufre ou $>N—R_6$ ; alcénylène en $C_2$-$C_{18}$, alkylidène en $C_2$-$C_{20}$, phénylalkylidène en $C_7$-$C_{20}$, cycloalkylène en $C_5$-$C_8$, bicycloalkylène en $C_7$-$C_8$, phénylène non substitué ou substitué par alkyle en $C_1$-$C_4$,

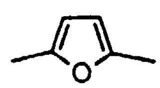

ou

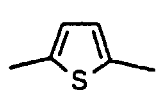

R$_9$ représente des groupes hydroxy, [$-O^{\ominus}\frac{1}{r}M^{r+}$], alkoxy en $C_1$-$C_{18}$ ou

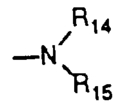

R$_{10}$ représente un atome d'oxygène,

-NH- ou 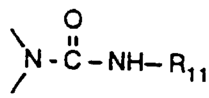

R$_{11}$ représente des groupes alkyle en $C_1$-$C_{18}$ ou phényle,

R$_{12}$ et R$_{13}$ représentent, indépendamment l'un de l'autre, un atome d'hydrogène, des groupes $CF_3$, alkyle en $C_1$-$C_{12}$ ou phényle, ou R$_{12}$ et R$_{13}$ forment, conjointement avec l'atome de carbone auquel ils sont liés, un cycle cycloalkylidène en $C_5$-$C_8$ non substitué ou substitué par 1 à 3 groupes alkyle en $C_1$-$C_4$ ;

R$_{14}$ et R$_{15}$, représentent, indépendamment l'un de l'autre, un atome d'hydrogène ou un groupe alkyle en $C_1$-$C_{18}$,

M représente un cation métallique à fonctionnalité r,

n vaut 0, 1 ou 2,

p vaut 0, 1 ou 2,

q vaut 1, 2, 3, 4, 5 ou 6 et

r vaut 1, 2 ou 3.

**18.** Composés selon la revendication 17, dans lesquels R$_3$ et R$_5$ représentent un atome d'hydrogène.

**19.** Composés selon la revendication 17, dans lesquels

R$_1$ représente un atome de chlore, de brome ou -OR$_1'$,

R$_1'$ représente un atome d'hydrogène, des groupes alcanoyle en $C_1$-$C_{12}$, alcanoyle en $C_3$-$C_{12}$ interrompu par des atomes d'oxygène ; cyclohexylcarbonyle, benzoyle, naphtoyle, (alcane en $C_1$-$C_{12}$)sulfonyle, (alcane en $C_1$-$C_{12}$)sulfonyle substitué par du fluor ; phénylsulfonyle ou phénylsulfonyle substitué par alkyle en $C_1$-$C_4$ ;

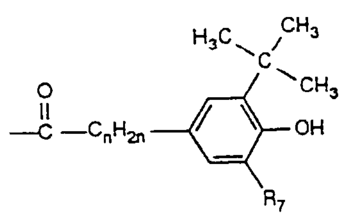 , 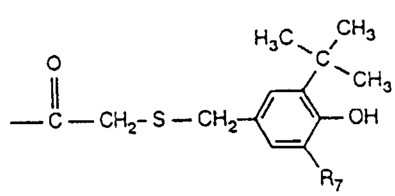 ,

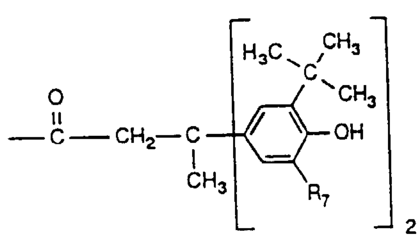 , 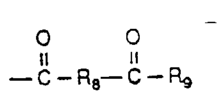

ou 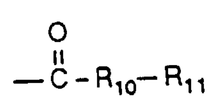

| | |
|---|---|
| $R_2$, $R_3$, $R_4$ et $R_5$, | représentent, indépendamment les uns des autres, un atome d'hydrogène, un atome de chlore, des groupes hydroxy, alkyle en $C_1$-$C_{18}$, phénylalkyle en $C_7$-$C_9$, phényle, cycloalkyle en $C_5$-$C_8$, alkoxy en $C_1$-$C_6$, cyclohexylcarbonyloxy ou benzoyloxy, ou de plus les restes $R_2$ et $R_3$ ou les restes $R_3$ et $R_4$ ou les restes $R_4$ et $R_5$ forment, conjointement avec les atomes de carbone auxquels ils sont liés, un cycle benzo ; à la condition qu'au moins l'un des restes $R_2$, $R_3$, $R_4$ ou $R_5$ soit différent de l'hydrogène ; lorsque $R_1$ représente un groupe hydroxy, un atome de chlore ou de brome, $R_2$ est différent de l'hydrogène ; et lorsque des $R_2$ représente des groupes méthyle ou hydroxy, $R_4$ est différent de l'hydrogène et du groupe hydroxy ; $R_4$ représente de plus $-(CH_2)_p-COR_9$, ou lorsque $R_3$ et $R_5$ représentent un atome d'hydrogène, $R_4$ représente de plus un reste de formule IIa |

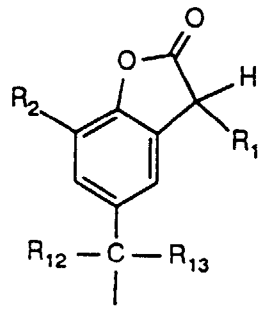

(IIa)

| | |
|---|---|
| $R_7$ | représente un atome d'hydrogène ou un groupe alkyle en $C_1$-$C_4$, |
| $R_8$ | représente des groupes alkylène en $C_1$-$C_{12}$ ou phénylène, |
| $R_9$ | représente des groupes hydroxy ou alkoxy en $C_1$-$C_8$, |
| $R_{10}$ | représente -NH-, |
| $R_{11}$ | représente des groupes alkyle en $C_1$-$C_8$ ou phényle et |
| $R_{12}$ et $R_{13}$ | représentent des groupes méthyle ou forment, conjointement avec l'atome de carbone auquel ils sont liés, un cycle cycloalkylidène en $C_5$-$C_8$. |

**20.** Composés selon la revendication 17, dans lesquels

| | |
|---|---|
| $R_1$ | représente un atome de chlore ou $-OR_1'$, |
| $R_1'$ | représente un atome d'hydrogène, des groupes alcanoyle en $C_1$-$C_8$, benzoyle, méthanesulfonyle, trifluorométhanesulfonyle ; phénylsulfonyle ou phénylsulfonyle substitué par alkyle en $C_1$-$C_4$ ; |

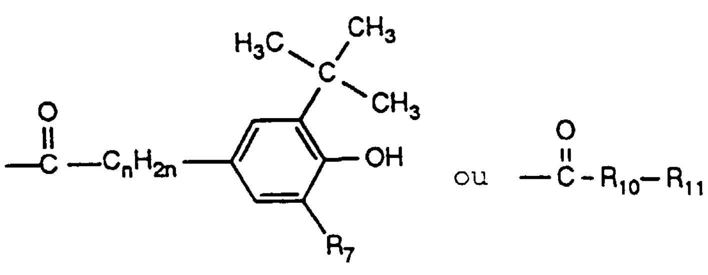

| | |
|---|---|
| $R_2$, $R_3$, $R_4$ et $R_5$ | représentent, indépendamment les uns des autres, un atome d'hydrogène, un atome de chlore, des groupes hydroxy, alkyle en $C_1$-$C_{18}$, phénylalkyle en $C_7$-$C_9$, phényle, cyclohexyle, alkoxy en $C_1$-$C_6$ ; à la condition qu'au moins l'un des restes $R_2$, $R_3$, $R_4$ ou $R_5$ soit différent de l'hydrogène ; et lorsque $R_2$ représente un groupe méthyle, $R_4$ est diffèrent de l'hydrogène ; $R_4$ représente de plus $-(CH_2)_p-COR_9$, ou lorsque $R_3$ et $R_5$ représentent un atome d'hydrogène, $R_4$ représente de plus un reste de formule IIa |

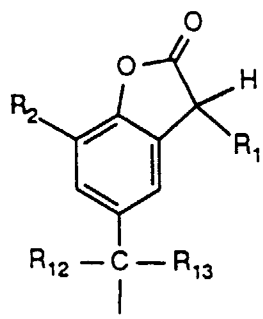

(IIa)

| | |
|---|---|
| $R_7$ | représente un atome d'hydrogène ou un groupe alkyle en $C_1$-$C_4$, |
| $R_9$ | représente des groupes hydroxy ou alkoxy en $C_1$-$C_8$, |
| $R_{10}$ | représente -NH-, |
| $R_{11}$ | représente des groupes alkyle en $C_1$-$C_4$ ou phényle, |
| $R_{12}$ et $R_{13}$ | forment, conjointement avec l'atome de carbone auquel ils sont liés, un cycle cyclohexylidène, |
| n | vaut 2 et |
| p | vaut 2. |

**21.** Utilisation des composés de formule I définie dans la revendication 1, en tant que stabilisants de matières organiques contre la dégradation oxydative, thermique ou induite par la lumière.

**22.** Procédé pour la stabilisation d'une matière organique contre la dégradation oxydative, thermique ou induite par la lumière, caractérisé en ce que l'on incopore dans celle-ci ou l'on applique sur celle-ci au moins un composé de formule I définie dans la revendication 1.

**23.** Procédé pour la préparation de composés de formule I, dans laquelle les symboles généraux sont définis comme dans la revendication 1, caractérisé en ce que

a) on fait réagir un équivalent de phénol de formule III

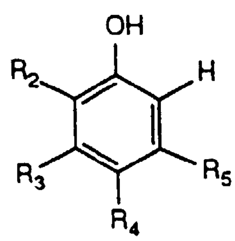

(III)

dans laquelle les symboles généraux sont définis comme dans la revendication 1, avec 0,8 à 2,0 équivalents d'acide glyoxylique pour obtenir un composé de formule IV

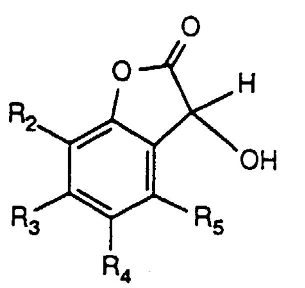

(IV)

dans laquelle les symboles généraux sont définis comme dans la revendication 1, et
b) pour préparer des composés de formule I, dans lesquels $R_1^{'}$ ne représente pas un atome d'hydrogène, on fait réagir le composé obtenu de formule IV avec un hydracide halogéné, un halogénure d'un acide sulfo-oxygéné, un halogénure de l'acide phosphorique, un halogénure de l'acide phosphoreux, un acide de formule V

$$R_1^{'}\text{-OH} \qquad (V)$$

un halogénure d'acide de formule VI

$$R_1^{'}\text{-Y} \qquad (VI)$$

un ester de formule VII

$$R_1^{'}\text{-O-}R_{16} \qquad (VII)$$

un anhydride symétrique ou asymétrique de formule VIII

$$R_1^{'}\text{-O-}R_1^{'} \qquad (VIII)$$

ou un isocyanate de formule IX

$$R_{11}\text{-N=C=O} \qquad (IX)$$

dans laquelle $R_1^{'}$ et $R_{11}$ dans les composés de formules V, VI, VII, VIII et IX sont définis comme dans la revendication 1, à la condition que $R_1^{'}$ ne représente pas l'hydrogène ;

$R_{16}$ représente un groupe alkyle en $C_1$-$C_8$, et
Y représente des atomes de fluor, de chlore, de brome ou d'iode.

**24.** Procédé selon la revendication 23, dans lequel on utilise l'acide glyoxylique sous forme d'acide glyoxylique aqueux à une concentration de 40 à 60 %, en particulier sous forme d'acide glyoxylique aqueux à 50 %.